# EUROPÄISCHE PATENTSCHRIFT

(11) **EP 3 658 254 B1**
(45) Veröffentlichungstag und Bekanntmachung des Hinweises auf die Patenterteilung: **24.03.2021**
(21) Anmeldenummer: 18765097.3
(22) Anmeldetag: 03.09.2018
(51) Int. Cl.: B01D 39/16

(54) **LUFTDURCHLÄSSIGES FLÄCHENFILTERMATERIAL, SEINE HERSTELLUNGSVERFAHREN UND SEINE VERWENDUNG**
AIR-PERMEABLE SHEET FILTER MATERIAL, METHODS FOR THE PRODUCTION AND USE THEREOF
MATÉRIAU FILTRANT PLAN PERMÉABLE À L'AIR, SON PROCÉDÉ DE FABRICATION ET SON UTILISATION

(30) Priorität: 28.11.2017 DE 102017010986; 17.01.2018 DE 102018100935
(43) Veröffentlichungstag der Anmeldung: 03.06.2020
(73) Patentinhaber: Blücher GmbH, 40699 Erkrath (DE)
(72) Erfinder: BÖHRINGER, Bertram, 42115 Wuppertal (DE); FISCHER, Rainer, 41470 Neuss (DE); NGUYEN, Congminh, 42277 Wuppertal (DE)
(74) Vertreter: Strehlke, Ingo Kurt
(86) Internationale Anmeldenummer: PCT/EP2018/073606
(87) Internationale Veröffentlichungsnummer: WO 2019/105611

(56) Entgegenhaltungen:
- DE-A1-102014 216 979
- DE-U1-202015 104 218
- US-A1- 2012 312 744

## Beschreibung

Die vorliegende Erfindung betrifft das technische Gebiet funktioneller Flächenfiltermaterialien, wie insbesondere textiler Schutzmaterialien, welche im militärischen oder zivilen Bereich beispielsweise für die Herstellung von Schutzausrüstungen und Schutzbekleidung sowie von funktioneller Sport- bzw. Freizeitbekleidung sowie für diverse Filteranwendungen eingesetzt werden können.

In diesem Zusammenhang betrifft die vorliegende Erfindung ein Flächenfiltermaterial als solches, welches mit einer Aerosol- bzw. Partikelfilterfunktion bzw. mit einer Schutzfunktion gegenüber chemischen, biologischen bzw. radioaktiven (nuklearen) Schad- und Giftstoffen ausgerüstet ist bzw. eine entsprechende Filter- bzw. Schutzfunktion bereitstellt, wobei das erfindungsgemäße Flächenfiltermaterial einen speziellen Aufbau auf Basis eines schaumbasierten bzw. schaumförmigen Trägermaterials einerseits und einer dem schaumbasierten bzw. schaumförmigen Trägermaterial zugeordneten Nanofaserschicht andererseits aufweist.

Darüber hinaus betrifft die vorliegende Erfindung auch ein Verfahren zur Herstellung des Flächenfiltermaterials nach der Erfindung sowie spezielle Verwendungen des erfindungsgemäßen Flächenfiltermaterials zur Herstellung von Schutzausrüstungen bzw. Schutzgegenständen, Sport- bzw. Freizeitbekleidung bzw. -ausrüstung sowie zur Herstellung von Filtern und Filtermaterialien aller Art. Zudem betrifft die vorliegende Erfindung auch Schutzausrüstungen sowie Schutzgegenstände und Filter sowie Filtermaterialien, welche das erfindungsgemäße Flächenfiltermaterial umfassen bzw. welche unter Verwendung des Flächenfiltermaterials nach der Erfindung hergestellt sind.

An funktionelle Flächenfiltermaterialien, wie sie beispielsweise im Bereich der Textilindustrie zur Herstellung von Schutzbekleidung für den militärischen Bereich sowie darüber hinaus auch für den Sport- bzw. Freizeitbereich zur Herstellung funktioneller Bekleidungsstücke bzw. Ausrüstungsgegenstände eingesetzt werden können, werden im Allgemeinen hohe Anforderungen hinsichtlich der Bereitstellung eines guten Tragekomforts (wie z. B. einer hohen Atmungsaktivität sowie eines angenehmen Tragegefühls) einerseits und der Bereitstellung einer darüber hinausgehenden Schutzfunktion gegenüber äußeren Einwirkungen, wie Umwelteinflüssen (z. B. Wind, Regen oder dergleichen), sowie gegenüber toxischen Substanzen (wie sie z. B. im Hinblick auf militärisches Personal bzw. Soldaten im Verteidigungs- bzw. Kampfeinsatz oder aber im Hinblick auf beispielsweise im Katastrophen- bzw. Brandeinsatz befindliches Personal, wie Feuerwehrleuten oder dergleichen, auftreten können) andererseits gestellt. In diesem Zusammenhang besteht bei der Entwicklung entsprechender Flächenfiltermaterialien eine große Herausforderung darin, die diametralen Eigenschaften eines hohen Tragekomforts sowie einer effektiven Schutzfunktion in ein und demselben Material zu vereinen, was bei den im Stand der Technik bekannten Materialien bislang oftmals nicht in zufriedenstellender Weise gelungen ist. Darüber hinaus ist es nicht zuletzt auch vor dem Hintergrund des jeweils vorgesehenen Anwendungs- bzw. Einsatzbereich (z.B. Einsatz im militärischen Bereich bzw. bei der Katastrophen- oder Brandbekämpfung) mit der damit einhergehenden hohen Beanspruchung des Materials erforderlich, dass die entsprechende Schutzausrüstung bzw. Bekleidung eine hohe mechanische Stabilität aufweist, beispielsweise was die Verhinderung von vorzeitiger bzw. übermäßiger Rissbildung und Delaminierung oder dergleichen des zugrundeliegenden Materials anbelangt.

In diesem Zusammenhang ist auch hervorzuheben, dass insbesondere im militärischen Einsatzbereich bzw. im Bereich der Brand- bzw. Katastrophenabwehr, aber auch im Freizeit- bzw. Sportbereich, oftmals eine hohe körperliche Aktivität bzw. Belastung in Bezug auf die jeweiligen Personen vorliegt, zumal in Kombination mit widrigen Einsatzbedingungen, wie beispielsweise dem Vorliegen einer hohen Temperaturen oder dergleichen, so dass auch von daher ein effektiver Abtransport von Schweiß bzw. ein effektiver Luftaustausch durch eine zum Einsatz kommende (Schutz-)Bekleidung zu gewährleisten ist. Dabei ist es auch erforderlich, dass die hierzu eingesetzten Materialien ein möglichst angenehmes Tragegefühl bereitstellen, um auch unter diesem Aspekt eine möglichst weitreichende Entlastung von mit entsprechender Bekleidung ausgerüsteten Personen zu ermöglichen.

Im Hinblick auf die Bereitstellung eines umfassenden Schutzkonzepts ist zudem beachtlich, dass insbesondere im militärischen Einsatz befindliche Personen bzw. Soldaten sowie im Rahmen der Katastrophenabwehr bzw. in der Feuerbekämpfung tätige Personen oftmals der zusätzlichen Gefahr einer Kontamination mit chemischen, biologischen bzw. radioaktiven Schad- und Giftstoffen ausgesetzt sind. Von chemischen, biologischen und radioaktiven nuklearen Gift- bzw. Kampfstoffen geht dabei im Allgemeinen ein hohes Gefährdungspotential für mit solchen Substanzen in Kontakt kommenden Personen, wie beispielsweise sich im Kampfeinsatz oder dergleichen befindliche Soldaten Feuerwehrmännern, einher, zumal bereits geringe Mengen bzw. Konzentrationen derartiger Substanzen zu einer nachhaltigen gesundheitlichen Beeinträchtigung bis hin zum Tod von mit diesen Substanzen konfrontierten Personen führen können. In diesem Zusammenhang ist auch von Bedeutung, dass die vorgenannten Substanzen oftmals in Form von feinteiligen bzw. feinpartikulären Aerosolen oder dergleichen bzw. in Form von in der (Umgebungs-)Luft vorhandenen Schadstoffpartikeln vorliegen können, was jedoch eine große und zusätzliche Herausforderung hinsichtlich der Bereitstellung einer entsprechenden Schutzfunktion darstellt, da die in Rede stehenden Aerosole bzw. feinteiligen Schadstoffpartikel entsprechend hohe Penetrationseigenschaften beispielsweise in Bezug auf luftdurchlässige Bekleidungsstücke oder dergleichen aufweisen.

In diesem Zusammenhang gibt es eine Reihe von Substanzen bzw. Stoffen, die bei Kontakt von der Haut aufgenommen werden und bereits in geringen Mengen bzw. Konzentrationen zu schwerwiegenden körperlichen Schäden führen. Im Bereich der chemischen Schad- bzw. Giftstoffe (Kampfstoffe) können hierzu insbesondere das blasenziehende Lost (Gelbkreuz) und das Nervengift Sarin genannt werden. Personen, die mit solchen hochtoxischen Giften in Kontakt kommen können, müssen demnach eine geeignete Schutzbekleidung tragen bzw. durch geeignete Schutzmaterialien gegen diese Substanzen geschützt werden.

Dies gilt grundsätzlich auch für biologische Schad- bzw. Giftstoffe, wie sie gleichermaßen auch als Kampfstoffe eingesetzt werden können und welche gleichermaßen bei Kontakt und insbesondere direkt oder indirekt (d. h. durch nachfolgende Aufnahme in den Körper, beispielsweise über Schleimhäute oder dergleichen) zu nachhaltigen gesundheitlichen Problemen führen. Weiterhin ist auch ein direkter Kontakt bzw. eine Kontamination mit radioaktiven Substanzen, insbesondere auch in Form von radioaktiven Partikeln oder dergleichen, zu vermeiden.

Zur Gewährleistung einer gewissen Schutzfunktion gegenüber den vorgenannten Schad- und Giftstoffen sind im Stand der Technik beispielsweise luft- und wasserdampfundurchlässige Schutzanzüge bekannt, welche mit einer gegenüber Gift- bzw. Kampfstoffen, insbesondere der vorgenannten Art, undurchlässigen Sperr- bzw. Gummischicht ausgestattet sind. Die undurchlässige Gummischicht führt infolge der Luftundurchlässigkeit bzw. Impermeabilität der Membran auch zu einer gewissen Schutzfunktion gegenüber Aerosolen und Schadstoffpartikeln. Zudem bietet die in Rede stehende Gummischicht grundsätzlich auch einen Schutz gegenüber Wasser auch in flüssiger Form und somit einen entsprechenden Regenschutz. Derartige Schutzsysteme sind jedoch mit zentralen Nachteil verbunden, dass hieraus hergestellte Bekleidung bzw. Schutzausrüstungen, beispielsweise in Form von Schutzanzügen, im Trage- bzw. Anwendungszustand (beispielsweise im Rahmen eines Kampfeinsatzes mit hoher körperlicher Belastung des Trägers) sehr schnell zu einem Hitzestau führen, da aufgrund der fehlenden Luft- und Wasserdampfdurchlässigkeit keine feuchtigkeits- und thermoregulativen Eigenschaften vorliegen, zumal weder eine Atmungsaktivität noch ein effizienter Luftaustausch vorhanden ist. Aus diesem Grund eignen sich derartige Schutzsysteme grundsätzlich auch nicht für den mit einer hohen körperlichen Aktivität eingehergehenden Sport- bzw. Freizeitbereich, da, wie zuvor angeführt, unter körperlicher Belastung anfallende Körperwärme sowie Körperschweiß nicht effektiv von der Bekleidung entfernt werden kann.

Darüber hinaus kommen im Stand der Technik beispielsweise zur Herstellung von Schutzanzügen auch solche Schutzmaterialien zum Einsatz, welche mit einer luftundurchlässigen, jedoch wasserdampfdurchlässig ausgebildeten Membran ausgerüstet sind, welche insbesondere als Sperrschicht gegenüber toxischen Substanzen fungieren soll. Ein derartiges Schutzmaterial ist beispielsweise in der WO 96/37365 A1 sowie in der zu derselben Patentfamilie gehörenden US 5 743 775 A bzw. der DE 195 18 683 A1 beschreiben. Schutzanzüge mit einer für Wasserdampf durchlässigen, jedoch für Gifte, insbesondere Hautgifte, im Wesentlichen undurchlässigen Membran besitzen den grundsätzlichen Nachteil, dass an undichten Stellen, welche beispielsweise durch mechanische Beschädigungen hervorgerufen werden können, eingedrungene Gifte in das Innere des Schutzanzugs eindringen und folglich durch die Haut des Trägers aufgenommen werden können. Zudem ist der Tragekomfort aufgrund des insgesamt beschränkten Luftaustauschs nicht immer zufriedenstellend.

Zur Verbesserung insbesondere des Tragekomforts sind im Stand der Technik zudem auch luftdurchlässige, permeable Schutzmaterialien bekannt, welche zu Zwecken der Gewährleistung einer Schutzfunktion gegenüber Schad- und Giftstoffen mit einer Adsorptionsfilterschicht auf Basis von Aktivkohle ausgerüstet sein können. Wie zuvor angeführt, weisen derartige Schutzsysteme durch den erhöhten Austausch von Luft und Wasser bzw. Wasserdampf einen verbesserten Tragekomfort bei grundsätzlich guter Schutzfunktion gegenüber Gift- bzw. Schadstoffen auf, zumal ein Vorteil darin liegt, dass die gegebenenfalls eingesetzte Aktivkohle auch an der Innenseite, d. h. auf der dem Träger zugewandten Seite des Schutzmaterials zugänglich ist, so dass an Beschädigungen oder sonstigen undichten Stellen eingedrungene Gifte schnell adsorbiert und unschädlich gemacht werden können. Somit besitzen die permeablen, luftdurchlässigen, adsorptiv wirkenden Schutzanzüge zwar eine im Allgemeinen gute Schutzwirkung auf, welche aber bei Vorliegen der topischen Substanzen in Form von Aerosolen und Schadstoffpartikeln nicht immer zufriedenstellend ist. Grundsätzlich weisen die in Rede stehenden luftdurchlässigen Systeme als solche auch eine verbesserte Eignung im Hinblick auf einen Einsatz im Sport- bzw. Freizeitbereich auf. Jedoch kann der Tragekomfort insbesondere aufgrund eines nicht optimalen Tragegefühls verschlechtert sein, da die in Rede stehenden Materialien nicht immer optimale Materialeigenschaften aufweisen, beispielsweise was deren Biegsamkeit bzw. Dehnbarkeit sowie deren Anschmiegsamkeit anbelangt, so dass das damit einhergehende Tragegefühl entsprechend beeinflusst ist. Zudem sind auch die windabweisenden Eigenschaften und der Nässeschutz derartiger Materialien nicht immer optimal, was unter gegebenen Bedingungen (starker Wind, Regenfälle etc.) zu einem übermäßigen Auskühlen des Trägers einer solchen Schutzbekleidung führen kann. Folglich weisen die in Rede stehenden Materialien auch nicht immer eine optimale Thermo- bzw. Klimaregulation auf.

Die DE 10 2014 216 979 A1 betrifft einen Endabscheider für eine Endabscheidereinrichtung zum Abtrennen von Wasser aus einer von Wasser verschiedenen Flüssigkeit, insbesondere aus einem Öl- oder Kraftstoffstrom, mit einem für die Flüssigkeit durchströmbaren Filterkörper, welcher zumindest ein für die Flüssigkeit durchströmbares Filtermaterial aufweist, wobei der Filterkörper eine erste Seite und eine von der ersten Seite abgewandte zweite Seite aufweist, wobei auf der ersten Seite zumindest bereichsweise eine Nanofaserschicht aufgebracht ist und wobei die Nanofaserschicht hydrophob ausgestaltet ist.

Weiterhin betrifft die US 2012/312744 A1 eine Filtervorrichtung für ein Aquarium, wobei die Filtervorrichtung eine Filterkammer zur Aufnahme von Wasser und eines Filtermediums umfasst, wobei das Filtermedium Fasern mit speziellen Durchmesser und einem definierten Verhältnis von Faserlänge zu Faserdurchmesser aufweisen soll. Dabei sollen die Fasern einen Bewuchs mit Nitrobakterien bzw. Nitrosomonas-Bakterien ermöglichen.

Zudem betrifft die DE 20 2015 104 218 U1 ein textiles Schutzmaterial, welches einen textilen Träger und eine auf den textilen Träger aufgebrachte Klebstoffschicht aufweist, wobei die Klebstoffschicht eine luftdurchlässige bzw. diskontinuierlich ausgebildete Schicht auf Basis eines getrockneten bzw. ausgehärteten gebrochenen Klebstoffpolymerschaums ist. Das Schutzmaterial soll zudem eine an der Klebstoffschicht zum Haften gebrachte Adsorptionsschicht aufweisen.

Zusammenfassend ist somit festzustellen, dass die im Stand der Technik bekannten Schutzmaterialien bzw. Filtermaterialien, welche insbesondere zur Herstellung von Schutzbekleidung bzw. -ausrüstung eingesetzt werden können, nicht immer optimale und insbesondere auch dem jeweiligen Anwendungs- bzw. Einsatzzweck berücksichtigende Materialeigenschaften im Hinblick auf die Gewährleistung eines hohen Tragekomforts einerseits bei gleichzeitig hoher Schutzfunktion andererseits aufweisen.

Vor diesem Hintergrund besteht somit die Aufgabe der vorliegenden Erfindung darin, ein Flächenfiltermaterial bzw. ein Schutzfiltermaterial bereitzustellen, welches die zuvor geschilderten Nachteile des Standes der Technik zumindest weitgehend vermeidet oder aber wenigstens abschwächt. Insbesondere soll sich ein solches Flächenfiltermaterial bzw. Schutzmaterial für zahlreiche Anwendungen im textil- bzw. bekleidungstechnischen Bereich eignen, beispielsweise für die Herstellung von Schutzausrüstungen bzw. Schutzgegenständen mit Schutzfunktion gegenüber chemischen bzw. biologischen bzw. radioaktiven Schad- bzw. Giftstoffen sowie von Bekleidung bzw. Ausrüstungen für den Sport- bzw. Freizeitbereich. Zudem soll sich das erfindungsgemäße Merkmal zur Herstellung von Filtern und Filtermaterialien eignen.

Eine weitere Aufgabe der vorliegenden Erfindung liegt zudem darin, ein insbesondere Flächenfiltermaterial bzw. Schutzmaterial bereitzustellen, welches eine wirksame Schutzfunktion in Bezug auf chemische, biologische bzw. radioaktive Schad- und Giftstoffe mit einem darüber hinausgehenden Schutz gegenüber entsprechenden schädlichen Partikeln und/oder Aerosolen bereitstellt. In diesem Zusammenhang soll insbesondere ein entsprechendes Flächenfiltermaterial mit integriertem Partikel- bzw. Aerosolschutz bereitgestellt werden.

Zudem ist eine nochmals weitere Aufgabe der vorliegenden Erfindung auch darin zu sehen, ein entsprechendes Flächenfiltermaterial bzw. Schutzmaterial bereitzustellen, welches einen verbesserten Tragekomfort insbesondere im Hinblick auf die Bereitstellung einer guten Thermo- bzw. Klimaregulation im Anwendungs- bzw. Tragezustand (beispielsweise in Bezug auf aus dem Material hergestellte Schutzanzüge bzw. Bekleidung für den Sport- bzw. Freizeitbereich) aufweist. In diesem Zusammenhang soll bei insgesamt hoher Wasserdampf- bzw. Luftdurchlässigkeit auch ein effektiver Abtransport von Feuchtigkeit bzw. Körperschweiß sowie windabweisende Eigenschaften bereitgestellt werden. Zudem soll das Material einen gewissen Nässeschutz aufweisen.

Eine nochmals weitere Aufgabe der vorliegenden Erfindung ist zudem darin zu sehen, ein entsprechendes Flächenfiltermaterial bzw. Schutzmaterial bereitzustellen, welches sich insbesondere zur Verwendung in Schutzausrüstungen bzw. Schutzgegenständen bzw. Bekleidung für den Sport- bzw. Freizeitbereich eignet, wobei das Material einen hohen Tragekomfort im Hinblick auf ein gegenüber dem Stand der Technik verbessertes Tragegefühl gewährleisten soll.

Schließlich besteht eine wiederum weitere Aufgabe der vorliegenden Erfindung in der Bereitstellung eines Flächenfiltermaterials bzw. Schutzmaterials, welches sich insbesondere auch zur Verwendung in Filtern und Filtermaterialien (wie z. B. zur Entfernung von Schad-, Geruchs- und Giftstoffen aller Art, insbesondere aus Luft- bzw. Gasströmen, wie Schutzmaskenfilter, Geruchsfiltern, Flächenfiltern, Luftfiltern, Raumfiltern und Filtern für den medizinischen Bereich) eignet und dabei bei guter Durchströmbarkeit für das aufzureinigende Medium auch eine gute Filtereffizienz gewährleistet, und zwar insbesondere auch im Hinblick auf eine wirksame Entfernung von in dem aufzureinigenden Medium vorhandenen Partikeln bzw. Aerosolen.

Zudem soll das erfindungsgemäß bereitgestellt Flächenfiltermaterial bzw. Schutzmaterial eine hohe Dauerhaftigkeit und mechanische Stabilität und zudem ein optimiertes bzw. geringes Flächengewicht aufweisen. Zudem sollen im Fall der Verwendung für bzw. als Schutzbekleidung die Trageeigenschaften bzw. der Tragekomfort im Hinblick auf das Tragegefühl bzw. die Anschmiegsamkeit des zugrundeliegenden Materials verbessert werden.

Zur Lösung der zuvor geschilderten Aufgabe schlägt die vorliegende Erfindung somit - gemäß einem **ersten** Aspekt der vorliegenden Erfindung - ein Flächenfiltermaterial mit Aerosol- bzw. Partikelfilterfunktion und mit Schutzfunktion gegenüber chemischen, biologischen und/oder radioaktiven Schad- bzw. Giftstoffen gemäß Patentanspruch 1 vor; jeweils vorteilhafte Weiterbildungen und Ausgestaltungen dieses Erfindungsaspekts sind Gegenstand der entsprechenden Unteransprüche.

Weiterer Gegenstand der vorliegenden Erfindung - gemäß einem **zweiten** Aspekt der vorliegenden Erfindung - ist zudem ein Verfahren zur Herstellung des erfindungsgemäßen Flächenfiltermaterials gemäß dem diesbezüglichen unabhängigen Verfahrensanspruch.

Ein weiterer Gegenstand der vorliegenden Erfindung - gemäß einem **dritten** Aspekt der vorliegenden Erfindung - ist zudem die Verwendung des erfindungsgemäßen Flächenfiltermaterials zur Herstellung von Schutzausrüstungen bzw. Schutzgegenständen aller Art bzw. zur Herstellung von Sport- bzw. Freizeitbekleidung und/oder -ausrüstung bzw. zur Herstellung von Filtern und Filtermaterialien aller Art gemäß den jeweiligen unabhängigen Verwendungsansprüchen.

Weiterer Gegenstand der vorliegenden Erfindung - gemäß einem **vierten** Aspekt der vorliegenden Erfindung - sind zudem die erfindungsgemäßen Schutzausrüstungen bzw. Schutzgegenstände bzw. die erfindungsgemäßen Sport- und/oder Freizeitbekleidungen und/oder -ausrüstungen, welche das erfindungsgemäße Flächenfiltermaterial enthalten bzw. unter Verwendung des Flächenfiltermaterials nach der Erfindung hergestellt sind, gemäß dem diesbezüglichen unabhängigen, die erfindungsgemäßen Schutzausrüstungen bzw. Schutzgegenstände sowie die Sport- und/oder Freizeitbekleidungen bzw. -ausrüstungen betreffenden unabhängigen Anspruch.

Schließlich sind weiterer Gegenstand der vorliegenden Erfindung - gemäß einem **fünften** Aspekt der vorliegenden Erfindung - auch Filter und Filtermaterialien, welche das erfindungsgemäße Flächenfiltermaterial aufweisen bzw. welche unter Verwendung des erfindungsgemäßen Flächenfiltermaterials hergestellt sind, gemäß dem diesbezüglichen unabhängigen, die Filter bzw. Filtermaterialien nach der Erfindung betreffenden Anspruch.

Es versteht sich von selbst, dass bei der nachfolgenden Beschreibung der vorliegenden Erfindung solche Ausgestaltungen, Ausführungsformen, Vorteile, Beispiele oder dergleichen, welche nachfolgend - zu Zwecken der Vermeidung unnötiger Wiederholungen - nur zu einem einzelnen Erfindungsaspekt ausgeführt werden, selbstverständlich auch in Bezug auf die übrigen Erfindungsaspekte entsprechend gelten, ohne dass es hierzu einer ausdrücklichen Erwähnung bedarf.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von den angegebenen Bereichen bzw. Angaben abgewichen werden kann, ohne dass der Rahmen der vorliegenden Erfindung verlassen ist.

Zudem gilt, dass sämtliche im Folgenden genannten Werte- bzw. Parameterangaben oder dergleichen grundsätzlich mit genormten bzw. standardisierten oder explizit angegebenen Bestimmungsverfahren oder aber anderenfalls mit dem Fachmann auf diesem Gebiet an sich geläufigen Bestimmungs- bzw. Messmethoden ermittelt bzw. bestimmt werden können. Sofern nicht anders angegeben, werden die zugrundeliegenden Werte bzw. Parameter unter Standardbedingungen (d.h. insbesondere bei einer Temperatur von 20 °C und/oder bei einem Druck von 1.013,25 hPa bzw. 1,01325 bar) ermittelt.

Im Übrigen gilt, dass bei sämtlichen nachstehend aufgeführten relativen bzw. prozentualen, insbesondere gewichtsbezogenen Mengenangaben zu beachten ist, dass diese Angaben im Rahmen der vorliegenden Erfindung vom Fachmann derart auszuwählen bzw. zu kombinieren sind, dass in der Summe - gegebenenfalls unter Einbeziehung weiterer Komponenten bzw. Inhaltsstoffe, insbesondere wie nachfolgend definiert - stets 100 % bzw. 100 Gew.-% resultieren. Dies versteht sich für den Fachmann aber von selbst.

Dies vorausgeschickt, wird im Folgenden die vorliegende Erfindung näher beschrieben und erläutert, und zwar auch anhand von bevorzugte Ausführungsformen bzw. Ausführungsbeispiele darstellenden Zeichnungen bzw. Figurendarstellungen.

Gegenstand der vorliegenden Erfindung - gemäß einem **ersten** Aspekt der vorliegenden Erfindung - ist somit ein Flächenfiltermaterial (synonym auch als Schutzmaterial bezeichnet) mit Aerosol- bzw. Partikelfilterfunktion und mit Schutzfunktion gegenüber chemischen, biologischen bzw. radioaktiven (nuklearen) Schad- und Giftstoffen, wobei das Flächenfiltermaterial umfasst:
(a) ein luft- bzw. wasserdampfdurchlässiges, flächiges schaumbasiertes bzw. schaumförmiges Trägermaterial mit zwei gegenüberliegenden Flachseiten, wobei das Trägermaterial (2) eine luftdurchlässige und/oder diskontinuierlich ausgebildete Schicht auf Basis einer getrockneten oder ausgehärteten, vorzugsweise vernetzten, gebrochenen Polymerschaumstruktur ist, wobei die gebrochene Polymerschaumstruktur offenporig und/oder nicht geschlossen ausgebildet ist;
(b) eine dem schaumbasierten und/oder schaumförmigen Trägermaterial zugeordnete bzw. an einer (ersten) Flachseite des schaumbasierten und/oder schaumförmigen Trägermaterials fixierte bzw. zum Haften gebrachte luft- bzw. wasserdampfdurchlässige, vorzugsweise flächige Nanofaserschicht, insbesondere Aerosol- bzw. Partikelfilter-Nanofaserschicht, wobei die Nanofaserschicht eine Vielzahl von Nanofasern umfasst bzw. hieraus besteht;
wobei das Flächenfiltermaterial weiterhin mindestens eine Adsorptionsschicht aufweist, wobei die Adsorptionsschicht eine Vielzahl einzelner und/oder diskreter Adsorberpartikel umfasst oder hieraus gebildet ist, wobei die Adsorptionsschicht zwei gegenüberliegende Flachseiten aufweist, wobei die Adsorptionsschicht dem Trägermaterial zugeordnet ist und wobei die Adsorptionsschicht auf der der Nanofaserschicht abgewandten (zweiten) Flachseite des Trägermaterials angeordnet ist.

Erfindungsgemäß wird somit auf ein spezielles Flächenfiltermaterial bzw. Schutzmaterial abgestellt, welches eine insbesondere unmittelbar bzw. direkt auf ein luft- bzw. wasserdampfdurchlässiges schaumbasiertes bzw. schaumförmiges Trägermaterial aufgebrachte Nanofaserschicht aufweist, wobei die Nanofaserschicht eine Vielzahl von Nanofasern umfasst bzw. hieraus besteht. Erfindungsgemäß kann es, wie nachfolgend noch angeführt, insbesondere vorgesehen sein, dass das Flächenfiltermaterial nach der Erfindung zudem als Schicht (c) mindestens einen textilen Träger aufweist, welcher insbesondere auf der der Nanofaserschicht abgewandten Seite des schaumbasierten bzw. schaumförmigen Trägermaterials angeordnet ist und auf welches wiederum das schaumbasierte bzw. schaumförmige Trägermaterial aufgebracht ist. Demnach kann es sich erfindungsgemäß gleichermaßen um ein textiles Flächenfiltermaterial bzw. ein textiles Schutzmaterial handeln.

Im Rahmen der vorliegenden Erfindung wird durch die gezielte Kombination eines luft- bzw. wasserdampfdurchlässigen schaumbasierten bzw. schaumförmigen Trägermaterials mit einer hierauf aufgebrachten bzw. hieran fixierten Nanofaserschicht insgesamt ein Flächenfiltermaterial bereitgestellt, welches bei hoher Luft- bzw. Wasserdampfdurchlässigkeit eine wirksame Schutzfunktion gegenüber chemischen, biologischen bzw. radioaktiven Schad- bzw. Giftstoffen aufweist, wobei das erfindungsgemäße Flächenfiltermaterial in diesem Zusammenhang insbesondere eine sozusagen integrierte Aerosol- bzw. Partikelfilterfunktion bzw. eine diesbezügliche Schutzfunktion auch gegenüber Aerosolen und Partikeln aufweist.

In diesem Zusammenhang hat die Anmelderin in völlig überraschender Weise gefunden, dass bei gleichzeitiger Bereitstellung einer hohen Luftdurchlässigkeit bzw. Wasserdampfdurchlässigkeit eine effiziente Schutzfunktion gegenüber den in Rede stehenden Schad- bzw. Giftstoffen, insbesondere chemischen Kampfstoffen, bereitgestellt werden kann, indem in zweckgerichteter Art und Weise hinsichtlich des erfindungsgemäßen Flächenfiltermaterials eine spezielle Schichtstruktur bzw. ein spezieller Schichtaufbau vorgesehen ist, wonach nämlich, wie zuvor angeführt, eine eine Vielzahl von Nanofasern umfassende bzw. hieraus bestehende Nanofaserschicht auf ein luft- bzw. wasserdampfdurchlässiges schaumbasiertes bzw. schaumförmiges Trägermaterial aufgebracht bzw. hieran fixiert ist, was im Ergebnis zu hervorragenden Aerosol- und Partikelfiltereigenschaften führt.

Dabei hat die Anmelderin gleichermaßen überraschend gefunden, dass sich in Bezug auf das erfindungsgemäße, insbesondere schichtförmig ausgebildete Flächenfiltermaterial die jeweiligen Filtereigenschaften der Nanofaserschicht einerseits und des schaumbasierten bzw. schaumförmigen Trägermaterials andererseits komplementär ergänzen, wobei in diesem Zusammenhang die durch das erfindungsgemäße Flächenfiltermaterial insgesamt bereitgestellte Schutzfunktion gegenüber den in Rede stehenden Schad- bzw. Giftstoffen über die Summe der Filtereigenschaften der jeweiligen Einzelkomponenten bzw. -schichten hinausgeht, so dass hinsichtlich der erfindungsgemäß bereitgestellten Aerosol- bzw. Partikelfilterfunktion ein synergistisches Zusammenwirken der jeweiligen Schichten in Form der Nanofaserschicht einerseits und des schaumbasierten bzw. schaumförmigen Trägermaterials andererseits vorliegt. Ohne sich in diesem Zusammenhang auf diese Theorie beschränken oder festlegen zu wollen, führt der zweckgerichtete Einsatz des luft- bzw. wasserdampfdurchlässigen schaumbasierten bzw. schaumförmigen Trägermaterials insgesamt auch zu einer verbesserten Anströmung bzw. Durchströmung der darauf fixierten Nanofaserschicht mit dem aufzureinigendem Medium, insbesondere Luft, und damit auch zu einer optimierten Ausnutzung der durch die Nanofaserschicht bereitgestellten Aerosol- bzw. Partikelfilterfunktion, wobei es sich erfindungsgemäß gleichermaßen so verhalten kann, dass das luft- bzw. wasserdampfdurchlässig ausgebildete schaumbasierte bzw. schaumförmige Trägermaterial eine ergänzende bzw. eigenständige Aerosol- bzw. Partikelfilterfunktion aufweist.

Im Ergebnis können somit in einem aufzureinigenden Medium, insbesondere Luft, vorhandene Aerosole bzw. Partikel im hohen Maße von dem erfindungsgemäßen Flächenfiltermaterial aufgenommen werden, einhergehend mit einer entsprechend hohen Aufreinigung des aufzureinigenden Mediums, insbesondere Luft.

Diesbezüglich kommt auch der erfindungsgemäßen Konzeption eine große Bedeutung zu, wonach es nämlich insbesondere vorgesehen ist, die Nanofaserschicht unmittelbar bzw. direkt (d. h. ohne dazwischen angeordnete weitere Schichten bzw. ohne dazwischen angeordnete andersartige Materialien bzw. Komponenten, wie z. B. Adsorptionsmaterialien) auf das luft- bzw. wasserdampfdurchlässige schaumbasierte bzw. schaumförmige Trägermaterial aufzubringen bzw. hieran zu fixieren, da hierdurch eine optimale Wirkverstärkung der jeweiligen Schichten gewährleistet ist, und zwar auch was das verbesserte An- bzw. Durchströmungsverhalten der eine Vielzahl von Nanofasern enthaltenen Nanofaserschicht anbelangt.

Infolge des gezielten Zusammenwirkens der in Rede stehenden Schichten in Form des luft- bzw. wasserdampfdurchlässigen schaumbasierten bzw. schaumförmigen Trägermaterials einerseits und der Nanofaserschicht andererseits kann in Bezug auf das erfindungsgemäße Flächenfiltermaterial die Materialdicke insgesamt verringert werden, was zudem auch der Luft- bzw. Wasserdampfdurchlässigkeit bzw. Atmungsaktivität sowie dem resultierenden Flächengewicht insgesamt zuträglich ist. Auf dieser Basis wird auch die Trageeigenschaften bzw. der Tragekomfort von aus dem Flächenfiltermaterial hergestellten Bekleidungsstücken bzw. Ausrüstungsgegenständen verbessert. Zudem geht das erfindungsgemäße Flächenfiltermaterial insbesondere im Hinblick auf seine Verwendung für (Schutz-)-Bekleidungsstücke mit einem hohen Tragekomfort einher, und zwar dahingehend, dass nicht zuletzt aufgrund der hohen Luft- bzw. Wasserdampfdurchlässigkeit bzw. Atmungsaktivität des Materials eine effiziente Thermo- bzw. Klimaregulation ermöglicht wird, beispielsweise da Feuchtigkeit bzw. Körperschweiß effizient von einem Träger entsprechender (Schutz-)Bekleidungsstücke entfernt bzw. abtransportiert werden kann. Aufgrund der speziellen Ausbildung des erfindungsgemäßen Flächenfiltermaterials wird bzw. werden dabei trotz der hohen Luftdurchlässigkeit auch ein hoher Windschutz bzw. windabweisende Eigenschaften bereitgestellt, was den Tragekomfort insbesondere hinsichtlich der zugrundeliegenden Thermo- bzw. Klimaregulation weiterführend verbessert. Insbesondere wird auf diese Weise, beispielsweise bei sehr niedrigen Umgebungstemperaturen bzw. bei hohen Windstärken, einem Auskühlen entgegengewirkt.

Erfindungsgemäß ist es insbesondere vorgesehen, dass das Flächenfiltermaterial nach der Erfindung als insbesondere schichtartiges bzw. schichtförmiges Verbundmaterial vorliegt oder dass das Flächenfiltermaterial selbsttragend ausgebildet ist. Auch infolgedessen weist das erfindungsgemäße Flächenfiltermaterial eine hohe Robustheit sowie Abriebfestigkeit auf.

Aufgrund der speziellen Materialien bzw. Komponenten des erfindungsgemäßen Flächenfiltermaterials resultiert zudem ein hervorragendes Tragegefühl beispielsweise von daraus bzw. damit hergestellter (Schutz-)Bekleidung. Dabei weist das erfindungsgemäße Flächenfiltermaterial bei geringem Flächengewicht auch hervorragende Eigenschaften hinsichtlich seiner Flexibilität bzw. seines Biegeverhaltens auf, und zwar insbesondere auch von daher, dass insbesondere auch das schaumbasierte bzw. schaumförmige Trägermaterial als solches flexibel bzw. reversibel dehnbar (elastisch) ausgebildet ist, was grundsätzlich auch für die erfindungsgemäß vorgesehene Nanofaserschicht als solche gilt. Zudem weist die insbesondere in Form eines Vlieses vorliegende Nanofaserschicht als auch das schaumbasierte bzw. schaumförmige Trägermaterial und damit das erfindungsgemäße Flächenfiltermaterial insgesamt eine hohe Anschmiegsamkeit und aufgrund der jeweiliger Struktur der Nanofaserschicht bzw. des Trägermaterials auch entsprechend gute haptische Eigenschaften auf, was den Tragekomfort weiterführend verbessert.

Zudem weist das erfindungsgemäße Flächenfiltermaterial auch wasserdichte bzw. wasserabweisende Eigenschaften auf, insbesondere da die spezielle Nanofaserschicht bzw. das Trägermaterial eine Barriere gegenüber Wasser in flüssiger Form darstellt, so dass das erfindungsgemäße Flächenfiltermaterial in Ergänzung zu den weiteren Eigenschaften auch einen gewissen Wasser- bzw. Regenschutz bzw. eine definierte Wasserdichtigkeit bzw. ein definiertes Wasserabweiseverhalten bereitstellt bzw. aufweist (beispielsweise eine Wassersäule von mindestens 500 mm, gemessen nach DIN EN 20 811:1992 (August 1992)). In diesem Zusammenhang kann das erfindungsgemäße Material beispielsweise für wasserabweisende (d.h. Wasser in flüssiger Form abweisende), aber atmungsaktive (d.h. wasserdampfdurchlässige und bevorzugt auch luftdurchlässige) Schutzausrüstung, Sport- bzw. Freizeitbekleidung bzw. -ausrüstung oder dergleichen, insbesondere auch für Schuhmaterialien oder Schuhwerk, eingesetzt werden.

Aufgrund der dem erfindungsgemäßen Flächenfiltermaterial zugrundeliegenden Eigenschaften kann das erfindungsgemäße Flächenfiltermaterial als solches universell eingesetzt werden. So kommt beispielsweise neben dem Einsatz im Rahmen des ABC-Schutzes bzw. des NBC-Schutzes (*nuclear, biological, chemical*) auch eine Verwendung im Sport- bzw. Freizeitbereich in Frage, insbesondere auch infolge des hohen Tragekomforts mit der bereitgestellten Luft- und Wasserdampfdurchlässigkeit bei gleichzeitig hohem Windschutz und Schutz gegenüber Wasser in flüssiger Form, wie zuvor angeführt. Zudem eignet sich das Flächenfiltermaterial nach der Erfindung für Filteranwendungen, beispielsweise für Raumluftfilter oder dergleichen.

Insgesamt wird somit im Rahmen der vorliegenden Erfindung ein Flächenfiltermaterial mit hoher Luftdurchlässigkeit bzw. Wasserdampfdurchlässigkeit bereitgestellt, welches sich aufgrund seiner hervorragenden Schutzfunktion gegenüber Schad- bzw. Giftstoffen infolge einer diesbezüglich bereitgestellten Aerosol- bzw. Partikelfilterfunktion für den Einsatz im Bereich des ABC-Schutzes bzw. NBC-Schutzes eignet, wobei sich darüber hinaus und nicht zuletzt aufgrund des dem Material zugrundeliegenden Wind- und Nässeschutzes auch eine Verwendung im Sport- bzw. Freizeitbereich, insbesondere zur Herstellung von Sport- bzw. Freizeitbekleidung bzw. -ausrüstung, anbietet.

Die Schutzfunktion des erfindungsgemäßen Flächenfiltermaterial gegenüber Partikeln und Aerosolen ist vergleichbar mit herkömmlichen Membransystemen; jedoch ist aufgrund der Luftdurchlässigkeit bzw. Wasserdampfdurchlässigkeit des erfindungsgemäßen Flächenfiltermaterials der Tragekomforts bei der Verarbeitung zu ABC-Schutzanzügen oder dergleichen im Vergleich zu solchen Schutzanzügen, welche eine Membran aufweisen, deutlich erhöht.

Was die vorliegende Erfindung weiterhin anbelangt, so zeichnet sich die vorgesehene flächige Nanofaserschicht insbesondere durch den Einsatz bzw. das Vorhandensein einer Vielzahl von Nanofasern aus. Was in diesem Zusammenhang den Begriff "Nanofasern" anbelangt, wie er erfindungsgemäß verwendet wird, so ist dieser im Rahmen der vorliegenden Erfindung breit zu verstehen. Insbesondere bezieht sich der in Rede stehende Begriff auf spezielle Fasern, welche sich insbesondere durch die nachfolgend noch angeführten bzw. definierten Eigenschaften auszeichnen, und zwar insbesondere was spezielle Faserdurchmesser, spezielle Materialien sowie die den Nanofasern zugrundeliegende Herstellung anbelangt. Hierzu kann auf nachfolgende Ausführungen verwiesen werden.

Im Nachfolgenden wird die vorliegende Erfindung anhand von bevorzugte Ausführungsformen bzw. Ausführungsformen darstellenden Zeichnungen bzw. Figurendarstellungen beschrieben, wobei die diesbezüglichen Ausführungen für sämtliche erfindungsgemäße Aspekte gelten und wobei die entsprechenden bevorzugten Ausführungsformen keinesfalls beschränkend sind.

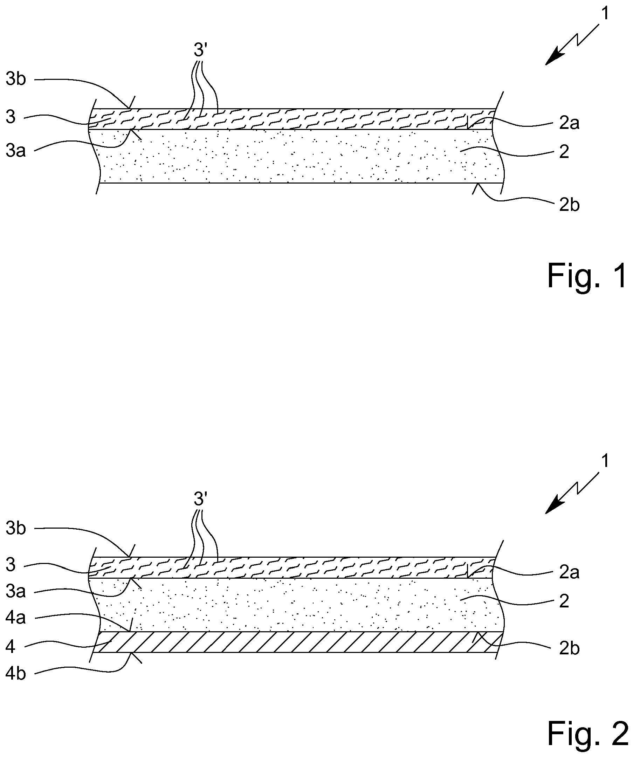

In den Figurendarstellungen zeigt
- Fig. 1: eine schematische Querschnittsdarstellung eines Flächenfiltermaterials, wobei das Flächenfiltermaterial ein luft- bzw. wasserdampfdurchlässiges schaumbasiertes bzw. schaumförmiges Trägermaterial sowie eine dem Trägermaterial zugeordnete bzw. an einer (ersten) Seite, insbesondere Flachseite, des Trägermaterials fixierte bzw. zum Haften gebrachte luft- bzw. wasserdampfdurchlässige Nanofaserschicht aufweist;
- Fig. 2: eine Querschnittsdarstellung eines Flächenfiltermaterials wonach das Flächenfiltermaterial zusätzlich einen textilen Träger aufweist, welcher insbesondere auf der der Nanofaserschicht abgewandten Seite des schaumbasierten bzw. schaumförmigen Trägermaterials angeordnet ist;

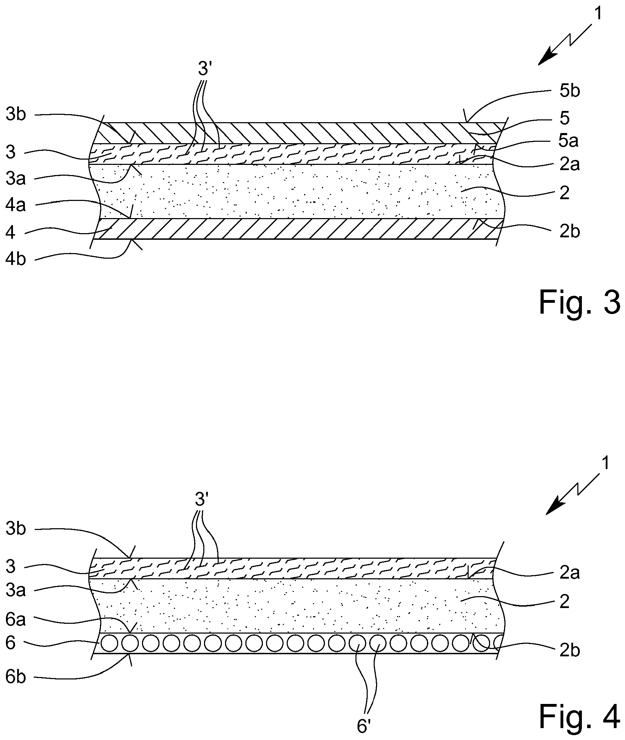

- Fig. 3: eine Querschnittsdarstellung eines Flächenfiltermaterials wonach das Flächenfiltermaterial neben dem schaumbasierten bzw. schaumförmigen Trägermaterial und der Nanofaserschicht sowie dem textilen Träger eine zusätzliche Abdeckschicht aufweist, welche insbesondere auf der dem schaumbasierten bzw. schaumförmigen Trägermaterial abgewandten Seite der Nanofaserschicht angeordnet ist;
- Fig. 4: eine Querschnittsdarstellung eines erfindunasaemäßen Flächenfiltermaterials gemäß einer eines erfindungsgemäßen Ausführungsform der vorliegenden Erfindung, wonach das Flächenfiltermaterial zusätzlich mit einer Adsorptionsschicht versehen ist, insbesondere wobei die Adsorptionsschicht auf der der Nanofaserschicht abgewandten Seite des schaumbasierten bzw. schaumförmigen Trägermaterials angeordnet ist.

Die die vorliegende Erfindung betreffende Figurendarstellung gemäß Fig. 4 verdeutlicht dabei insbesondere auch den ersten Aspekt der vorliegenden Erfindung, wonach nämlich ein Flächenfiltermaterial 1 mit Aerosol- bzw. Partikelfilterfunktion und mit Schutzfunktion gegenüber chemischen, biologischen und/oder radioaktiven Schad- bzw. Giftstoffen, bereitgestellt wird, wobei das Flächenfiltermaterial 1 umfasst:
(a) ein luft- und/oder wasserdampfdurchlässiges, flächiges schaumbasiertes und/oder schaumförmiges Trägermaterial 2 mit zwei gegenüberliegenden Flachseiten 2a, 2b, wobei das Trägermaterial (2) eine luftdurchlässige und/oder diskontinuierlich ausgebildete Schicht auf Basis einer getrockneten oder ausgehärteten, vorzugsweise vernetzten, gebrochenen Polymerschaumstruktur ist, wobei die gebrochene Polymerschaumstruktur offenporig und/oder nicht geschlossen ausgebildet ist;
(b) eine dem schaumbasierten und/oder schaumförmigen Trägermaterial 2 zugeordnete und/oder an einer (ersten) Flachseite (2a) des schaumbasierten und/oder schaumförmigen Trägermaterials 2 fixierte bzw. zum Haften gebrachte luft- und/oder wasserdampfdurchlässige, vorzugsweise flächige Nanofaserschicht 3, insbesondere Aerosol- und/oder Partikelfilter-Nanofaserschicht, wobei die Nanofaserschicht 3 eine Vielzahl von Nanofasern 3' umfasst oder hieraus besteht;
wobei das Flächenfiltermaterial (1) weiterhin mindestens eine Adsorptionsschicht (6) aufweist, wobei die Adsorptionsschicht (6) eine Vielzahl einzelner und/oder diskreter Adsorberpartikel (6') umfasst oder hieraus gebildet ist, wobei die Adsorptionsschicht (6) zwei gegenüberliegende Flachseiten (6a, 6b) aufweist, wobei die Adsorptionsschicht (6) dem Trägermaterial (2) zugeordnet ist und wobei die Adsorptionsschicht (6) auf der der Nanofaserschicht (3) abgewandten (zweiten) Flachseite (2b) des Trägermaterials (2) angeordnet ist.

Wie zuvor angeführt, wird im Rahmen der vorliegenden Erfindung insgesamt ein Flächenfiltermaterial bereitgestellt, welches die diametralen Eigenschaften eines hohen Tragekomforts, welcher insbesondere infolge einer hohen Luft- und/oder Wasserdampfdurchlässigkeit gewährleistet wird, einerseits und einer hohen Schutzfunktion gegenüber Schad- bzw. Giftstoffen, insbesondere Kampfstoffen, andererseits in ein und demselben Material vereint, wobei das erfindungsgemäße Flächenfiltermaterial aufgrund seiner speziellen Eigenschaften zudem insbesondere für den Fall seiner Verwendung in oder als (Schutz-)Bekleidung oder dergleichen ein hervorragendes Tragegefühl bei gleichzeitig guter Klima- bzw. Temperaturregulation aufweist. Die speziellen Eigenschaften und Vorteile des erfindungsgemäßen Flächenfiltermaterials resultieren dabei aus der erfindungsgemäßen Konzeption, wonach nämlich in zweckgerichteter Weise ein insgesamt luft- bzw. wasserdampfdurchlässiges schichtartiges bzw. schichtförmiges Flächenfiltermaterial auf Basis einer speziellen Kombination eines schaumbasierten bzw. schaumförmigen Trägermaterials mit einer speziellen Nanofaserschicht bereitgestellt wird.

Weiterhin kann es erfindungsgemäß vorgesehen sein, dass das Trägermaterial 2 ein Flächengewicht im Bereich von 5 g/m² bis 500 g/m², insbesondere im Bereich von 10 g/m² bis 300 g/m², vorzugsweise im Bereich von 15 g/m² bis 200 g/m², bevorzugt im Bereich von 20 g/m² bis 150 g/m², besonders bevorzugt im Bereich von 25 g/m² bis 100 g/m², ganz besonders bevorzugt im Bereich von 25 g/m² bis 50 g/m², aufweist.

Insbesondere kann das Trägermaterial 2 eine Dicke von höchstens 10 mm, insbesondere höchstens 5 mm, vorzugsweise höchstens 4 mm, bevorzugt höchstens 3 mm, besonders bevorzugt höchstens 1 mm, ganz besonders bevorzugt höchstens 0,5 mm, aufweisen. Insbesondere kann das Trägermaterial 2 eine Dicke im Bereich von 0,01 mm bis 10 mm, insbesondere im Bereich von 0,03 mm bis 5 mm, vorzugsweise im Bereich von 0,05 mm bis 4 mm, bevorzugt im Bereich von 0,08 mm bis 3 mm, besonders bevorzugt im Bereich von 0,09 mm bis 1 mm, ganz besonders bevorzugt im Bereich von 0,1 mm bis 0,5 mm, weiter bevorzugt im Bereich von 0,1 mm bis 0,3 mm, aufweisen.

In diesem Zusammenhang wird unter dem Begriff der Dicke des Trägermaterials 2 insbesondere der Abstand der (ersten) Seite 2a zu der (zweiten) Seite 2b des Trägermaterials 2 verstanden.

Erfindungsgemäß kann es insbesondere vorgesehen sein, dass das Trägermaterial 2 eine Dichte im Bereich von 50 g/l bis 500 g/l, insbesondere im Bereich von 100 g/l bis 400 g/l, vorzugsweise im Bereich von 150 g/l bis 350 g/l, bevorzugt im Bereich von 200 g/l bis 325 g/l, aufweist.

Wie zuvor angeführt, zeichnet sich das erfindungsgemäß eingesetzte Trägermaterial 2 durch eine hohe Luftdurchlässigkeit aus, was wiederum das Anström- bzw. Durchströmverhalten in Bezug auf die erfindungsgemäß eingesetzte Nanofaserschicht 3 verbessert: Insbesondere kann es erfindungsgemäß vorgesehen sein, dass das Trägermaterial 2 eine Luftdurchlässigkeit von mindestens 100 l·m⁻²·s⁻¹, insbesondere mindestens 200 l·m⁻²·s⁻¹, vorzugsweise mindestens 300 l·m⁻²·s⁻¹, besonders bevorzugt mindestens 400 l·m⁻²·s⁻¹ , ganz besonders bevorzugt mindestens 600 l·m⁻²·s⁻¹, bei einem Strömungswiderstand von 127 Pa, aufweist.

Erfindungsgemäß wird im Allgemeinen unter einem schaumbasierten bzw. schaumförmigen (Träger-)Material insbesondere eine feste (d.h. getrocknete bzw. ausgehärtete, vorzugsweise vernetzte), jedoch flexible bzw. biegsame bzw. reversibel komprimierbare bzw. reversibel dehnbare Schaumstruktur (Schaumschicht) bzw. ein fester poröser Körper - d.h. insbesondere auf Basis einer Dispersion mit einem Feststoff als Dispersionsmedium und einem Gas als disperse Phase - verstanden. Für weitergehende Einzelheiten zum Begriff "Schaum" kann insbesondere verwiesen werden auf Römpp, Chemielexikon, 10. Auflage, Georg Thieme Verlag, Stuttgart / New York, Stichwort: "Schaum", Seiten 3950 und 3951 sowie die darin referierte Literatur, deren diesbezüglicher gesamter Inhalt hiermit durch Bezugnahme vollumfänglich eingeschlossen ist.

Erfindungsgemäß ist es vorgesehen, dass das schaumbasierte und/oder schaumförmige Trägermaterial 2 eine getrocknete und/oder ausgehärtete, vorzugsweise vernetzte, gebrochene Schaumstruktur (synonym auch als gebrochene Schaumschicht bzw. gebrochener Schaum bezeichnet) in Form einer gebrochenen Polymerschaumstruktur (synonym auch als gebrochene Polymerschaumschicht bzw. gebrochener Polymerschaum bezeichnet) umfasst oder hieraus besteht. Mit anderen Worten kann es erfindungsgemäß insbesondere vorgesehen sein, als Trägermaterial 2, insbesondere auf Grundlage des schaumbasierten und/oder schaumförmigen Trägermaterials, eine gebrochene Schaumstruktur in Form einer gebrochenen Polymerschaumstruktur einzusetzen. In diesem Zusammenhang ist das schaumbasierte und/oder schaumförmige Trägermaterial 2 somit eine luftdurchlässige und/oder diskontinuierlich ausgebildete Schicht auf Basis bzw. in Form einer getrockneten bzw. ausgehärteten, vorzugsweise vernetzten, gebrochenen Polymerschaumstruktur.

Gemäß der vorliegenden Erfindung liegt somit das schaumbasierte bzw. schaumförmige Trägermaterial 2 als gebrochene Schaumstruktur bzw. Polymerschaumstruktur vor. Im Rahmen der vorliegenden Erfindung ist der Begriff "gebrochen" (synonym auch als "zerfallen" bzw. "zerstört" bezeichnet), wie er in Bezug auf die getrocknete bzw. ausgehärtete, insbesondere vernetzte, Schaumstruktur bzw. Polymerschaumstruktur bzw. das schaumbasierte bzw. schaumförmige Trägermaterial als solches gemäß der vorliegenden Ausführungsform verwendet wird, insbesondere so zu verstehen, dass es sich bei der gebrochenen Schaumstruktur, insbesondere Polymerschaumstruktur, im Rahmen der vorliegenden Erfindung gleichermaßen um ein insgesamt luftdurchlässiges System handelt, welches - insbesondere infolge einer speziellen Verfahrensführung zur Herstellung des Materials ausgehend von einer zuvor aufgeschäumten nicht getrockneten Dispersion bzw. Lösung, welche beispielsweise auf einem textilen Träger 4 bzw. einer entfernbaren Trägerschicht, wie nachfolgend definiert, aufgebracht werden kann, wobei nach folgender Trocknung bzw. Aushärtung eine Brechung bzw. Zerstörung der Schaumstrukturen vorgenommen werden kann - im getrockneten bzw. ausgehärteten Zustand eine Vielzahl zerstörter bzw. geplatzter bzw. kollabierter Schaumblasen aufweist. Dementsprechend kann in Bezug auf die getrocknete bzw. ausgehärtete, insbesondere vernetzte, gebrochene Schaumstruktur, insbesondere Polymerschaumstruktur, bzw. das schaumbasierte bzw. schaumförmige Trägermaterial 2 auch von einer zerfallenen Schaumstruktur (zerfallener Schaum) gesprochen werden. Dabei weist die gebrochene Schaumstruktur bzw. weisen die zugrundeliegenden zerstörten Schaumblasen eine Vielzahl an zerstörten bzw. gebrochenen bzw. kollabierten Wandungen, Lamellen bzw. Stegen aus dem der Schaumstruktur zugrundeliegenden Material, insbesondere Polymermaterial, insbesondere wie zuvor definiert, auf. Dabei verhält es sich zudem insbesondere derart, dass die so erhaltene Schaumstruktur gleichermaßen flexibel bzw. biegsam sowie reversibel komprimierbar bzw. reversibel dehnbar ist.

Infolge der offenporigen Schaumstruktur bzw. der zerstörten bzw. gebrochenen bzw. zerfallenen Schaumstruktur resultiert, wie nachfolgend noch angeführt, zudem eine Vielzahl an Durchbrechungen, Poren, Kanälen bzw. Öffnungen in dem zugrundeliegenden Schaumsystem des Trägermaterials 2, welche sich insbesondere durch die gesamte Schichtdicke des Trägermaterials 2 ziehen, was zu der im Rahmen der Brechung bzw. des Zerfalls der Schaumstruktur bzw. der Offenporigkeit der Schaumstruktur entstehenden verbesserten Luftdurchlässigkeit des Trägermaterials 2 insgesamt führt.

Erfindungsgemäß verhält es sich dabei insbesondere derart, dass die gebrochene Polymerschaumstruktur eine Vielzahl getrockneter und/oder ausgehärteter, insbesondere vernetzter, zerstörter und/oder geplatzter bzw. kollabierter Schaumblasen aufweist.

In diesem Zusammenhang kann es erfindungsgemäß vorgesehen sein, dass die gebrochene Polymerschaumstruktur, bevorzugt die getrockneten und/oder ausgehärteten, insbesondere vernetzten, zerstörten und/oder geplatzten und/oder kollabierten Schaumblasen des Trägermaterials 2, insbesondere der gebrochenen Schaumstruktur, vorzugsweise der gebrochenen Polymerschaumstruktur, eine Vielzahl an zerstörten und/oder gebrochenen und/oder kollabierten Wandungen und/oder Stegen insbesondere aus Schaumstrukturmaterial (Schaumstrukturpolymer) aufweist bzw. aufweisen.

Insbesondere kann es erfindungsgemäß vorgesehen sein, dass die gebrochene Polymerschaumstruktur einen Anteil an zerstörten und/oder geplatzten und/oder kollabierten Schaumblasen von mindestens 10 %, insbesondere mindestens 30 %, vorzugsweise mindestens 50 %, bevorzugt mindestens 70 %, besonders bevorzugt mindestens 90 %, ganz besonders bevorzugt mindestens 95 %, bezogen auf die Gesamtanzahl an Schaumblasen in dem Trägermaterial 2, insbesondere der gebrochenen Schaumstruktur, vorzugsweise der gebrochene Polymerschaumstruktur, aufweist.

Gemäß einer erfindungsgemäßen Ausführungsform kann es zudem vorgesehen sein, dass die gebrochene Polymerschaumstruktur einen Anteil an zerstörten und/oder geplatzten und/oder kollabierten Schaumblasen im Bereich von 10 % bis 100 %, insbesondere im Bereich von 30 % bis 99,9 %, vorzugsweise im Bereich von 50 % bis 99 %, bevorzugt im Bereich von 70 % bis 99 %, besonders bevorzugt im Bereich von 90 % bis 98 %, bezogen auf die Gesamtanzahl an Schaumblasen in dem Trägermaterial 2, insbesondere in der gebrochenen Schaumstruktur, vorzugsweise in der gebrochenen Polymerschaumstruktur, aufweist.

Erfindungsgemäß kann es in diesem Zusammenhang insbesondere vorgesehen sein, dass die gebrochene Polymerschaumstruktur nicht geschlossen bzw. offenporig ausgebildet ist bzw. dass die gebrochene Polymerschaumstruktur eine Vielzahl von insbesondere sich in der gebrochenen Polymerschaumstruktur erstreckenden Durchbrechungen, Poren, Kanälen bzw. Öffnungen bzw. eine Vielzahl von insbesondere die jeweiligen Flachseiten 2a, 2b des Trägermaterials 2 verbindenden Durchbrechungen, Poren, Kanälen und/oder Öffnungen aufweist. Hierdurch wird die erfindungsgemäß vorgesehene hohe Luftdurchlässigkeit bzw. Wasserdampfdurchlässigkeit des Trägermaterials weiterführend verbessert.

Im Allgemeinen kann es erfindungsgemäß vorgesehen sein, dass die gebrochene Polymerschaumstruktur im Vergleich zu einem entsprechenden nichtgeschäumten und/oder kontinuierlich ausgebildeten Trägermaterial, insbesondere im Vergleich zu einem entsprechenden nichtgeschäumten und/oder kontinuierlich ausgebildeten Schaumstrukturmaterial (Schaumstrukturpolymer), bevorzugt im Vergleich zu einer entsprechenden nichtgeschäumten und/oder kontinuierlich ausgebildeten Schaumstruktur bzw. Polymerschaumstruktur , eine bzw. ein um mindestens 5 %, insbesondere mindestens 10%, vorzugsweise mindestens 15 %, bevorzugt mindestens 20 %, besonders bevorzugt mindestens 25 %, verringerte Dichte und/oder verringertes insbesondere flächenbezogenes Volumengewicht aufweist, bezogen auf das nichtgeschäumte und/oder kontinuierlich ausgebildete Trägermaterial, insbesondere bezogen auf das nichtgeschäumte und/oder kontinuierlich ausgebildete Schaumstrukturmaterial, bevorzugt bezogen auf die nichtgeschäumte und/oder kontinuierlich ausgebildete Schaumstruktur bzw. Polymerschaumstruktur.

Zudem kann die gebrochene Polymerschaumstruktur im Vergleich zu einem entsprechenden nichtgeschäumten und/oder kontinuierlich ausgebildeten Trägermaterial, insbesondere im Vergleich zu einem entsprechenden nichtgeschäumten und/oder kontinuierlich ausgebildeten Schaumstrukturmaterial (Schaumstrukturpolymer), eine bzw. ein im Bereich von 5 % bis 80 %, insbesondere im Bereich von 10 % bis 70 %, vorzugsweise im Bereich von 15 % bis 60 %, bevorzugt im Bereich von 20 % bis 55 %, verringerte Dichte und/oder verringertes insbesondere flächenbezogenes Volumengewicht aufweisen, bezogen auf das nichtgeschäumte und/oder kontinuierlich ausgebildete Trägermaterial, insbesondere bezogen auf das nichtgeschäumte und/oder kontinuierlich ausgebildete Schaumstrukturmaterial.

Erfindungsgemäß kann es weiterhin vorgesehen sein, dass die gebrochene Polymerschaumstruktur im Vergleich zu einem entsprechenden nichtgeschäumten und/oder kontinuierlich ausgebildeten Trägermaterial, insbesondere im Vergleich zu einem entsprechenden nichtgeschäumten und/oder kontinuierlich ausgebildeten Schaumstrukturmaterial (Schaumstrukturpolymer), eine bzw. ein um höchstens 10 %, insbesondere höchstens 5 %, vorzugsweise höchstens 1 %, erhöhte Dichte und/oder erhöhtes insbesondere flächenbezogenes Volumengewicht aufweist, bezogen auf das nichtgeschäumte und/oder kontinuierlich ausgebildete Trägermaterial, insbesondere bezogen auf das nichtgeschäumte und/oder kontinuierlich ausgebildete Schaumstrukturmaterial.

Zudem ist es im Rahmen der vorliegenden Erfindung möglich, dass die gebrochene Polymerschaumstruktur im Vergleich zu einem entsprechenden nichtgeschäumten und/oder kontinuierlich ausgebildeten Trägermaterial, insbesondere im Vergleich zu einem entsprechenden nichtgeschäumten und/oder kontinuierlich ausgebildeten Schaumstrukturmaterial (Schaumstrukturpolymer), eine um höchstens 30 %, insbesondere höchstens 20 %, vorzugsweise höchstens 10%, bevorzugt höchstens 5 %, verringerte Elastizität und/oder reversible Dehnbarkeit aufweist, bezogen auf das nichtgeschäumte und/oder kontinuierlich ausgebildete Trägermaterial, insbesondere bezogen auf das nichtgeschäumte und/oder kontinuierlich ausgebildete Schaumstrukturmaterial.

Gleichermaßen kann die gebrochene Polymerschaumstruktur im Vergleich zu einem entsprechenden nichtgeschäumten und/oder kontinuierlich ausgebildeten Trägermaterial, insbesondere im Vergleich zu einem entsprechenden nichtgeschäumten und/oder kontinuierlich ausgebildeten Schaumstrukturmaterial (Schaumstrukturpolymer), eine im Bereich von 5 % bis 30 %, insbesondere im Bereich von 10 % bis 20 %, verringerte Elastizität und/oder reversible Dehnbarkeit aufweisen, bezogen auf das nichtgeschäumte und/oder kontinuierlich ausgebildete Trägermaterial, insbesondere bezogen auf das nichtgeschäumte und/oder kontinuierlich ausgebildete Schaumstrukturmaterial.

Im Allgemeinen verhält es sich im Rahmen der vorliegenden Erfindung derart, dass die gebrochene Polymerschaumstruktur zusammenhängend bzw. kohärent ausgebildet ist. Hierdurch wird in Bezug auf das erfindungsgemäße Flächenfiltermaterial auch eine hohe Stabilität bzw. mechanische Belastbarkeit erreicht.

Erfindungsgemäß kann es sich insbesondere derart verhalten, dass die gebrochene Polymerschaumstruktur erhältlich ist durch Trocknung und/oder Aushärtung, insbesondere Vernetzung, einer aufgeschäumten, vorzugsweise unter mechanischem Energieeintrag aufgeschäumten, wässrig oder organisch basierten, vorzugsweise wässrig basierten, Lösung und/oder Dispersion eines Schaumstrukturmaterials (Schaumstrukturpolymer), insbesondere einhergehend mit einer zumindest teilweisen Brechung des bzw. der durch die aufgeschäumte Lösung und/oder Dispersion des Schaumstrukturmaterials bereitgestellten Schaums bzw. Schaumstruktur.

Insbesondere kann in diesem Zusammenhang die Trocknung bzw. Aushärtung, insbesondere Vernetzung, in Gegenwart mindestens eines Schaumbildners und gegebenenfalls mindestens eines Schaumstabilisators und gegebenenfalls mindestens eines Vernetzers und gegebenenfalls mindestens eines Emulgators und gegebenenfalls mindestens eines Verdickers durchgeführt werden.

Was das zugrundeliegende Schaumstrukturmaterial anbelangt, welches zur Ausbildung der gebrochenen Polymerschaumstruktur eingesetzt wird, so kann dieses ausgewählt sein aus der Gruppe von Polyacrylat (PAC), Polymethacrylat (PMA), Polymethylmethacrylat (PMMA), Polycarbonat (PC), Polyurethan (PUR) und Silikonen sowie Mischungen oder Kombinationen von mindestens zwei der vorgenannten Materialien und/oder Verbindungen, vorzugsweise Polyurethan (PUR). Erfindungsgemäß ist es dabei bevorzugt, dass das Schaumstrukturmaterial ein Polyurethan (PUR) umfasst bzw. hieraus besteht.

Im Allgemeinen kann die Lösung bzw. Dispersion des Schaumstrukturmaterials eine wässrig oder organisch basierte, vorzugsweise wässrig basierte, Ausgangslösung und/oder -dispersion des Schaumstrukturmaterials enthalten. Zudem kann die Ausgangslösung das für die Trägerschicht 2 eingesetzte Schaumstrukturmaterial in Mengen im Bereich von 45 Gewichtsteilen bis 160 Gewichtsteilen, insbesondere im Bereich von 50 Gewichtsteilen bis 140 Gewichtsteilen, vorzugsweise im Bereich von 70 Gewichtsteilen bis 120 Gewichtsteilen, bezogen auf die Lösung bzw. Dispersion des Schaumstrukturmaterials, enthalten. Zudem kann die Ausgangslösung und/oder -dispersion des Schaumstrukturmaterials einen Feststoffgehalt, insbesondere in Form des Schaumstrukturmaterials, im Bereich von 15 Gew.-% bis 90 Gew.-%, insbesondere im Bereich von 25 Gew.-% bis 80 Gew.-%, vorzugsweise im Bereich von 40 Gew.-% bis 65 Gew.-%, bezogen auf die Lösung und/oder Dispersion des Schaumstrukturmaterial, aufweisen.

Gleichermaßen kann die Lösung und/oder Dispersion des Schaumstrukturmaterials den Schaumbildner in Mengen im Bereich von 0,3 Gewichtsteilen bis 10 Gewichtsteilen, insbesondere im Bereich von 0,6 Gewichtsteilen bis 5 Gewichtsteilen, vorzugsweise im Bereich von 0,8 Gewichtsteilen bis 1,8 Gewichtsteilen, bezogen auf die Lösung und/oder Dispersion des Schaumstrukturmaterials, enthalten.

Zudem kann der Schaumstabilisator ein insbesondere anionisches Fettsäuresalz sein. Insbesondere kann die Lösung bzw. Dispersion des Schaumstrukturmaterials den Schaumstabilisator in Mengen im Bereich von 2,5 Gewichtsteilen bis 25 Gewichtsteilen, insbesondere im Bereich von 4 Gewichtsteilen bis 20 Gewichtsteilen, vorzugsweise im Bereich von 5 Gewichtsteilen bis 10 Gewichtsteilen, bezogen auf die Lösung und/oder Dispersion des Schaumstrukturmaterials, enthalten.

Erfindungsgemäß kann es zudem vorgesehen sein, dass der Vernetzer ein insbesondere blockiertes Isocyanat, vorzugsweise ein insbesondere blockiertes aliphatisches Polyisocyanat, ist.

Im Allgemeinen kann dabei die Lösung bzw. Dispersion des Schaumstrukturmaterials den Vernetzer in Mengen im Bereich von 0,5 Gewichtsteilen bis 15 Gewichtsteilen, insbesondere im Bereich von 1 Gewichtsteil bis 10 Gewichtsteilen, vorzugsweise im Bereich von 3 Gewichtsteilen bis 8 Gewichtsteilen, bezogen auf die Lösung und/oder Dispersion des Schaumstrukturmaterial, enthalten.

Zudem kann der Emulgator ein insbesondere nichtionischer Polyglykolether, vorzugsweise ein insbesondere nichtionischer Arylpolyglykolether, sein. Insbesondere kann die Lösung bzw. Dispersion des Schaumstrukturmaterials den Emulgator in Mengen im Bereich von 0,3 Gewichtsteilen bis 9 Gewichtsteilen, insbesondere im Bereich von 0,6 Gewichtsteilen bis 4 Gewichtsteilen, vorzugsweise im Bereich von 1,2 Gewichtsteil bis 2,5 Gewichtsteilen, bezogen auf die Lösung und/oder Dispersion des Schaumstrukturmaterials, enthalten.

Zudem kann die Lösung bzw. Dispersion des Schaumstrukturmaterials den Verdicker in Mengen im Bereich von 0,06 Gewichtsteilen bis 4,5 Gewichtsteilen, insbesondere im Bereich von 0,12 Gewichtsteilen bis 3 Gewichtsteilen, vorzugsweise im Bereich von 0,25 Gewichtsteilen bis 1 Gewichtsteil, bezogen auf die Lösung bzw. Dispersion des Schaumstrukturmaterials, enthalten.

Auf diese Weise können insgesamt besonders leistungsfähige Schäume mit hoher Stabilität und hoher Luftdurchlässigkeit für das Trägermaterial 2 erhalten werden.

Was darüber hinaus die erfindungsgemäß vorgesehene Nanofaserschicht 3 anbelangt, so weist diese im Allgemeinen zwei gegenüberliegende Flachseiten 3a, 3b auf, wie gleichermaßen in Fig. 1 bis Fig. 4 angeführt.

Wie in Fig. 4 dargestellt, verhält es sich erfindungsgemäß insbesondere derart, dass die Nanofaserschicht 3 über eine (erste) Flachseite 3a an der (ersten) Flachseite 2a des Trägermaterials 2 fixiert bzw. zum Haften gebracht ist.

Der Fig. 4 ist zu entnehmen, dass die Nanofaserschicht 3 zumindest im Wesentlichen vollflächig und/oder ganzseitig, insbesondere über eine (erste) Flachseite 3a der Nanofaserschicht 3 dem Trägermaterial 2 zugeordnet sein kann.

In diesem Zusammenhang kann es erfindungsgemäß insbesondere vorgesehen sein, dass die Nanofaserschicht 3 zumindest im Wesentlichen vollflächig und/oder ganzseitig über eine (erste) Flachseite 3a der Nanofaserschicht 3 an der (ersten) Flachseite 2a des Trägermaterials 2 fixiert bzw. hieran zum Haften gebracht ist. Diesbezüglich wird erfindungsgemäß insbesondere erreicht, dass die in Rede stehenden Schichten in optimaler Weise hinsichtlich der erfindungsgemäß bereitgestellten Luftdurchlässigkeit bzw. der dem erfindungsgemäßen Flächenfiltermaterial zugrundeliegenden Aerosol- bzw. Partikelfilterfunktion interagieren bzw. Zusammenwirken können. Zudem wird durch die zumindest im Wesentlichen und vollflächige Verbindung der in Rede stehenden Schichten ein mechanisch stabiler Verbund bereitgestellt.

Gemäß einer erfindungsgemäß bevorzugten Ausführungsform kann es zudem vorgesehen sein, dass die Nanofaserschicht 3 unmittelbar und/oder direkt und/oder ohne dazwischenliegende Schicht(en), insbesondere ohne eine dazwischenliegende Adsorptionsschicht, dem Trägermaterial 2 zugeordnet ist, insbesondere über eine (erste) Flachseite 3a der Nanofaserschicht 3.

Folglich kann die Nanofaserschicht 3 unmittelbar bzw. direkt bzw. ohne dazwischenliegende Schichten, insbesondere ohne eine dazwischenliegende Adsorptionsschicht, an der (ersten) Flachseite 2a des Trägermaterials 2 fixiert und/oder zum Haften gebracht sein, insbesondere über eine (erste) Flachseite 3a der Nanofaserschicht 3. Auch in diesem Zusammenhang kann somit insbesondere eine vollflächige bzw. ganzseitige Verbindung der in Rede stehenden Schichten vorliegen. Hierzu kann auch auf die Figurendarstellung gemäß Fig. 4 verwiesen werden.

Insbesondere verhält es sich erfindungsgemäß entsprechend einer bevorzugten Ausführungsform derart, dass die Nanofaserschicht 3 ohne Verwendung bzw. Zugabe eines Haft- und/oder Klebemittels an der (ersten) Flachseite 2a des Trägermaterials 2 fixiert und/oder zum Haften gebracht ist.

Insbesondere kann es in diesem Zusammenhang erfindungsgemäß vorgesehen sein, dass die Nanofaserschicht 3 mittels bzw. infolge einer insbesondere bei der Herstellung des Flächenfiltermaterials 1 und/oder der Nanofaserschicht (3) vorliegenden Eigenklebrigkeit der Nanofasern 3' und/oder des Trägermaterials 2, vorzugsweise der Nanofasern 3', an der (ersten) Flachseite 2a des Trägermaterials 2 fixiert und/oder zum Haften gebracht ist.

Infolge des Verzichts auf weitere Haft- bzw. Klebemittel kann das Gesamtgewicht des erfindungsgemäßen Flächenfiltermaterials weiterführend verringert werden.

Im Allgemeinen kann die Nanofaserschicht 3 als ein die Nanofasern 3' aufweisendes bzw. hieraus bestehendes textiles Flächengebilde, insbesondere dreidimensionales textiles Flächengebilde, ausgebildet sein. Hierdurch wird eine hohe Effizienz im Hinblick auf die Bereitstellung der in Rede stehenden Aerosol- bzw. Partikelfilterfunktion erreicht.

Insbesondere können die Nanofasern 3' in der Nanofaserschicht 3 zufällig bzw. wirr angeordnet sein. Auf diese Weise wird somit eine dichte, jedoch luftdurchlässige Anordnung der Nanofasern 3' in der Nanofaserschicht 3 gewährleistet, was zu guten Aerosol- bzw. Partikelfiltereigenschaften führt.

Zudem können die Nanofasern 3' in der Nanofaserschicht 3 miteinander verbunden sein, insbesondere dauerhaft miteinander verbunden bzw. verklebt sein, insbesondere mittels bzw. infolge einer insbesondere bei der Herstellung des Flächenfiltermaterials 1 bzw. der Nanofaserschicht 3 vorliegenden Eigenklebrigkeit der Nanofasern 3'. Folglich kann, wie zuvor angeführt, auch von daher auf die Verwendung zusätzlicher Haft- bzw. Klebemittel verzichtet werden.

Erfindungsgemäß verhält es sich insbesondere derart, dass die Nanofasern 3' in der Nanofaserschicht 3 ein zusammenhängendes und/oder kohärentes Flächengebilde, insbesondere Flächenverbundmaterial, ausbilden. Insbesondere verhält es sich somit derart, dass die Nanofaserschicht als zusammenhängendes bzw. kohärentes Flächengebilde, insbesondere Flächenverbundmaterial, ausgebildet ist.

Erfindungsgemäß ist es dabei bevorzugt, dass die Nanofaserschicht 3 als ein Gelege oder Textilverbundstoff, insbesondere Vlies (Non-Woven), vorzugsweise als ein Vlies (Non-Woven), bevorzugt als ein Wirrvlies, ausgebildet ist.

Insbesondere kann die Nanofaserschicht 3, insbesondere die die Nanofaserschicht 3 ausbildenden Nanofasern 3', durch Elektrospinnen (Elektrospinnverfahren bzw. Elektrospinning), Spunbonding-Verfahren, Meltblow-Verfahren oder eine Kombination der vorgenannten Verfahren, vorzugsweise durch Elektrospinnen, hergestellt sein. In unerwarteter Weise lassen sich auf Basis der vorgenannten Verfahren, insbesondere durch Elektrospinnen, Materialien mit den besten Ergebnissen in Bezug auf den Aerosol- bzw. Partikelschutz bei gleichzeitig guter Luftdurchlässigkeit und geringem Flächengewicht herstellen bzw. bereitstellen.

Die der Herstellung der Nanofaserschicht 3 bzw. der diesbezüglichen Nanofasern 3' zugrundeliegenden Verfahren sind dem Fachmann an sich wohlbekannt. Im Allgemeinen wird unter dem Verfahren des Elektrospinnens die Herstellung von entsprechenden Nanofasern insbesondere aus Polymerlösungen und/oder -dispersionen durch eine Behandlung in einem elektrischen Feld verstanden. Hierzu kann insbesondere derart vorgegangen werden, dass die der herzustellenden Nanofasern zugrundeliegende Polymerlösung an einer Elektrode dosiert und durch ein elektrisches Feld von der Elektrode abgezogen und beschleunigt wird, wobei die Polymerlösung bzw. -dispersion in kleine und kleinste Fasern bzw. Gespinste aufgespalten wird, die nachfolgend auf dem Trägermaterial 2, welches diesbezüglich als Gegenelektrode fungieren kann, abgelagert bzw. aufgebracht werden können.

Im Allgemeinen weist die erhaltene Nanofaserschicht 3 ein dreidimensionales Netzwerk aus sozusagen aufeinander gestapelten bzw. aufeinander gelagerten und insbesondere miteinander über Kreuzungspunkte verbundenen Nanofasern 3' auf. In diesem Zusammenhang liegt für die Nanofaserschicht 3 sozusagen ein Netz aus untereinander in Verbindung stehenden Nanofasern vor, wobei eine durch die Anordnung und Struktur der Nanofasern 3' hervorgerufene hohe Porosität und große Oberfläche resultiert. Dabei zeichnet sich die erfindungsgemäß eingesetzte Nanofaserschicht 3 im Allgemeinen durch eine geringe Dicke und ein geringes Flächengewicht aus. Im Allgemeinen weist die Nanofaserschicht 3 dabei eine hohe Luft- bzw. Wasserdampfdurchlässigkeit und eine hohe Wasserdichtigkeit bzw. ein hohes Wasserabweiseverhalten auf, wobei zudem auch wind- und nässeabweisende Eigenschaften vorliegen, so dass die erfindungsgemäß eingesetzte Nanofaserschicht 3 eine hohe Funktionalität aufweist, einhergehend mit einem entsprechend großen Einsatz- bzw. Anwendungsspektrum des die Nanofaserschicht 3 aufweisenden erfindungsgemäßen Flächenfiltermaterials 1.

Der Begriff "Vlies" - synonym auch als Non-Woven bezeichnet - wird im Rahmen der vorliegenden Erfindung insbesondere als Bezeichnung für zu den Textilverbundstoffen zählende, flexible, poröse Flächengebilde verwendet, die nicht durch die klassischen Methoden der Gewebebindung von Kette und Schuss oder durch Maschenbildung, sondern durch Verschlingung bzw. kohäsive bzw. adhäsive Verbindungen von entsprechenden Fasern, vorliegend den in Rede stehenden Nanofasern 3', hergestellt sind. Vliese sind im Allgemeinen lockere Materialien aus Spinnfasern oder Filamenten, insbesondere aus synthetischen Fasern bzw. Chemiefasern hergestellt, deren Zusammenhalt im Allgemeinen durch die den Fasern, insbesondere bei der Herstellung vorliegende eigene Haftung bzw. Klebrigkeit gegeben ist. Hierbei können die einzelnen Fasern eine Vorzugsrichtung aufweisen (sogenannte orientierte oder Kreuzlage-Vliese) oder aber auch ungerichtet sein (sogenannte Wirrvliese, wie erfindungsgemäß bevorzugt). Die Vliese können mechanisch verfestigt werden, insbesondere durch Vernadeln, Vermaschen oder durch Verwirbeln mittels scharfer Wasserstrahlen (sogenanntes Spunlaced-Vliese). Erfindungsgemäß besonders geeignete Vliese bzw. Wirrvliese können beispielsweise durch Spunbonding-, Meltblow-Verfahren und bevorzugt durch Elektrospinning bzw. Elektrospinnen hergestellt werden.

Für weitergehende Einzelheiten zum Begriff der Vliese und der Vliesstoffe kann beispielsweise verwiesen werden auf Römpp Chemie Lexikon, 10. Auflage, Georg Thieme Verlag Stuttgart / New York, Band 6, 1999, Seiten 4889/4890, Stichwort: "Vliesstoffe", wobei der diesbezügliche Offenbarungsgehalt, einschließlich der dort referierten Literaturstellen, hiermit durch Bezugnahme vollumfänglich eingeschlossen ist.

Insbesondere kann die erfindungsgemäß eingesetzte Nanofaserschicht 3 bzw. können die die Nanofaserschicht 3 ausbildenden Nanofasern 3' entsprechend den Ausführungen in der US 6 641 773 B2, der WO 2016/171327 A1, WO 2016/171326 A1, WO 2015/053443 A1 sowie der WO 2008/136581 A1, hergestellt werden.

Erfindungsgemäß kann die Nanofaserschicht 3 ein Flächengewicht im Bereich von 0,2 g/m² bis 60 g/m², insbesondere im Bereich von 0,5 g/m² bis 40 g/m², vorzugsweise im Bereich von 0,8 g/m² bis 20 g/m², bevorzugt im Bereich von 0,9 g/m² bis 10 g/m², besonders bevorzugt im Bereich von 0,95 g/m² bis 5 g/m², ganz besonders bevorzugt im Bereich von 1 g/m² bis 3 g/m², aufweisen. Die Anmelderin hat überraschenderweise herausgefunden, dass sich auf diese Weise insbesondere der Aerosol- bzw. Partikelschutz wirksam verbessern bzw. optimieren lässt.

Zudem kann die Nanofaserschicht eine Dicke von höchstens 100 µm, insbesondere höchstens 50 µm, vorzugsweise höchstens 20 µm, bevorzugt höchstens 10 µm, besonders bevorzugt höchstens 5 µm, aufweisen. In diesem Zusammenhang kann die Nanofaserschicht 3 eine Dicke im Bereich von 0,001 µm bis 100 µm, insbesondere im Bereich von 0,005 µm bis 50 µm, vorzugsweise im Bereich von 0,008 µm bis 20 µm, bevorzugt im Bereich von 0,01 µm bis 10 µm, besonders bevorzugt im Bereich von 0,15 µm bis 8 µm, ganz besonders bevorzugt im Bereich von 1 µm bis 7 µm, weiter bevorzugt im Bereich von 2 µm bis 6 µm, noch weiter bevorzugt im Bereich von 3 µm bis 5 µm, wiederum weiter bevorzugt bevorzugt im Bereich von 4 µm bis 5 µm, aufweisen. In diesem Zusammenhang wird unter dem Begriff der Dicke der Nanofaserschicht 3 insbesondere der Abstand der (ersten) Seite 3a zu der (zweiten) Seite 3b der Nanofaserschicht 3 verstanden. Im Allgemeinen kann die Nanofaserschicht 3 zudem eine Dichte im Bereich von 10 kg/m³ bis 1.000 kg/m³, insbesondere im Bereich von 100 kg/m³ bis 800 kg/m³, vorzugsweise im Bereich von 150 kg/m³ bis 500 kg/m³, bevorzugt im Bereich von 200 kg/m³ bis 300 kg/m³, aufweisen.

Zudem kann die Nanofaserschicht 3 eine Luftdurchlässigkeit von mindestens 30 l·m⁻²·s⁻¹ , insbesondere mindestens 40 l·m⁻²·s⁻¹, vorzugsweise mindestens 50 l·m⁻²·s⁻¹, besonders bevorzugt mindestens 100 l·m⁻²·s⁻¹, ganz besonders bevorzugt mindestens 150 l·m⁻²·s⁻¹, bei einem Strömungswiderstand von 127 Pa, aufweisen.

Aufgrund der definierten Luftdurchlässigkeit auch der Nanofaserschicht 3 wird insbesondere auch der Tragekomfort des erfindungsgemäßen Flächenfiltermaterials erhöht, was zu einer hohen Eignung des Materials für Schutzausrüstungen, wie ABC-Schutzanzügen, sowie Bekleidungsstücke für den Sport- bzw. Freizeitbereich führt, insbesondere kann der Tragekomfort im Vergleich zu Membrananzügen oder dergleichen deutlich erhöht werden.

Zudem werden insbesondere durch die Nanofaserschicht 3, insbesondere auch im Zusammenwirken mit der Trägerschicht 2, windabweisende und nässeabweisende Eigenschaften bereitgestellt, was die Eignung des Materials für die vorgenannten Einsatzzwecke nochmals verbessert.

Was die der Nanofaserschicht zugrundeliegenden Nanofasern 3' anbelangt, so weisen diese im Allgemeinen einen Faserdurchmesser im Bereich von 1 nm bis 5.000 nm, insbesondere im Bereich von 5 nm bis 2.500 nm, vorzugsweise im Bereich von 10 nm bis 2.000 nm, bevorzugt im Bereich von 15 nm bis 1.500 nm, besonders bevorzugt im Bereich von 20 nm bis 1.000 nm, ganz besonders bevorzugt im Bereich von 25 nm bis 750 nm, weiter bevorzugt im Bereich von 50 nm bis 500 nm, auf. Insbesondere können die Nanofasern 3' einen mittleren Faserdurchmesser D₅₀ im Bereich von 2 nm bis 4.500 nm, insbesondere im Bereich von 10 nm bis 2.000 nm, vorzugsweise im Bereich von 15 nm bis 1.750 nm, bevorzugt im Bereich von 20 nm bis 1.250 nm, besonders bevorzugt im Bereich von 25 nm bis 750 nm, ganz besonders bevorzugt im Bereich von 30 nm bis 500 nm, weiter bevorzugt im Bereich von 60 nm bis 400 nm, aufweisen. Bei Vorliegen der vorgenannten Faserdurchmesser liegt eine weiterführende Optimierung der Nanofaserschicht 3 insbesondere im Hinblick auf die diesbezüglich bereitgestellten Aerosol- bzw. Partikelfiltereigenschaften vor.

Die vorgenannten Faserdurchmesser können dabei mit dem Fachmann an sich bekannten Methoden bestimmt werden. Insbesondere können die in Rede stehenden Faserdurchmesser mikroskopisch bestimmt werden, beispielsweise unter Verwendung von insbesondere digitaler Lichtmikroskopie, Rasterelektronenmikroskopie (REM) bzw. Transmissionelektronenmikroskopie (TEM), insbesondere jeweils gekoppelt mit digitaler bzw. elektronischer Bildauswertung. Insbesondere können die vorgenannten Faserdurchmesser entsprechend den Ausführungen gemäß der WO 2015/009962 A1, deren diesbezüglicher Offenbarungsgehalt hiermit durch Bezugnahme vollumfänglich eingeschlossen ist, bestimmt werden. Zudem können die vorgenannten Faserdurchmesser insbesondere entsprechend den Ausführungen in der Publikation gemäß Ziabari et al., "A New Image Analysis Based Method for Measuring Electrospun Nanofiber Diameter", Nanoscale Research Letters, December 2007, 2:597, bestimmt werden.

Im Allgemeinen weist die Nanofaserschicht 3 durch die Nanofasern 3' begrenzte Öffnungen oder Poren auf. In diesem Zusammenhang kann die Nanofaserschicht 3 eine mittlere Öffnungsgröße oder mittlere Porengröße von höchstens 150 µm , insbesondere höchstens 100 µm, vorzugsweise höchstens 50 µm, besonders bevorzugt höchstens 30 µm, ganz besonders bevorzugt höchstens 20 µm, noch mehr bevorzugt höchstens 10 µm, aufweisen. Zudem kann das Verhältnis der mittleren Öffnungsgröße oder der mittleren Porengröße einerseits zum mittleren Faserdurchmesser D₅₀ der Nanofasern 3' im Bereich von 0,2 bis 1.800, insbesondere 1 bis 400, vorzugsweise 5 bis 300, besonders bevorzugt 10 bis 250, ganz besonders bevorzugt 25 bis 225, liegen. Auch hierdurch werden die Aerosol- bzw. Partikelfiltereigenschaften weiterführend verbessert bzw. können die diesbezüglichen Eigenschaften gezielt vorgegeben bzw. maßgeschneidert werden, wobei insbesondere gleichzeitig eine hohe Luftdurchlässigkeit bzw. Wasserdampfdurchlässigkeit gewährleistet ist.

Im Allgemeinen können als Nanofasern 3' synthetische Fasern (Chemiefasern) eingesetzt sein. Insbesondere können die Nanofasern 3' ein Material und/oder eine Verbindung aus der Gruppe von Polyestern (PES); Polyolefinen, wie Polyethylen (PE), Polypropylen (PP), Polyoxyethylen und Polyoxypropylen; Polyvinylchloriden (CLF); Polyvinylidenchloriden (CLF); Acetaten (CA); Triacetaten (CTA); Polyacryl (PAN), insbesondere Polyacrylnitrilen; Polyamiden (PA); Polyamidimiden (PAI); Polyethersulfonen (PESU); Polystyrolen (PS); Polyvinylalkoholen (PVAL); Polyurethanen; Polyvinylestern; Poly(meth-)acrylaten; Polyvinylidenfluoriden (PVDF); Viskose (CV); Lyocell; Silikonen; sowie Mischungen oder Kombinationen von mindestens zwei der vorgenannten Materialien und/oder Verbindungen, besonders bevorzugt Polyestern, Polyolefinen, Polyamiden, Polyacrylnitrilen, Poly(meth-)acrylaten, Polyamidimiden; Polyethersulfonen, Polystyrolen und Polyvinylidenfluoriden sowie Mischungen oder Kombinationen von mindestens zwei der vorgenannten Materialien und/oder Verbindungen, umfassen oder hieraus bestehen.

Gleichermaßen kann es erfindungsgemäß vorgesehen sein, dass als Nanofasern 3' Fasern auf Basis und/oder in Form mindestens einer biopolymeren Verbindung eingesetzt sind. Insbesondere können die Nanofasern 3' ein Material bzw. eine Verbindung aus der Gruppe von Gelatine, Chitosan, Kollagen, Polyaramiden (PAA), Polylactiden (PLA), sowie Mischungen oder Kombinationen von mindestens zwei der vorgenannten Materialien und/oder Verbindungen umfassen oder hieraus bestehen.

Die vorgenannten Materialien gehen insbesondere mit einer hohen Stabilität und definierten Ausbildung der Nanofasern 3' bzw. der Nanofaserschicht 3 einher.

Um eine effiziente Abscheiderate in Bezug auf die unschädlich zu machenden Aerosole bzw. Partikel zu erreichen, sollte die Nanofaserschicht 3 einen mittleren Wirkungsgrad Eₘ nach DIN EN 779 (Juli 1993) von mindestens 45 %, insbesondere mindestens 55 %, vorzugsweise mindestens 75 %, besonders bevorzugt mindestens 90 %, ganz besonders bevorzugt mindestens 95 %, aufweisen. Weiterhin sollte die Nanofaserschicht einen mittleren Abscheidegrad Aₘ nach DIN EN 779 (Juli 1993) von mindestens 55 %, insbesondere mindestens 75 %, vorzugsweise mindestens 90 %, besonders bevorzugt mindestens 95 %, ganz besonders bevorzugt mindestens 99 %, aufweisen.

Die DIN EN 779 vom Juli 1993 betrifft im Allgemeinen die Anforderungen, Prüfung und Kennzeichnung von Partikel-Luftfiltern für allgemeine Raumlufttechnik. Nach dieser Vorschrift wird der mittlere Abscheidegrad Aₘ durch eine gravimetrische Prüfmethode bestimmt, wobei eine mehrmalige Bestaubung des Prüflings mit einer bekannten Menge eines standardisierten künstlichen Prüfstaubs in strömender Luft bis zum maximalen Enddruckverlust von 250 Pa erfolgt, wobei jeweils der Abscheidegrad aus den Massenverhältnissen durch Wägen eines dem Prüfling nachgeschalteten Schwebstofffilters bestimmt wird, wobei der mittlere Abscheidegrad Aₘ, berechnet aus allen Einzelmessungen, gilt; für weitergehende diesbezügliche Einzelheiten kann auf die DIN EN 779 verwiesen werden. Der mittlere Wirkungsgrad Eₘ dagegen wird nach DIN 779 vom Juli 1993 mittels einer Verfärbungsprüfmethode durch mehrfache Messung des Wirkungsgrades gegenüber natürlichem atmosphärischem Staub in der Luft gemessen, wobei der Prüfling nach einer ersten Messung im Neuzustand mit einer bekannten Menge von standardisierten künstlichem Prüfstaub nach DIN EN 779 beladen und danach die Bestimmung des Wirkungsgrads erneut vorgenommen wird, bis ein Enddruckverlust von 450 Pa erreicht ist, wobei die Messung des Wirkungsgrades auf dem Vergleich jener Prüfluftvolumina beruht, die vor und nach dem Prüfling durch je ein weißes Schwebstoffilterpapier gesaugt werden müssen, bis diese gleich verfärbt bzw. getrübt sind, wobei der mittlere Wirkungsgrad Eₘ, berechnet aus allen Einzelmessungen, gilt; für weitere diesbezügliche Einzelheiten kann auf DIN EN 779 verwiesen werden.

Zudem sollte zu Zwecken der Erzielung einer guten Partikel- und Aerosolabscheidung die Nanofaserschicht 3 einen integralen Anfangsdurchlassgrad Dᵢ nach DIN EN 1822 (April 1998; DEHS-Aerosol (Diethylhexylsebacat), Most Penetrating Particle Size = MPPS = 0,1 bis 0,3 µm) von höchstens 45%, insbesondere höchstens 40 %, vorzugsweise höchstens 30 %, besonders bevorzugt höchstens 20 %, ganz besonders bevorzugt höchstens 10 %, aufweisen.

Die Prüfmethode gemäß DIN EN 1822 wird an unverschmutzten Prüflingen mit einem flüssigen Prüfaerosol (DEHS = Diethylhexylsebacat), basierend auf Messwerten für jeweils einen dem Durchlassgradmaximum entsprechenden Partikeldurchmesser (sogenannter MPPS, hier: 0,1 bis 0,3 µm), durchgeführt. In einem ersten Schritt der Untersuchung wird an flachen Mustern des Filtermedium jene Partikelgröße ermittelt, bei welcher das Durchlassmaximum (MPPS) erreicht wird, wobei die nachfolgende Beurteilung und Klassierung der Filter nur noch für den MPPS erfolgt. In einem zweiten Schritt wird dann der über die Ausblasfläche ermittelte integrale Durchlassgrad Dᵢ für den MPPS und der Druckverlust des Filters, beides beim Nennvolumenstrom, gemessen. Für weitergehende diesbezügliche Einzelheiten kann auf die DIN EN 1822 verwiesen werden.

Weiterhin sollte die Nanofaserschicht 3 eine Abscheidegradleistung nach DIN EN 1822 (April 1998) von mindestens 45 %, insbesondere mindestens 55 %, vorzugsweise mindestens 75 %, besonders bevorzugt mindestens 90 %, ganz besonders bevorzugt mindestens 95 %, aufweisen.

Zudem sollte die Nanofaserschicht 3 bei einer Anströmgeschwindigkeit von 0,1 m/s eine mittlere Abscheiderate gegenüber Partikeln und/oder Aerosolen mit Durchmessern im Bereich von 0,1 bis 0,3 µm von mindestens 85 %, insbesondere mindestens 90 %, vorzugsweise mindestens 95 %, aufweisen.

Insbesondere sollte die Nanofaserschicht 3 bei einer Anströmgeschwindigkeit von 0,1 m/s eine mittlere Abscheiderate gegenüber Partikeln und/oder Aerosolen mit Durchmessern ≥ 2 µm, insbesondere ≥ 1,5 µm, vorzugsweise ≥ 1,0 µm, von mindestens 97 %, insbesondere mindestens 98 %, bevorzugt mindestens 99 %, aufweisen.

Gemäß einer erfindungsgemäßen Ausführungsform kann es zudem vorgesehen sein, das die Nanofaserschicht 3, insbesondere die Nanofasern 3' der Nanofaserschicht 3, teilweise in das Trägermaterial 2 eingedrungen vorliegt bzw. vorliegen. Insbesondere kann sich die Nanofaserschicht 3 insbesondere teil- und/oder abschnittsweise in das Trägermaterial 2 hineinerstrecken. Dies kann beispielsweise dadurch erreicht werden, dass die im Rahmen der Herstellung des erfindungsgemäßen Flächenfiltermaterials 1 die Nanofaserschicht 3 auf einem noch nicht vollständig gehärteten bzw. getrockneten Trägermaterial aufgebracht bzw. hierauf angedrückt wird. Hierdurch kann der Verbund zwischen und Trägermaterial 2 einerseits und Nanofaserschicht 3 andererseits weiterführend verfestigt bzw. stabilisiert werden.

In diesem Zusammenhang kann die Nanofaserschicht 3, insbesondere die Nanofasern 3' der Nanofaserschicht 3, sich über mindestens 1 %, insbesondere mindestens 5 %, vorzugsweise mindestens 10 %, der Dicke des Trägermaterials 2 in das Trägermaterial 2 hineinerstrecken. Insbesondere kann die Nanofaserschicht 3, insbesondere die Nanofasern 3' der Nanofaserschicht 3, sich über höchstens 30 %, insbesondere höchstens 25 %, vorzugsweise höchstens 20 % der Dicke des Trägermaterials 2 in das Trägermaterial 2 hineinerstrecken.

Erfindungsgemäß kann es darüber hinaus vorgesehen sein, dass das Flächenfiltermaterial 1 mindestens eine weitere Nanofaserschicht aufweist. In diesem Zusammenhang kann die weitere Nanofaserschicht dem schaumbasierten bzw. schaumförmigen Trägermaterial 2 zugeordnet sein. Zudem kann die weitere Nanofaserschicht auf der der Nanofaserschicht 3 abgewandten Seite des Trägermaterials 2 angeordnet sein. Insbesondere kann in diesem Zusammenhang die weitere Nanofaserschicht an einer (zweiten) Seite 2b, insbesondere Flachseite, des Trägermaterials 2 fixiert bzw. zum Haften gebracht sein. Insbesondere kann die weitere Nanofaserschicht eine Vielzahl von Nanofasern umfassen oder hieraus bestehen.

Grundsätzlich kann die weitere Nanofaserschicht vergleichbar zu bzw. identisch zu der Nanofaserschicht 3 aufgebaut bzw. ausgebildet sein, so dass hierzu auf obige Ausführungen zu der Nanofaserschicht 3 verwiesen werden kann, welche für die weitere Nanofaserschicht bzw. die hierzu eingesetzten Nanofasern gleichermaßen gelten.

Gemäß einer erfindungsgemäß bevorzugten Ausführungsform kann es zudem vorgesehen sein, dass, das erfindungsgemäße Flächenfiltermaterial 1 weiterhin (c) mindestens einen textilen Träger 4 aufweist.

In diesem Zusammenhang kann der textile Träger 4 zwei gegenüberliegende Flachseiten 4a, 4b aufweisen.

Darüber hinaus kann der textile Träger 4 dem Trägermaterial 2 zugeordnet sein. In diesem Zusammenhang kann der textile Träger 4 auf der der Nanofaserschicht 3 abgewandten Seite 2b des Trägermaterials 2 angeordnet sein.

Erfindungsgemäß kann es insbesondere vorgesehen sein, dass der textile Träger 4 an dem Trägermaterial 2 fixiert bzw. zum Haften gebracht ist.

Es kann in diesem Zusammenhang erfindungsgemäß vorgesehen sein, dass der textile Träger 4 an einer (zweiten) Flachseite 2b des Trägermaterials 2 fixiert bzw. zum Haften gebracht ist, insbesondere über eine (erste) Flachseite 4a des textilen Trägers 4.

Durch die Verwendung eines textilen Trägers 4 kann das erfindungsgemäße Flächenfiltermaterial 1 weiterführend stabilisiert werden und zudem in gezielter Weise mit speziellen textilen Eigenschaften ausgerüstet werden. Zudem kann der textile Träger 4 im Rahmen der Herstellung des erfindungsgemäßen Flächenfiltermaterials 1 zur Aufnahme einer bzw. eines insbesondere nicht ausgehärteten bzw. insbesondere nicht getrockneten Schaumstruktur bzw. Schaumstrukturmaterials zur Ausbildung des Trägermaterials 2 eingesetzt werden. Im Allgemeinen kann der textile Träger 4 erfindungsgemäß beispielsweise mittels bzw. infolge einer insbesondere bei der Herstellung des Flächenfiltermaterials 1 vorliegenden Eigenklebrigkeit des Trägermaterials 2 an dem Trägermaterial 2, insbesondere an der (zweiten) Flachseite 2b des Trägermaterials 2, fixiert bzw. zum Haften gebracht sein.

Gemäß einer alternativen Ausführungsform der vorliegenden Erfindung kann es aber auch vorgesehen sein, dass textile Träger 4 mittels Verwendung eines insbesondere diskontinuierlich-punktförmig aufgetragenen Haftmittels an dem Trägermaterial 2, insbesondere an der (zweiten) Flachseite 2b des Trägermaterials 2, fixiert und/oder zum Haften gebracht ist.

Insbesondere kann der textile Träger 4 als ein textiles Flächengebilde, vorzugsweise ein luftdurchlässiges und/oder wasserdampfdurchlässiges Textilmaterial, bevorzugt ein Gewebe, Gewirke, Gestricke, Gelege, Vlies oder Textilverbundstoff, ausgebildet sein.

Im Allgemeinen kann der textile Träger 4 zudem ein Flächengewicht im Bereich von 15 g/m² bis 500 g/m², insbesondere im Bereich von 25 g/m² bis 300 g/m², bevorzugt im Bereich von 35 g/m² bis 175 g/m², aufweisen.

Erfindungsgemäß kann es insbesondere vorgesehen sein, dass der textile Träger 4 ein aus natürlichen und/oder synthetischen Fasern, vorzugsweise synthetischen Fasern (Chemiefasern), bestehendes textiles Flächengebilde ist.

Insbesondere kann der textile Träger 4 ein textiles Flächengebilde mit oder aus natürlichen und/oder synthetischen Fasern, insbesondere aus der Gruppe von Polyestern (PES); Polyolefinen, wie Polyethylen (PE) und Polypropylen (PP), Polyvinylchlorid (CLF); Polyvinylidenchlorid (CLF); Acetat (CA); Triacetat (CTA); Polyacryl (PAN), Polyamid (PA); Polyaramiden; Polyvinylalkohol (PVAL); Polyurethanen; Polyvinylestern; (Meth-)Acrylaten; Viskose (CV); Lyocell; sowie deren Mischungen oder Kombinationen, sein.

Der textile Träger 4 kann eine Dicke im Bereich von 0,005 bis 10 mm, insbesondere im Bereich von 0,015 bis 5 mm, vorzugsweise im Bereich von 0,02 bis 1 mm, aufweisen. Unter dem Begriff der Dicke des textilen Trägers 4 wird dabei insbesondere der Abstand der (ersten) Seite 4a zur (zweiten) Seite 4b des textilen Trägers verstanden.

Zudem kann der textile Träger 4 eine Luftdurchlässigkeit von mindestens 150 l·m⁻²·s⁻¹, insbesondere mindestens 200 l·m⁻².s⁻¹, vorzugsweise mindestens 250 l·m⁻²·s⁻¹, besonders bevorzugt mindestens 400 l·m⁻²·s⁻¹, ganz besonders bevorzugt mindestens 800 l·m⁻²·s⁻¹, bei einem Strömungswiderstand von 127 Pa, aufweisen.

Wie in Fig. 3 dargestellt, kann es erfindungsgemäß zudem vorgesehen sein, dass das Flächenfiltermaterial 1 weiterhin (d) mindestens eine Abdeckschicht 5 aufweist.

Hierdurch kann insbesondere die Nanofaserschicht 3 mit einem zusätzlichen Schutz versehen werden. Zudem kann das Flächenfiltermaterial 1 mit weiterführenden textilen Eigenschaften, z. B. im Hinblick auf dessen Optik bzw. Haptik, ausgerüstet werden.

Im Allgemeinen weist die Abdeckschicht 5 zwei gegenüberliegende Flachseiten 5a, auf, wie gleichermaßen in Fig. 3 schematisch dargestellt.

Im Allgemeinen kann die Abdeckschicht 5 der Nanofaserschicht 3 zugeordnet sein. Insbesondere kann die Abdeckschicht 5 auf der dem Trägermaterial 2 abgewandten Flachseite 3b der Nanofaserschicht 3 zugeordnet sein.

Zudem kann die Abdeckschicht 5 auf der dem Trägermaterial 2 abgewandten Flachseite 3b der Nanofaserschicht 3 angeordnet sein. Hierzu kann gleichermaßen auf Fig. 3 verwiesen werden.

In diesem Zusammenhang kann die Abdeckschicht 5 an einer (zweiten) Flachseite 3b der Nanofaserschicht 3 fixiert bzw. zum Haften gebracht sein, und zwar insbesondere über eine (erste) Flachseite 5a der Abdeckschicht 5, wie in Fig. 3 veranschaulicht.

Die Abdeckschicht 5 kann im Rahmen der vorliegenden Erfindung an der Nanofaserschicht 3 fixiert bzw. hieran zum Haften gebracht sein, so dass auch diesbezüglich ein dauerhafter Verbund vorliegt.

Im Rahmen der vorliegenden Erfindung kann es in diesem Zusammenhang vorgesehen sein, dass die Abdeckschicht 5 mittels bzw. infolge einer insbesondere bei der Herstellung des Flächenfiltermaterials 1 und/oder der Nanofaserschicht 3 vorliegenden Eigenklebrigkeit der Nanofaserschicht 3, insbesondere der Nanofasern 3' der Nanofaserschicht 3, an der Nanofaserschicht 3, insbesondere an der (zweiten) Seite 3b der Nanofaserschicht 3, fixiert bzw. zum Haften gebracht ist.

Erfindungsgemäß ist es jedoch auch möglich, dass die Abdeckschicht 5 mittels Verwendung eines insbesondere diskontinuierlich-punktförmig aufgetragenen Haftmittels an der Nanofaserschicht 3, insbesondere an der (zweiten) Seite 3b der Nanofaserschicht 3, fixiert und/oder zum Haften gebracht ist.

Im Allgemeinen kann die Abdeckschicht 5 als ein textiles Flächengebilde, vorzugsweise ein luftdurchlässiges und/oder wasserdampfdurchlässiges Textilmaterial, bevorzugt ein Gewebe, Gewirke, Gestricke, Gelege, Vlies oder Textilverbundstoff, ausgebildet sein.

In diesem Zusammenhang kann die Abdeckschicht 5 ein Flächengewicht im Bereich von 5 g/m² bis 400 g/m², insbesondere im Bereich von 10 g/m² bis 200 g/m², bevorzugt im Bereich von 20 g/m² bis 150 g/m², aufweisen.

Erfindungsgemäß kann es vorgesehen sein, dass die Abdeckschicht 5 ein aus natürlichen und/oder synthetischen Fasern, vorzugsweise synthetischen Fasern (Chemiefasern), bestehendes textiles Flächengebilde ist.

Insbesondere kann die Abdeckschicht 5 ein textiles Flächengebilde mit oder aus natürlichen und/oder synthetischen Fasern, insbesondere aus der Gruppe von Polyestern (PES); Polyolefinen, wie Polyethylen (PE) und Polypropylen (PP), Polyvinylchlorid (CLF); Polyvinylidenchlorid (CLF); Acetat (CA); Triacetat (CTA); Polyacryl (PAN), Polyamid (PA); Polyaramiden; Polyvinylalkohol (PVAL); Polyurethanen; Polyvinylestern; (Meth-)Acrylaten; Viskose (CV); Lyocell; sowie deren Mischungen oder Kombinationen, sein.

Zudem kann die Abdeckschicht 5 eine Dicke im Bereich von 0,001 bis 8 mm, insbesondere im Bereich von 0,005 bis 4 mm, vorzugsweise im Bereich von 0,01 bis 0,9 mm, aufweisen.

Im Allgemeinen wird unter der Dicke der Abdeckschicht 5b insbesondere der Abstand der (ersten) Seite 5a zu der (zweiten) Seite 5b der Abdeckschicht 5 verstanden.

Zudem kann die Abdeckschicht 5 eine Luftdurchlässigkeit von mindestens 200 l·m⁻²·s⁻¹, insbesondere mindestens 250 l·m⁻²·s⁻¹, vorzugsweise mindestens 300 l·m⁻²·s⁻¹, besonders bevorzugt mindestens 450 l·m⁻²·s⁻¹, ganz besonders bevorzugt mindestens 850 l·m⁻²·s⁻¹, bei einem Strömungswiderstand von 127 Pa, aufweisen.

Gemäß der vorliegenden Erfindung ist es zudem vorgesehen, dass das Flächenfiltermaterial 1 weiterhin mindestens eine Adsorptionsschicht 6 aufweist, wie in Fig. 4 dargestellt. Durch die Ausrüstung des erfindungsgemäßen Flächenfiltermaterials 1 mit mindestens einer Adsorptionsschicht 6 kann die Schutzfunktion des Flächenfiltermaterials 1 gegenüber chemischen, biologischen bzw. radioaktiven Schad- und Giftstoffen weiterführend erhöht werden, da das Flächenfiltermaterial 1 nach der Erfindung auf Basis der Adsorptionsschicht 6 zusätzlich mit adsorptiven Eigenschaften ausgerüstet ist.

Erfindungsgemäß ist es dabei vorgesehen, dass die Adsorptionsschicht 6 eine Vielzahl einzelner bzw. diskreter Adsorberpartikel 6' umfasst oder hieraus gebildet ist, wie gleichermaßen in Fig. 4 schematisch dargestellt.

Fig. 4 verdeutlicht zudem, dass die Adsorptionsschicht 6 zwei gegenüberliegende Flachseiten 6a, 6b aufweist.

Erfindungsgemäß ist es vorgesehen, dass die Adsorptionsschicht 6 dem Trägermaterial 2 zugeordnet ist. In diesem Zusammenhang ist es erfindungsgemäß vorgesehen, dass die Adsorptionsschicht 6 auf der der Nanofaserschicht 3 abgewandten (zweiten) Flachseite 2b des Trägermaterials 2 angeordnet ist, wie gleichermaßen in Fig. 4 veranschaulicht.

Im Allgemeinen sollte die Adsorptionsschicht 6 im Rahmen der vorliegenden Erfindung diskontinuierlich ausgebildet sein. Insbesondere kann es erfindungsgemäß vorgesehen sein, dass die Adsorptionsschicht 6 als ein Adsorptionsflächenfilter ausgebildet ist. Hierdurch wird zum einen gewährleistet, dass das erfindungsgemäße Flächenfiltermaterial 1 insgesamt eine hohe Luft- bzw. Wasserdampfdurchlässigkeit aufweist und dass zum anderen die adsorptiven Eigenschaften und damit die Gesamtschutzfunktion des erfindungsgemäßen Flächenfiltermaterials 1 aufgrund der guten Zugänglichkeit der Adsorptionsschicht 6 bzw. der Adsorberpartikel 6' nochmals verbessert sind.

Im Rahmen der vorliegenden Erfindung verhält es sich insbesondere derart, dass sich die Nanofaserschicht 3, das schaumbasierte bzw. schaumförmige Trägermaterial 2 sowie die gegebenenfalls vorhandene Adsorptionsschicht 6 hinsichtlich der Bereitstellung der in Rede stehenden Schutzfunktion funktional über die bloße Summe der Schutzwirkungen der einzelnen Schichten hinaus ergänzen. Insbesondere wird die Adsorptionsschicht 6 hinsichtlich zu adsorbierender Schad- bzw. Giftstoffe durch die Nanofaserschicht 3 bzw. das Trägermaterial 2 entlastet, da durch diese Schichten bereits eine effektive Aerosol- bzw. Partikelfilterfunktion bereitgestellt wird und somit ein Großteil der Schad- bzw. Giftstoffe vor Erreichen der Adsorptionsschicht 6 abgefangen werden. Im Hinblick auf die Verwendung bzw. Verarbeitung des erfindungsgemäßen Flächenfiltermaterials 1 für bzw. zu Schutzausrüstung, insbesondere Schutzbekleidung, ist es dabei insbesondere vorgesehen, dass zum einen die Adsorptionsschicht 6 im Trage- bzw. Benutzungszustand auf der dem Benutzer bzw. Träger der Bekleidung zugeordneten Seite des Flächenfiltermaterials 1 bzw. des Trägermaterials 2 bzw. dass zum anderen die Nanofaserschicht 3 auf der dem Benutzer bzw. Träger der Bekleidung abgewandten Seite des Flächenfiltermaterials 1 bzw. des Trägermaterials 2 angeordnet ist.

Gemäß einer ersten erfindungsgemäßen Ausführungsform kann es vorgesehen sein, dass die Adsorptionsschicht 6 an dem Trägermaterial 2 fixiert bzw. zum Haften gebracht ist.

Erfindungsgemäß kann es dabei insbesondere vorgesehen sein, dass die Adsorptionsschicht 6 an einer (zweiten) Flachseite 2b des Trägermaterials 2 fixiert bzw. zum Haften gebracht ist. Diesbezüglich kann die Fixierung bzw. das Zumhaftenbringen, insbesondere über eine (erste) Flachseite 6a der Adsorptionsschicht 6 erfolgen, wie in Fig. 4 schematisch veranschaulicht.

Im Rahmen dieser erfindungsgemäßen Ausführungsform kann es dabei vorgesehen sein, dass die Adsorptionsschicht 6, insbesondere die Adsorberpartikel 6' der Adsorptionsschicht 6, mittels Verwendung eines insbesondere diskontinuierlich-punktförmig aufgetragenen Haftmittels an dem Trägermaterial 2, insbesondere an der (zweiten) Flachseite 2b des Trägermaterials 2 fixiert und/oder zum Haften gebracht ist. Zur diesbezüglichen Fixierung kann beispielsweise auch ein Klebstoffweb oder dergleichen eingesetzt sein.

Erfindungsgemäß kann es zudem vorgesehen sein, dass die Adsorptionsschicht 6, insbesondere die Adsorptionspartikel 6 der Adsorptionsschicht 6, an dem gegebenenfalls vorgesehenen textilen Träger 4 fixiert bzw. zum Haften gebracht ist bzw. sind.

Diesbezüglich kann die Adsorptionsschicht 6, insbesondere die Adsorptionspartikel 6 der Adsorptionsschicht 6, an einer (ersten) Flachseite 4a des textilen Trägers 4 fixiert bzw. zum Haften gebracht sein, insbesondere über eine (zweite) Flachseite 6b der Adsorptionsschicht 6. Auch diesbezüglich kann ein insbesondere diskontinuierlich-punktförmig aufgetragenes Haftmittel zum Einsatz kommen.

Insbesondere kann es erfindungsgemäß vorgesehen sein, dass die Adsorptionsschicht 6, insbesondere die Adsorberpartikel 6' der Adsorptionsschicht 6, zwischen dem Trägermaterial 2 und dem textilen Träger 4 angeordnet ist bzw. sind. In diesem Zusammenhang kann die Adsorptionsschicht an einer (zweiten) Flachseite 2b des Trägermaterials 2 fixiert bzw. zum Haften gebracht sein, insbesondere über eine (erste) Flachseite 6a der Adsorptionsschicht 6. In diesem Zusammenhang kann es gleichermaßen vorgesehen sein, dass die Adsorptionsschicht 6, insbesondere die Adsorberpartikel 6' der Adsorptionsschicht 6, an einer (ersten) Flachseite 4a des textilen Trägers 4 fixiert bzw. zum Haften gebracht ist bzw. sind, insbesondere über eine (zweite) Flachseite 6b der Adsorptionsschicht 6. Zur diesbezüglichen Fixierung kann gleichermaßen jeweils ein insbesondere diskontinuierlich-punktförmig aufgetragenes Haftmittel verwendet sein. Zur diesbezüglichen Fixierung kann beispielsweise auch ein Klebstoffweb oder dergleichen eingesetzt sein.

Auf diese Weise wird insgesamt ein stabiler Verbund im Hinblick auf das erfindungsgemäße Flächenfiltermaterial 1 bereitgestellt.

Erfindungsgemäß ist es grundsätzlich auch möglich, dass die Adsorptionsschicht 6, insbesondere die Adsorberpartikel 6' der Adsorptionsschicht 6, an einer (zweiten) Flachseite 4b des textilen Trägers 4 fixiert bzw. zum Haften gebracht ist bzw. sind, insbesondere über eine (erste) Flachseite 6a der Adsorptionsschicht 6. Für diesen Fall verhält es sich somit insbesondere derart, dass der textile Träger 4 zwischen dem Trägermaterial 2 einerseits und der Adsorptionsschicht 6 andererseits angeordnet ist.

Erfindungsgemäß ist es insbesondere vorgesehen, dass die Adsorptionsschicht 6, insbesondere die Adsorberpartikel 6' der Adsorptionsschicht 6, nicht der Nanofaserschicht 3 zugeordnet ist bzw. sind bzw. dass die Adsorptionsschicht 6, insbesondere die Adsorberpartikel 6' der Adsorptionsschicht 6, nicht zwischen dem Trägermaterial 2 und der Nanofaserschicht 3 angeordnet ist bzw. sind. Wie zuvor angeführt, verhält es sich erfindungsgemäß nämlich insbesondere derart, dass die Nanofaserschicht 3 unmittelbar bzw. ohne dazwischen angeordnete weitere Schichten dem schaumbasierten bzw. schaumförmigen Trägermaterial 2 zugeordnet bzw. hieran fixiert und/oder zum Haften gebracht ist. Auf diese Weise ist zum einen die Ausbildung eines besonders stabilen Verbundes und zum anderen eine optimale Interaktion bzw. Wirkverstärkung der jeweiligen Schichten 2, 3, 6 im Hinblick auf die Verbindung der insgesamt bereitgestellten Schutzfunktion gewährleistet.

Gemäß einer weiteren erfindungsgemäßen Ausführungsform verhält es sich insbesondere derart, dass die Adsorptionsschicht 6, insbesondere die Adsorberpartikel 6' der Adsorptionsschicht 6, nicht an dem Trägermaterial 2 bzw. nicht an dem textilen Träger 4 fixiert ist bzw. sind bzw. dass die Adsorptionsschicht 6 nicht an dem Trägermaterial 2 bzw. nicht an dem textilen Träger 4 zum Haften gebracht ist. In entsprechender Weise ist es erfindungsgemäß vorgesehen, dass die Adsorptionsschicht 6, insbesondere die Adsorberpartikel 6' der Adsorptionsschicht 6, nicht an der Nanofaserschicht 3 fixiert bzw. nicht hieran zum Haften gebracht ist bzw. sind.

In diesem Zusammenhang kann es sich erfindungsgemäß insbesondere derart verhalten, dass die Adsorptionsschicht 6 als separate bzw. nicht mit dem Trägermaterial 2 bzw. nicht mit der Nanofaserschicht 3 bzw. nicht mit dem textilen Träger 4 verbundene, insbesondere nicht an den vorgenannten Schichten 2, 3, 4 fixierte bzw. nicht hieran zum Haften gebrachte Schicht ausgebildet ist. Die Adsorptionsschicht 6 kann somit sozusagen eine unverbundene bzw. eigenständige Schicht innerhalb des erfindungsgemäßen Flächenfiltermaterials 2 darstellen. Hierdurch kann beispielsweise die Flexibilität bzw. Biegsamkeit des erfindungsgemäßen Flächenfiltermaterials erhöht werden. Zudem resultiert hierdurch eine weiterführende Anpassbarkeit des Flächenfiltermaterials 1 nach der Erfindung insbesondere in Bezug auf den jeweiligen Anwendungs- bzw. Einsatzzweck des Materials.

Was die vorliegende Ausführungsform der vorliegenden Erfindung anbelangt, wonach die Adsorptionsschicht 6 nicht an den weiteren Schichten 2, 3, 4 fixiert bzw. nicht hieran zum Haften gebracht ist, so ist es im Rahmen der vorliegenden Erfindung von Vorteil, wenn die Adsorptionsschicht 6 an einem separaten, insbesondere textilen Träger fixiert bzw. zum Haften gebracht ist. Diesbezüglich können die Adsorptionsschicht 6 und der separate, insbesondere textile Träger einen separaten bzw. eigenständigen bzw. nicht an einem der weiteren Schichten 2, 3, 4 des Flächenfiltermaterials 1 fixierten bzw. nicht an einem der weiteren Schichten 2, 3, 4 des Flächenfiltermaterials 1 zum Haften gebrachten Verbund ausbilden. Infolge des Einsatzes eines (weiteren) textilen Trägers für die Adsorptionsschicht 6 kann deren Stabilität erhöht werden.

Im Rahmen der vorliegenden Erfindung können eine Vielzahl verschiedener Adsorptionsmaterialien für die Adsorberpartikel 6' zur Ausbildung der Adsorptionsschicht 6 eingesetzt werden:
Insbesondere können die Adsorberpartikel 6' der Adsorptionsschicht 6 ausgewählt sein aus der Gruppe von
   (i) insbesondere partikulärer Aktivkohle und/oder Aktivkohlepartikeln, vorzugsweise in Form von Aktivkohleteilchen in Kornform ("Kornkohle") oder Kugelform ("Kugelkohle");
   (ii) Zeolithen, insbesondere natürlichen und/oder synthetischen Zeolithen;
   (iii) Molekularsieben, insbesondere zeolithischen Molekularsieben, synthetischen Molekularsieben und/oder insbesondere synthetischen Molekularsieben auf Basis von Kohlenstoff, Oxiden und/oder Gläsern;
   (iv) Metalloxid- und/oder Metallpartikeln;
   (v) Ionenaustauscherharzen, insbesondere polydispersen und/oder monodispersen Kationen- und/oder Anionenaustauschern, insbesondere vom Geltyp und/oder makroporösen Typ;
   (vi) anorganischen Oxiden, insbesondere Siliciumdioxiden, Silicagelen und/oder Aluminiumoxiden;
   (vii) porösen organischen Polymeren und/oder porösen organisch-anorganischen Hybridpolymeren und/oder metallorganischen Gerüstmaterialien, insbesondere MOFs (*Metall Organic Framework*), COFs (*Covalent Organic Framework*), ZIFs (*Zeolithe Imidazolate Framework*), POMs (*Polymer Organic Material*) und/oder OFCs;
   (viii) mineralischen Granulaten;
   (ix) Klathraten; sowie
   (x) deren Mischungen und/oder Kombinationen.

Gemäß einer erfindungsgemäß besonders bevorzugten Ausführungsform können die Adsorberpatikel 6' der Adsorptionsschicht 6 aus Aktivkohle, insbesondere partikulärer Aktivkohle und/oder Aktivkohlepartikel, vorzugsweise in Form von Aktivkohleteilchen in Kornform ("Kornkohle") oder Kugelform ("Kugelkohle"), gebildet sein bzw. hieraus bestehen.

Die jeweiligen partikelbildenden Materialien der erfindungsgemäß eingesetzten Adsorberpartikel 6' sind dem Fachmann im Allgemeinen als solche wohlbekannt, und der Fachmann ist jederzeit in der Lage, die jeweiligen partikelbildenden Materialien im Lichte der Ausrüstung des erfindungsgemäßen Flächenfiltermaterials mit speziellen Eigenschaften, insbesondere Adsorptionseigenschaften, im Lichte der vorliegenden Erfindung auszuwählen und aufeinander abzustimmen.

Für die erfindungsgemäß einsetzbaren Adsorberpartikel 6' kann auch auf die nachfolgenden Ausführungen verwiesen werden. Darüber hinaus kann zu weiterführenden Einzelheiten zu den erfindungsgemäß gleichermaßen einsetzbaren MOF-Materialien insbesondere verwiesen werden auf die internationale Patentanmeldung WO 2009/096184 A1 sowie auf die parallele deutsche Patentanmeldung DE 10 2008 005 218 A1.

Was den Durchmesser bzw. den mittleren Durchmesser D₅₀ der Adsorberpartikel 6' anbelangt, so kann dieser in weiten Bereichen variieren. Besonders gute Ergebnisse werden jedoch erhalten, wenn der Durchmesser der Adsorberpartikel 6', insbesondere der partikulären Aktivkohle und/oder der Aktivkohlepartikel, im Bereich von 0,005 mm bis 5 mm, vorzugsweise im Bereich von 0,01 mm bis 2,5 mm, bevorzugt im Bereich von 0,015 mm bis 1 mm, besonders bevorzugt im Bereich von 0,02 mm bis 0,8 mm, ganz besonders bevorzugt im Bereich von 0,03 mm bis 0,6 mm, liegt.

Zudem kann der mittlere Durchmesser D₅₀ der Adsorberpartikel (6'), insbesondere der partikulären Aktivkohle und/oder der Aktivkohlepartikel, im Bereich von 0,01 mm bis 4 mm, insbesondere im Bereich von 0,02 mm bis 2 mm, bevorzugt im Bereich von 0,03 mm bis 0,75 mm, besonders bevorzugt im Bereich von 0,04 mm bis 0,6 mm, ganz besonders bevorzugt im Bereich von 0,05 mm bis 0,55 mm, liegen.

Die entsprechenden Teilchengrößen können insbesondere auf Basis der Methode nach ASTM D2862-97/04 bestimmt werden. Zudem können die vorgenannten Größen mit Bestimmungsmethoden auf Basis einer Siebanalyse, auf Basis von Röntgenbeugung, Laserdiffraktometrie oder dergleichen bestimmt werden. Die jeweiligen Bestimmungsmethoden sind dem Fachmann als solche wohlbekannt, so dass es diesbezüglich keiner weiteren Ausführungen bedarf.

Im Allgemeinen können die Adsorberpartikel 6', insbesondere die partikuläre Aktivkohle und/oder die Aktivkohlepartikel, in einer Menge im Bereich von 5 g/m² bis 500 g/m², insbesondere im Bereich von 10 g/m² bis 400 g/m², vorzugsweise im Bereich von 15 g/m² bis 300 g/m², bevorzugt im Bereich von 20 g/m² bis 250 g/m², besonders bevorzugt im Bereich von 30 g/m² bis 225 g/m², ganz besonders bevorzugt im Bereich von 40 g/m² bis 200 g/m², weiter bevorzugt im Bereich von 60 g/m² bis 200 g/m², eingesetzt sein bzw. vorliegen.

Insbesondere kann das Flächenfiltermaterial 1, insbesondere die Adsorptionsschicht 6, die Adsorberpartikel 6', insbesondere die partikuläre Aktivkohle und/oder die Aktivkohlepartikel, in einer Menge im Bereich von 5 g/m² bis 500 g/m², insbesondere im Bereich von 10 g/m² bis 400 g/m², vorzugsweise im Bereich von 15 g/m² bis 300 g/m², bevorzugt im Bereich von 20 g/m² bis 250 g/m², besonders bevorzugt im Bereich von 30 g/m² bis 225 g/m², ganz besonders bevorzugt im Bereich von 40 g/m² bis 200 g/m², weiter bevorzugt im Bereich von 60 g/m² bis 200 g/m², aufweisen. Erfindungsgemäß können aufgrund der insbesondere durch die Nanofaserschicht 3 bereitgestellten Aerosol- bzw. Partikelfilterfunktion entsprechend geringe Auftragsmengen an Adsorberpartikeln 6' realisiert werden, was zu einer weiteren Verringerung des Flächengewichts des erfindungsgemäßen Flächenfiltermaterials 1 insgesamt führt.

Was die im Rahmen der vorliegenden Erfindung verwendete(n) bzw. eingesetzte(n) Aktivkohle bzw. partikuläre Aktivkohle bzw. Aktivkohlepartikel (nachfolgend auch nur als Aktivkohle bezeichnet) als solche anbelangt, so werden die vorliegend aufgeführten Parameterangaben bezüglich der zugrundeliegenden Aktivkohle bzw. partikulären Aktivkohle bzw. Aktivkohlepartikel mit genormten oder explizit angegebenen Bestimmungsverfahren oder mit dem Fachmann an sich geläufigen Bestimmungsmethoden bestimmt. Insbesondere die Parameterangaben betreffend die Charakterisierung der Porosität der Porengrößenverteilung und andere Adsorptionseigenschaften ergeben sich im Allgemeinen jeweils aus den entsprechenden Stickstoffsorptionsisothermen der betreffenden Aktivkohle bzw. der vermessenen Produkte. Zudem kann die Porenverteilung, insbesondere auch im Hinblick auf den Gehalt an Mikroporen in Bezug auf das Gesamtporenvolumen, auf Basis der DIN 66135-1 bestimmt werden.

Gemäß einer erfindungsgemäß bevorzugten Ausführungsform, wonach als Adsorberpartikel 6' bzw. als Adsorptionsmaterial Aktivkohle bzw. Aktivkohlepartikel eingesetzt wird bzw. werden, kann die Aktivkohle erhältlich sein durch Carbonisierung und nachfolgende Aktivierung eines synthetischen und/oder nicht naturstoffbasierten Ausgangsmaterials, insbesondere auf Basis organischer Polymere.

In diesem Zusammenhang kann die Aktivkohle aus einem Ausgangsmaterial auf Basis organischer Polymere, insbesondere auf Basis sulfonierter organischer Polymere, vorzugsweise auf Basis von divinylbenzolvernetztem Polystyrol, bevorzugt auf Basis von Styrol/Divinylbenzol-Copolymeren, erhalten wird, insbesondere durch Carbonisierung und nachfolgende Aktivierung des Ausgangsmaterials. In diesem Zusammenhang kann der Gehalt an Divinylbenzol in dem Ausgangsmaterial im Bereich von 1 Gew.-% bis 20 Gew.-%, insbesondere im Bereich von 1 Gew.-% bis 15 Gew.-%, vorzugsweise im Bereich von 1,5 Gew.-% bis 12,5 Gew.-%, bevorzugt im Bereich von 2 Gew.-% bis 10 Gew.-%, liegen.

Erfindungsgemäß kann das Ausgangsmaterial für Aktivkohle ein insbesondere sulfoniertes und/oder Sulfonsäuregruppen enthaltendes lonenaustauscherharz, insbesondere vom Geltyp, sein.

Gemäß einer erfindungsgemäß besonders bevorzugten Ausführungsform kann als Aktivkohle eine polymerbasierte sphärische Aktivkohle (PBSAC; *Polymer-based Spherical Activated Carbon*) eingesetzt sein.

Insbesondere kann die Aktivkohle eine polymerbasierte sphärische Aktivkohle (PBSAC) sein. Derartige Aktivkohlen zeichnen sich durch hervorragende Adsorptionseigenschaften gegenüber dem zuvor genannten Schad- bzw. Giftstoffen und durch hervorragende mechanische Eigenschaften, wie eine hohe Materialhärte sowie eine hohe Abriebshärte, aus.

Die eingesetzte Aktivkohle kann dabei grundsätzlich nach bekannten Verfahren des Standes der Technik erhalten werden: Insbesondere werden zu diesem Zweck kugelförmige sulfonierte organische Polymere, insbesondere auf Basis von divinylbenzolvernetztem Polystyrol, carbonisiert und anschließend zu der betreffenden Aktivkohle aktiviert, insbesondere wie zuvor angeführt. Für weitergehende diesbezügliche Einzelheiten kann beispielsweise auf die Druckschriften DE 43 28 219 A1, DE 43 04 026 A1, DE 196 00 237 A1 sowie EP 1 918 022 A1 bzw. auf die parallele, zu derselben Patentfamilie gehörende US 7,737,038 B2 verwiesen werden.

Im Rahmen der vorliegenden Erfindung zum Einsatz kommende Aktivkohlen bzw. Aktivkohlepartikel sind im Allgemeinen kommerziell erhältlich bzw. handelsüblich. Insbesondere können Aktivkohlen zum Einsatz kommen, welche beispielsweise von der Blücher GmbH, Erkrath, Deutschland, hergestellt bzw. vertrieben werden.

Im Rahmen der vorliegenden Erfindung hat es sich als vorteilhaft erwiesen, wenn die erfindungsgemäß eingesetzte Aktivkohle ein weiterführend spezifiziertes Gesamtporenvolumen, insbesondere ein Gesamtporenvolumen nach Gurvich, aufweist:
In diesem Zusammenhang kann die Aktivkohle ein Gesamtporenvolumen, insbesondere ein Gesamtporenvolumen nach Gurvich, im Bereich von 0,4 cm³/g bis 3,9 cm³/g, insbesondere im Bereich von 0,45 cm³/g bis 3,5 cm³/g, vorzugsweise im Bereich von 0,5 cm³/g bis 3 cm³/g, besonders bevorzugt im Bereich von 0,6 cm³/g bis 2,5 cm³/g, ganz besonders bevorzugt im Bereich von 0,5 cm³/g bis 1,5 cm³/g, aufweisen.

Insbesondere können mindestens 65 %, insbesondere mindestens 70 %, vorzugsweise mindestens 75 %, bevorzugt mindestens 80 %, des Gesamtporenvolumens, insbesondere des Gesamtporenvolumens nach Gurvich, der Aktivkohle durch Poren mit Porendurchmessern von höchstens 50 nm, insbesondere durch Mikro- und/oder Mesoporen, gebildet sein.

Insbesondere können 50 % bis 95 %, insbesondere 60 % bis 90 %, vorzugsweise 70 % bis 85 %, des Gesamtporenvolumens, insbesondere des Gesamtporenvolumens nach Gurvich, der Aktivkohle durch Poren mit Porendurchmessern von höchstens 50 nm, insbesondere durch Mikro- und/oder Mesoporen, gebildet sein.

Zudem können 1 % bis 60 %, insbesondere 5 % bis 50 %, vorzugsweise 10 % bis 40 %, bevorzugt 15 % bis 35 %, des Gesamtporenvolumens, insbesondere des Gesamtporenvolumens nach Gurvich, der Aktivkohle durch Poren mit Porendurchmessern von mehr als 2 nm, insbesondere durch Meso- und/oder Makroporen, gebildet sein.

Insbesondere kann die Aktivkohle ein durch Poren mit Porendurchmessern von höchstens 2 nm (d.h. ≤ 2 nm) gebildetes Porenvolumen, insbesondere Mikroporenvolumen nach Carbon Black, im Bereich von 0,05 cm³/g bis 2,5 cm³/g, insbesondere im Bereich von 0,15 cm³/g bis 2 cm³/g, vorzugsweise im Bereich von 0,3 cm³/g bis 1,5 cm³/g, aufweisen.

Insbesondere können 15 % bis 98 %, insbesondere 25 % bis 95 %, vorzugsweise 35 % bis 90 %, des Gesamtporenvolumens der Aktivkohle durch Poren mit Porendurchmessern von höchstens 2 nm, insbesondere durch Mikroporen, gebildet sein.

In Bezug auf erfindungsgemäß einsetzbare mikroporöse Aktivkohle kann zudem auf die auf die Anmelderin selbst zurückgehende europäische Patentanmeldung EP 1 918 022 A1 sowie auf die parallele US 2008/0107589 A1 verwiesen werden, deren jeweilige Offenbarung hiermit durch Bezugnahme vollumfänglich eingeschlossen ist.

Was die Bestimmung des Gesamtporenvolumens nach Gurvich anbelangt, so handelt es sich um eine dem Fachmann auf diesem Gebiet an sich wohlbekannte Mess-/Bestimmungsmethode. Zu weitergehenden Einzelheiten bezüglich der Bestimmung des Gesamtporenvolumens nach Gurvich kann beispielsweise verwiesen werden auf L. Gurvich (1915), J. Phys. Chem. Soc. Russ. 47, 805*,* sowie auf S. Lowell et al., Characterization of Porous Solids and Powders: Surface Area Pore Size and Density, Kluwer Academic Publishers, Article Technology Series, Seiten 111 ff*.* Insbesondere kann das Porenvolumen der Aktivkohle auf Basis der Gurvich-Regel gemäß der Formel V_{P} = Wₐ / ρₗ bestimmt werden, wobei Wₐ die adsorbierte Menge eines zugrundeliegenden Adsorbats und ρₗ die Dichte des eingesetzten Adsorbats darstellt (vgl. auch Formel (8.20) gemäß Seite 111, Kapitel 8.4.) von S. Lowell et al.).

Die Bestimmungsmethode nach Carbon Black ist dem Fachmann an sich bekannt, wobei zudem für weitergehende Einzelheiten zur Bestimmung der Porenoberfläche und des Porenvolumens nach Carbon Black beispielsweise verwiesen werden kann auf R. W. Magee, Evaluation of the External Surface Area of Carbon Black by Nitrogen Adsorption, Presented at the Meeting of the Rubber Division of the American Chem. Soc., October 1994*,* z. B. referiert in: Quantachrome Instruments, AUTOSORB-1, AS1 WinVersion 1.50, Operating Manual, OM, 05061, Quantachrome Instruments 2004, Florida, USA, Seiten 71 ff*.* Insbesondere kann die diesbezügliche Auswertung mittels *t-plot-method* erfolgen.

Für weitergehende Einzelheiten zur Bestimmung der BET-Oberfläche bzw. zu der BET-Methode kann auf die vorgenannte ASTM D6556-04 sowie auf Römpp Chemielexikon, 10. Auflage, Georg Thieme Verlag, Stuttgart / New York, Stichwort: "BET-Methode", einschließlich der dort referierten Literatur, und auf Winnacker-Küchler (3. Auflage), Band 7, Seiten 93 ff. sowie auf Z. Anal. Chem. 238, Seiten 187 bis 193 (1968), verwiesen werden.

Im Rahmen der vorliegenden Erfindung bezeichnet der Begriff "Mikroporen" solche Poren mit Porendurchmessern von weniger als 2 nm, wohingegen der Begriff "Mesoporen" solche Poren mit Porendurchmessern im Bereich von 2 nm (d.h. 2 nm einschließlich) bis 50 nm einschließlich bezeichnet und der Begriff "Makroporen" solche Poren mit Porendurchmessern von mehr als 50 nm (d.h. > 50 nm) bezeichnet.

Weiterhin kann die Aktivkohle eine spezifische BET-Oberfläche im Bereich von 600 m²/g bis 4.500 m²/g, insbesondere im Bereich 800 m²/g bis 3.500 m²/g, vorzugsweise im Bereich 1.000 m²/g bis 3.000 m²/g, besonders bevorzugt im Bereich 1.200 m²/g bis 2.750 m²/g, ganz besonders bevorzugt im Bereich 1.300 m²/g bis 2.500 m²/g, aufweisen.

Die Bestimmung der spezifischen Oberfläche gemäß BET ist dem Fachmann grundsätzlich als solche bekannt, so dass diesbezüglich keine weitergehenden Einzelheiten ausgeführt zu werden brauchen. Sämtliche BET-Oberflächenangaben beziehen sich auf die Bestimmung gemäß ASTM D6556-04. Im Rahmen der vorliegenden Erfindung wird zur Bestimmung der BET-Oberfläche - im Allgemeinen und sofern nicht ausdrücklich abweichend angegeben - die so genannte MultiPoint-BET-Bestimmungsmethode (MP-BET) in einem Partialdruckbereich p/p₀ von 0,05 bis 0,1 angewendet.

Insbesondere kann die Aktivkohle eine durch Poren mit Porendurchmessern von höchstens 2 nm, insbesondere durch Mikroporen, gebildete Oberfläche im Bereich von 400 bis 3.500 m²/g, insbesondere im Bereich von 500 bis 3.000 m²/g, vorzugsweise im Bereich von 600 bis 2.500 m²/g, bevorzugt im Bereich 700 bis 2.000 m²/g, aufweisen.

Im Allgemeinen kann die Aktivkohle eine durch Poren mit Porendurchmessern im Bereich von 2 nm bis 50 nm, insbesondere durch Mesoporen, gebildete Oberfläche im Bereich von 200 bis 2.000 m²/g, insbesondere im Bereich von 300 bis 1.900 m²/g, vorzugsweise im Bereich von 400 bis 1.800 m²/g, bevorzugt im Bereich von 500 bis 1.700 m²/g, aufweisen. Die erfindungsgemäß verwendbare Aktivkohle kann zudem einen mittleren Porendurchmesser im Bereich von 0,1 nm bis 55 nm, insbesondere im Bereich von 0,2 nm bis 50 nm, vorzugsweise im Bereich von 0,5 nm bis 45 nm, bevorzugt im Bereich von 1 nm bis 40 nm, aufweisen.

Das Flächenfiltermaterial 1 nach der Erfindung weist insgesamt hervorragende Eigenschaften auf, wie nachfolgend angeführt:
So kann das Flächenfiltermaterial 1 nach der Erfindung selbsttragend sein. Im Allgemeinen kann das Flächenfiltermaterial 1 als Verbundmaterial ausgebildet sein. Dabei weist das erfindungsgemäße Flächenfiltermaterial 1 insbesondere eine hohe mechanische Stabilität auf, und dies bei gleichzeitig hoher Biegsamkeit des Materials insgesamt.

Insbesondere kann das Flächenfiltermaterial 1 nach der Erfindung ein Gesamtflächengewicht im Bereich von 10 g/m² bis 800 g/m², insbesondere im Bereich von 20 g/m² bis 600 g/m², vorzugsweise im Bereich von 50 g/m² bis 400 g/m², bevorzugt im Bereich von 75 g/m² bis 300 g/m², besonders bevorzugt im Bereich von 100 g/m² bis 200 g/m², aufweisen.

Zudem kann das Flächenfiltermaterial 1 eine Luftdurchlässigkeit von mindestens 10 l·m⁻²·s⁻¹, insbesondere mindestens 25l·m⁻²·s⁻¹, vorzugsweise mindestens 30 l·m⁻²·s⁻¹, bevorzugt mindestens 40 l·m⁻²·s⁻¹, besonders bevorzugt mindestens 50 l·m⁻²·s⁻¹, ganz besonders bevorzugt mindestens 100 l·m⁻²·s⁻¹, bei einem Strömungswiderstand von 127 Pa, aufweisen.

Wie zuvor angeführt, kann das Flächenfiltermaterial 1 wasserdicht und/oder wasserabweisend ausgebildet sein. Insbesondere kann das Flächenfiltermaterial 1 eine Wassersäule von mindestens 500 mm, insbesondere mindestens 600 mm, vorzugsweise mindestens 700 mm, gemessen nach DIN EN 20 811:1992 (August 1992), aufweisen. Erfindungsgemäß kann es zudem vorgesehen sein, dass das Flächenfiltermaterial 1 eine Wassersäule im Bereich von 500 mm bis 2.500 mm, insbesondere im Bereich von 600 mm bis 2.000 mm, vorzugsweise im Bereich von 700 mm bis 1.500 mm, gemessen nach DIN EN 20 811:1992 (August 1992), aufweist.

Zudem kann das Flächenfiltermaterial 1 bei 25 °C eine Wasserdampfdurchlässigkeit von mindestens 10 l/m² pro 24 h, insbesondere mindestens 20 l/m² pro 24 h, vorzugsweise mindestens 30 l/m² pro 24 h, aufweisen.

In diesem Zusammenhang kann das Flächenfiltermaterial 1 einen Wasserdampfdurchgangswiderstand Rₑₜ unter stationären Bedingungen, gemessen nach DIN EN 31 092:1993 (Februar 1994) und internationaler Norm ISO 11 092, bei 35 °C, von höchstens 25 (m²·Pascal)/Watt, insbesondere höchstens 20 (m²·Pascal)/Watt, vorzugsweise höchstens 10 (m²·Pascal)/Watt, aufweisen.

Weiterhin kann das Flächenfiltermaterial 1 eine Gesamtdicke im Bereich von 0,1 mm bis 30 mm, insbesondere im Bereich von 0,2 mm bis 20 mm, vorzugsweise im Bereich von 0,5 mm bis 10 mm, bevorzugt im Bereich von 0,6 mm bis 6 mm, besonders bevorzugt im Bereich von 0,8 mm bis 2 mm, aufweisen.

Darüber hinaus kann das Flächenfiltermaterial 1 einen mittleren Wirkungsgrad Eₘ nach DIN EN 779 (Juli 1993) von mindestens 50 %, insbesondere mindestens 60 %, vorzugsweise mindestens 80 %, besonders bevorzugt mindestens 95 %, ganz besonders bevorzugt mindestens 97 %, aufweisen. Zudem kann das Flächenfiltermaterial 1 einen mittleren Abscheidegrad Aₘ nach DIN EN 779 (Juli 1993) von mindestens 60 %, insbesondere mindestens 80 %, vorzugsweise mindestens 95 %, besonders bevorzugt mindestens 99 %, ganz besonders bevorzugt mindestens 99,5 %, aufweisen.

Zudem kann das Flächenfiltermaterial 1 eine Abscheidegradleistung nach DIN EN 1822 (April 1998) von mindestens 50 %, insbesondere mindestens 60 %, vorzugsweise mindestens 80 %, besonders bevorzugt mindestens 95 %, ganz besonders bevorzugt mindestens 97 %, aufweisen.

Weiterhin kann das erfindungsgemäße Flächenfiltermaterial 1 auch eine hervorragende Schutzfunktion gegenüber Schad- bzw. Giftstoffen, insbesondere chemischen Kampfstoffen, aufweisen: So kann das Flächenfiltermaterial 1 nach der Erfindung eine Barrierewirkung gegenüber Schad- und/oder Giftsoffen, insbesondere chemischen Kampfstoffen, insbesondere Bis[2-chlorethyl]sulfid, bestimmt nach Methode 2.2 der CRDC-SP-84010, von höchstens 4,2 µg/cm² pro 24 h, insbesondere höchstens 4 µg/cm² pro 24 h, vorzugsweise höchstens 3,5 µg/cm² pro 24 h, bevorzugt höchstens 3µg/cm² pro 24 h, besonders bevorzugt höchstens 2,5 µg/cm² pro 24 h, ganz besonders bevorzugt höchstens 2 µg/cm² pro 24 h, aufweisen.

Die vorliegende Erfindung betrifft auch den erfindungsgemäßen Aspekt, wonach das textile Flächenfiltermaterial 1 nach der Erfindung durch das nachfolgend beschriebene Verfahren zu seiner Herstellung erhältlich ist. Die vorliegende Erfindung betrifft somit auch ein textiles Flächenfiltermaterial 1, insbesondere mit Aerosol- und/oder Partikelfilterfunktion, vorzugsweise mit Schutzfunktion gegenüber chemischen, biologischen und/oder chemischen Schad- und Giftstoffen, wobei das Flächenfiltermaterial 1 erhältlich ist durch das nachfolgend beschriebene Verfahren nach der Erfindung.

Weiterer Gegenstand der vorliegenden Erfindung - gemäß einem **weiteren** Aspekt der vorliegenden Erfindung - ist zudem ein Verfahren zur Herstellung eines insbesondere wie zuvor beschriebenen Flächenfiltermaterials mit Aerosol- und/oder Partikelfilterfunktion und mit Schutzfunktion gegenüber chemischen, biologischen und/oder radioaktiven Schad- und Giftstoffen, wobei das Verfahren die nachfolgenden Schritte umfasst:
(a) Bereitstellung eines luft- und/oder wasserdampfdurchlässigen, flächigen schaumbasierten und/oder schaumförmigen Trägermaterials mit zwei gegenüberliegenden Flachseiten, wobei das Trägermaterial (2) eine luftdurchlässige und/oder diskontinuierlich ausgebildete Schicht auf Basis einer getrockneten oder ausgehärteten, vorzugsweise vernetzten, gebrochenen Polymerschaumstruktur ist, wobei die gebrochene Polymerschaumstruktur offenporig und/oder nicht geschlossen ausgebildet wird;
(b) Aufbringen einer luft- und/oder wasserdampfdurchlässigen, vorzugsweise flächigen Nanofaserschicht, insbesondere Aerosol- und/oder Partikelfilter-Nanofaserschicht, wobei die Nanofaserschicht eine Vielzahl von Nanofasern umfasst oder hieraus besteht, an einer (ersten) Flachseite des schaumbasierten und/oder schaumförmigen Trägermaterials, derart, dass die Nanofaserschicht an der (ersten) Flachseite des Trägermaterials fixiert und/oder zum Haften gebracht wird;
wobei das Flächenfiltermaterial (1) weiterhin mit mindestens einer Adsorptionsschicht (6) ausgerüstet und/oder versehen wird, wobei die Adsorptionsschicht (6) eine Vielzahl einzelner und/oder diskreter Adsorberpartikel (6') umfasst oder hieraus gebildet wird, wobei die Adsorptionsschicht (6) zwei gegenüberliegende Flachseiten (6a, 6b) aufweist, wobei die Adsorptionsschicht (6) dem Trägermaterial (2) zugeordnet wird und wobei die Adsorptionsschicht (6) auf der der Nanofaserschicht (3) abgewandten (zweiten) Seite (2b) des Trägermaterials (2) angeordnet wird.

Gemäß einer erfindungsgemäß bevorzugten Ausführungsform kann es im Rahmen des Verfahrens nach der Erfindung vorgesehen sein, dass Flächenfiltermaterial mit (c) einem textilen Träger ausgerüstet bzw. versehen wird. Somit kann eine zusätzliche Schicht (c) in Form eines textilen Trägers vorgesehen werden. In diesem Zusammenhang kann der textile Träger dem Trägermaterial zugeordnet werden. Insbesondere kann der textile Träger auf der der Nanofaserschicht abgewandten (zweiten) Seite des Trägermaterials angeordnet werden.

Im Rahmen der Herstellung des Schutzmaterials nach der Erfindung kann dabei im Allgemeinen derart vorgegangen werden, dass ein zunächst intakter nicht getrockneter bzw. nicht (vollständig) ausgehärteter Schaum zur Bereitstellung des schaumbasierten bzw. schaumförmigen Trägermaterials auf einem textilen Träger bzw. auf einer entfernbaren Trägerschicht oder dergleichen aufgebracht wird, wobei beispielsweise die Trocknungsbedingungen (Temperatur oder dergleichen) derart gewählt werden können, dass im Rahmen der Trocknung eine Zerstörung bzw. eine Öffnung der zugrundeliegenden Porenstruktur erfolgt. In diesem Zusammenhang kann beispielsweise auch eine Eintragung von mechanischer Energie in das Schaumsystem erfolgen bzw. durchgeführt werden. So kann im Rahmen der Aushärtung bzw. Trocknung eine (Auf-)brechnung bzw. Zerstörung bzw. ein Zerfall bzw. eine Öffnung des Schaumsystems zum Erhalt des gebrochenen bzw. offenporigen Schaums bzw. Polymerschaums bzw. des entsprechenden schaumbasierten bzw. schaumförmigen Trägermaterials im getrockneten bzw. ausgehärteten Zustand erfolgen, wobei der schichtartige, zusammenhängende Aufbau des Trägermaterials als solcher grundsätzlich erhalten bleibt. Nachfolgend kann bzw. können auf das so erhaltene schaumbasierte bzw. schaumförmige Trägermaterial die Nanofaserschicht bzw. die der Nanofaserschicht zugrundeliegenden Nanofasern aufgebracht werden, beispielsweise mittels Elektrospinnen oder dergleichen. Die erfindungsgemäße Konzeption ermöglicht dabei auch eine homogene bzw. gleichmäßige Ausrüstung des Trägermaterials mit den Nanofasern bzw. mit der Nanofaserschicht, wobei in diesem Zusammenhang auch geringe Flächengewichte des Trägermaterials bzw. der Nanofaserschicht realisiert werden können.

Gemäß einer ersten erfindungsgemäßen Ausführungsform kann zur Herstellung des erfindungsgemäßen Filtermaterials wie folgt vorgegangen werden:
- Gemäß dieser Ausführungsform kann die Bereitstellung bzw. Herstellung des Trägermaterials durch Aufbringen, insbesondere Fixieren bzw. Zumhaftenbringen, des Trägermaterials auf einem textilen Träger erfolgen.
- In diesem Zusammenhang kann das Trägermaterial insbesondere in Form einer aufgeschäumten, vorzugsweise unter mechanischem Energieeintrag aufgeschäumten, wässrig oder organisch basierten, vorzugsweise wässrig basierten, Lösung bzw. Dispersion eines Schaumstrukturmaterials (Schaumstrukturpolymer) auf dem textilen Träger aufgebracht werden.
- Insbesondere kann nachfolgend eine Trocknung bzw. Aushärtung, insbesondere Vernetzung, des Trägermaterials in Form der aufgeschäumten Lösung bzw. Dispersion des Schaumstrukturmaterials durchgeführt werden, insbesondere einhergehend mit einer offenporigen Ausbildung des durch die aufgeschäumte Lösung und/oder Dispersion des Schaumstrukturmaterials bereitgestellten Schaums bzw. insbesondere einhergehend mit einer Brechung des durch die aufgeschäumte Lösung und/oder Dispersion des Schaumstrukturmaterials bereitgestellten Schaums, insbesondere so dass das Trägermaterial als getrockneter bzw. ausgehärteter, insbesondere vernetzter, offenporiger bzw. gebrochener Schaum des Schaumstrukturmaterials erhalten wird.
- Insbesondere kann das Trägermaterial auf diese Weise an dem textilen Träger fixiert und/oder zum Haften gebracht werden.

Gemäß einer zweiten erfindungsgemäßen Ausführungsform kann alternativ auch wie folgt vorgegangen werden:
- So kann die Bereitstellung bzw. Herstellung des Trägermaterials durch Aufbringen, des Trägermaterials auf einer entfernbaren, insbesondere abziehbaren Trägerschicht, vorzugsweise auf einem Trägerpapier, erfolgen.
- In diesem Zusammenhang kann das Trägermaterial in Form einer aufgeschäumten, vorzugsweise unter mechanischem Energieeintrag aufgeschäumten, wässrig oder organisch basierten, vorzugsweise wässrig basierten, Lösung bzw. Dispersion eines Schaumstrukturmaterials (Schaumstrukturpolymer) auf der entfernbaren Trägerschicht aufgebracht werden.
- Nachfolgend kann eine Trocknung bzw. Aushärtung, insbesondere Vernetzung, des Trägermaterials in Form der aufgeschäumten Lösung bzw. Dispersion des Schaumstrukturmaterials durchgeführt werden, insbesondere einhergehend mit einer offenporigen Ausbildung des durch die aufgeschäumte Lösung bzw. Dispersion des Schaumstrukturmaterials bereitgestellten Schaums bzw. insbesondere einhergehend mit einer Brechung des durch die aufgeschäumte Lösung bzw. Dispersion des Schaumstrukturmaterials bereitgestellten Schaums, insbesondere so dass das Trägermaterial als getrockneter bzw. ausgehärteter, insbesondere vernetzter, offenporiger bzw. gebrochener Schaum bzw. gebrochene Schaumstruktur des Schaumstrukturmaterials erhalten wird.
- Anschließend kann das so erhaltene Trägermaterial, insbesondere in Form des getrockneten bzw. ausgehärteten, insbesondere vernetzten, offenporigen bzw. gebrochenen Schaum des Schaumstrukturmaterials, von der entfernbaren Trägerschicht abgelöst, insbesondere abgezogen, werden.
- In diesem Zusammenhang kann nachfolgend das so erhaltene Trägermaterial auf einem textilen Träger aufgebracht, insbesondere hierauf fixiert bzw. zum Haften gebracht werden, insbesondere mittels eines vorzugsweise diskontinuierlich-punktförmig aufgetragenen Haftmittels.
- Gemäß dieser erfindungsgemäßen Ausführungsform kann die Nanofaserschicht auf das Trägermaterial vor oder nach Entfernen bzw. Ablösen der entfernbaren Trägerschicht aufgebracht werden, insbesondere wie vorliegend beschrieben.

Was die vorgenannten Ausführungsformen des erfindungsgemäßen Verfahrens insgesamt anbelangt, so kann das Aufschäumen der aufgeschäumten Lösung bzw. Dispersion des Schaumstrukturmaterials durch Gas- bzw. Lufteintrag in die Lösung bzw. Dispersion des Schaumstrukturmaterials erfolgen. Dabei kann der Gas- bzw. Lufteintrag durch Eindüsen, Einrühren, Vibrationseintrag, Schütteleintrag, Einblasen von Gas und/oder Luft und/oder durch Gas- und/oder Lufteintrag mittels Scherkrafteintrag in die Lösung bzw. Dispersion des Schaumstrukturmaterials erfolgen. In diesem Zusammenhang kann die aufgeschäumte Lösung bzw. Dispersion des Schaumstrukturmaterials auf ein Schaumlitergewicht im Bereich von 50 g/l bis 500 g/l, insbesondere im Bereich von 75 g/l bis 400 g/l, vorzugsweise im Bereich von 100 g/l bis 300 g/l, eingestellt werden.

Im Allgemeinen kann das Aufbringen des Trägermaterials auf dem textilen Träger bzw. auf der entfernbaren Trägerschicht mittels (Auf-)Walzen und/oder Aufrakeln durchgeführt werden. Insbesondere kann das (Auf-)Walzen und/oder Aufrakeln mittels Walzenspaltrakeln (*Knife-over-Roll*), Luftrakeln (*Knife-on-Air*), Schablonenauftrag und/oder Auftrag unter Verwendung von Direkte-Rolle-auf-Rolle-Systemen (*Direct-Roll-on-Roll*) durchgeführt werden. Zudem kann das (Auf-) Walzen bzw. Auftragen mittels eines offenen Rakelsystems (*Open Squegee System*) und/oder eines geschlossenen Rakelsystems (*Closed Squegee System*) durchgeführt werden.

Im Allgemeinen kann das Trägermaterial in Form der aufgeschäumten Lösung und/oder Dispersion des Schaumstrukturmaterials in einer Menge im Bereich von 10 g/m² bis 150 g/m², insbesondere im Bereich von 20 g/m² bis 100 g/m², vorzugsweise im Bereich von 40 g/m² bis 80 g/m², auf dem textilen Träger oder auf der entfernbaren Trägerschicht aufgebracht werden.

Zudem kann das Trägermaterial in Form der aufgeschäumten Lösung bzw. Dispersion des Schaumstrukturmaterials in einer Dicke im Bereich von 0,01 mm bis 2 mm, insbesondere im Bereich von 0,05 mm bis 1,5 mm, vorzugsweise im Bereich von 0,1 mm bis 0,75 mm, bevorzugt im Bereich von 0,15 mm bis 0,5 mm, auf dem textilen Träger oder auf der entfernbaren Trägerschicht aufgebracht werden. Zudem kann die Trocknung bzw. Aushärtung des Trägermaterials bei einer Temperatur im Bereich von 30 °C bis 250 °C, insbesondere 50 °C bis 200 °C, vorzugsweise 70 °C bis 180 °C, bevorzugt 90 °C bis 160 °C, durchgeführt werden.

Zudem kann die Trocknung und/oder Aushärtung für eine Zeitdauer im Bereich von 0,1 min bis 15 min, insbesondere 0,5 min bis 10 min, vorzugsweise 1 min bis 5 min, durchgeführt werden.

Das Aufbringen der Nanofaserschicht, insbesondere der Nanofasern, kann mittels Elektrospinnen, Spunbonding-Verfahren, Meltblow-Verfahren oder eine Kombination der vorgenannten Verfahren, vorzugsweise mittels Elektrospinnen bzw. Elektrospinning (Elektrospinnverfahren), erfolgen.

Erfindungsgemäß hat es sich als vorteilhat erwiesen, wenn die Nanofaserschicht, insbesondere die Nanofasern, mit einem Flächengewicht im Bereich von 0,2 g/m² bis 60 g/m², insbesondere im Bereich von 0,5 g/m² bis 40 g/m², vorzugsweise im Bereich von 0,8 g/m² bis 20 g/m², bevorzugt im Bereich von 0,9 g/m² bis 10 g/m², besonders bevorzugt im Bereich von 0,95 g/m² bis 5 g/m², ganz besonders bevorzugt im Bereich von 1 g/m² bis 3 g/m², auf dem Trägermaterial aufgebracht wird bzw. werden.

Im Allgemeinen kann es zudem vorgesehen sein, dass die Nanofaserschicht, insbesondere die Nanofasern, mit einer Dicke der Nanofaserschicht bzw. zum Erhalt einer Dicke der Nanofaserschicht von höchstens 100 µm, insbesondere höchstens 50 µm, vorzugsweise höchstens 20 µm, bevorzugt höchstens 10 µm, besonders bevorzugt höchstens 5 µm, auf das Trägermaterial aufgebracht wird bzw. werden.

Im Allgemeinen kann es zudem vorgesehen sein, dass die Nanofaserschicht, insbesondere die Nanofasern, mit einer Dicke der Nanofaserschicht bzw. zum Erhalt einer Dicke der Nanofaserschicht im Bereich von 0,001 µm bis 100 µm, insbesondere im Bereich von 0,005 µm bis 50 µm, vorzugsweise im Bereich von 0,008 µm bis 20 µm, bevorzugt im Bereich von 0,01 µm bis 10 µm, besonders bevorzugt im Bereich von 0,15 µm bis 8 µm, ganz besonders bevorzugt im Bereich von 1 µm bis 7 µm, weiter bevorzugt im Bereich von 2 µm bis 6 µm, noch weiter bevorzugt im Bereich von 3 µm bis 5 µm, wiederum weiter bevorzugt im Bereich von 4 µm bis 5 µm, auf dem Trägermaterial aufgebracht wird bzw. werden. Weiterhin kann es vorgesehen sein, dass die Nanofaserschicht, insbesondere die Nanofasern, mit einer Dichte der Nanofaserschicht bzw. zum Erhalt einer Dichte der Nanofaserschicht im Bereich von 10 kg/m³ bis 1.000 kg/m³, insbesondere im Bereich von 100 kg/m³ bis 800 kg/m³, vorzugsweise im Bereich von 150 kg/m³ bis 500 kg/m³, bevorzugt im Bereich von 200 kg/m³ bis 300 kg/m³, auf dem Trägermaterial aufgebracht wird bzw. werden.

Im Rahmen der erfindungsgemäßen Verfahrensführung kann insbesondere derart vorgegangen werden, dass die Nanofaserschicht, insbesondere die Nanofasern, derart auf dem Trägermaterial aufgebracht bzw. zum Haften gebracht werden, dass eine zumindest im Wesentlichen vollflächige Belegung des Trägermaterials mit der Nanofaserschicht, insbesondere mit den Nanofasern, erfolgt. Dabei sollte so vorgegangen werden, dass mindestens 80 %, insbesondere mindestens 90 %, vorzugsweise mindestens 95 %, bevorzugt mindestens 99 %, bevorzugt mindestens 99,5 %, einer (ersten) Seite des Trägermaterials mit der Nanofaserschicht, insbesondere mit den Nanofasern, belegt wird.

Im Allgemeinen sollte das Aufbringen der Nanofaserschicht 3, insbesondere der Nanofasern, auf das zumindest im Wesentlichen getrocknete bzw. ausgehärtete, insbesondere vernetzte, Trägermaterial erfolgen.

Erfindungsgemäß kann dabei das Fixieren bzw. Zumhaftenbringen der Nanofaserschicht, insbesondere der Nanofasern, auf dem Trägermaterial infolge einer insbesondere bei der Herstellung der Nanofasern vorliegenden Eigenklebrigkeit der Nanofasern erfolgen.

Zudem kann das Aufbringen der Nanofaserschicht, insbesondere der Nanofasern, auf dem Trägermaterial vor oder nach, insbesondere nach Aufbringen des Trägermaterials auf dem textilen Träger erfolgen.

Insbesondere kann das Aufbringen der Nanofaserschicht, insbesondere der Nanofasern, auf das Trägermaterial vor oder nach, insbesondere nach Ablösen des Trägermaterials von der entfernbaren Trägerschicht erfolgen.

Gemäß der erfindungsgemäßen Verfahrensführung kann es zudem vorgesehen sein, dass das Flächenfiltermaterial weiterhin mit (d) mindestens einer Abdeckschicht ausgerüstet bzw. versehen wird. Folglich kann eine zusätzliche Schicht (d) in Form einer Abdeckschicht vorgesehen werden. In diesem Zusammenhang kann die Abdeckschicht der Nanofaserschicht zugeordnet werden. Insbesondere kann die Abdeckschicht auf der dem Trägermaterial abgewandten (zweiten) Seite der Nanofaserschicht angeordnet werden.

Darüber hinaus wird erfindungsgemäß derart verfahren, dass das Flächenfiltermaterial weiterhin mit (e) mindestens einer Adsorptionsschicht ausgerüstet bzw. versehen wird. Demnach ist eine zusätzliche Schicht (d) in Form einer Adsorptionsschicht vorgesehen. Diesbezüglich wird die Adsorptionsschicht dem Trägermaterial zugeordnet. Die Adsorptionsschicht wird auf der der Nanofaserschicht abgewandten (zweiten) Seite des Trägermaterials angeordnet.

Gemäß dem erfindungsgemäßen Verfahren wird derart vorgegangen, dass die Adsorptionsschicht nicht der Nanofaserschicht zugeordnet wird bzw. dass die Adsorptionsschicht nicht zwischen dem Trägermaterial und der Nanofaserschicht angeordnet wird.

Insbesondere kann auch derart verfahren werden, dass die Adsorptionsschicht nicht an dem Trägermaterial bzw. nicht an dem textilen Träger fixiert wird. Insbesondere kann derart vorgegangen werden, dass die Adsorptionsschicht nicht an dem Trägermaterial bzw. nicht an dem textilen Träger zum Haften gebracht wird.

Insgesamt wird im Rahmen der vorliegenden Erfindung ein effizientes Verfahren zur Herstellung des Flächenfiltermaterials nach der Erfindung bereitgestellt.

Weiterhin betrifft die vorliegende Erfindung - gemäß einem **weiteren** Aspekt der vorliegenden Erfindung - die Verwendung des zuvor definierten Flächenfiltermaterials
zur Herstellung von Schutzausrüstung und/oder Schutzgegenständen aller Art, insbesondere von Schutzbekleidung, insbesondere für den zivilen oder militärischen Bereich, wie Schutzanzügen, Schutzhandschuhen, Schutzschuhwerk, Schutzsocken, Kopfschutzbekleidung, oder dergleichen, und/oder zur Herstellung von Schutzabdeckungen aller Art, vorzugsweise alle vorgenannten Schutzmaterialien und/oder Schutzbekleidungsstücke für den ABC-Einsatz und/oder mit Schutzfunktion gegenüber chemischen, biologischen und/oder radioaktiven Schad- und Giftstoffen; bzw.
zur Herstellung von Sport- und/oder Freizeitbekleidung und/oder Sport- und/oder Freizeitausrüstung, wie Sport- und/oder Freizeithosen und/oder -jacken, Sport- und/oder Freizeitunterwäsche, Sport- und/oder Freizeitsocken, Sport- und/oder Freizeithandschuhe, Sport- und/oder Freizeitkopfbedeckungen und Sport- und/oder Freizeitschuhmaterialien, insbesondere Sport- und/oder Freizeitschuhwerk, vorzugsweise alle vorgenannten Materialien und/oder Bekleidungsstücke für den Außeneinsatz (Outdoor-Einsatz) und/oder mit wasserabweisenden (d.h. Wasser in flüssiger Form abweisenden) und/oder windabweisenden und/oder atmungsaktiven (d.h. wasserdampfdurchlässigen und bevorzugt auch luftdurchlässigen) Eigenschaften.

Gemäß diesem Aspekt der vorliegenden Erfindung betrifft die vorliegende Erfindung auch die Verwendung des zuvor definierten Flächenfiltermaterials zur Herstellung von Filtern und Filtermaterialien aller Art, insbesondere zur Entfernung von Schad-, Geruchs- und Giftstoffen aller Art, vorzugsweise zur Entfernung von chemischen biologischen und/oder radioaktiven Schad- und Giftstoffen, insbesondere aus Luft- und/oder Gasströmen, wie ABC-Schutzmaskenfiltern, Geruchsfiltern, Flächenfiltern, Luftfiltern, insbesondere Filtern für die Raumluftreinigung, adsorptionsfähigen Trägerstrukturen und Filtern für den medizinischen Bereich.

Außerdem sind Gegenstand der vorliegenden Erfindung - gemäß einem **weiteren** Aspekt der vorliegenden Erfindung - Schutzausrüstungen und/oder Schutzgegenstände aller Art, insbesondere für den zivilen oder militärischen Bereich, insbesondere Schutzbekleidung, wie Schutzanzüge, Schutzhandschuhe, Schutzschuhwerk, Schutzsocken, Kopfschutzbekleidung und dergleichen, sowie Schutzabdeckungen, vorzugsweise alle vorgenannten Schutzausrüstungen und/oder Schutzgegenstände für den ABC-Einsatz und/oder mit Schutzfunktion gegenüber chemischen, biologischen und/oder radioaktiven Schad- und Giftstoffen, und/oder

Sport- und/oder Freizeitbekleidung und/oder Sport- und/oder Freizeitausrüstung aller Art, insbesondere für den Außeneinsatz (Outdoor-Einsatz) und/oder mit wasserabweisenden (d.h. Wasser in flüssiger Form abweisenden) und/oder windabweisenden und/oder atmungsaktiven (d.h. wasserdampfdurchlässigen und bevorzugt auch luftdurchlässigen) Eigenschaften, wie Sport- und/oder Freizeithosen und/oder -jacken, Sport- und/oder Freizeitunterwäsche, Sport- und/oder Freizeitsocken, Sport- und/oder Freizeithandschuhe, Sport- und/oder Freizeitkopfbedeckungen und Sport- und/oder Freizeitschuhmaterialien, insbesondere Sport- und/oder Freizeitschuhwerk,
jeweils hergestellt unter Verwendung des zuvor beschriebenen Flächenfiltermaterials 1 nach der Erfindung und/oder aufweisend das zuvor beschriebene Flächenfiltermaterial 1 nach der Erfindung.

Schließlich sind weiterer Gegenstand der vorliegenden Erfindung - gemäß einem **weiteren** Aspekt der vorliegenden Erfindung - auch Filter und Filtermaterialien aller Art, insbesondere zur Entfernung von Schad-, Geruchs- und Giftstoffen aller Art, vorzugsweise zur Entfernung von chemischen, biologischen und/oder radioaktiven Schad- und Giftstoffen, insbesondere aus Luft- und/oder Gasströmen, wie Schutzmaskenfilter, Geruchsfilter, Flächenfilter, Luftfilter, insbesondere Filter für die Raumluftreinigung, adsorptionsfähige Trägerstrukturen und Filter für den medizinischen Bereich, hergestellt unter Verwendung des zuvor beschriebenen Flächenfiltermaterials 1 nach der Erfindung und/oder aufweisend das zuvor beschriebene Flächenfiltermaterial 1 nach der Erfindung.

### AUSFÜHRUNGSBEISPIELE:

1. Es werden unterschiedliche Flächenfiltermaterial hergestellt, und zwar
   A) ein Flächenfiltermaterial A (nicht erfindungsgemäß), welches ein auf einem textilen Träger aufgebrachtes schaumbasiertes bzw. schaumförmiges Trägermaterial mit einer hieran fixierten Nanofaserschicht aufweist, wobei die Nanofaserschicht auf der dem textilen Träger abgewandten Seite des Trägermaterials angeordnet ist und wobei das schaumbasierte bzw. schaumförmige Trägermaterial und die Nanofaserschicht identische Schichtdicken aufweisen;
   B) ein Flächenfiltermaterial B (Vergleich), welches ein auf einem textilen Träger aufgebrachtes schaumbasiertes bzw. schaumförmiges Trägermaterial, jedoch keine Nanofaserschicht aufweist, wobei die Dicke des Trägermaterials der Gesamtdicke des in Material A vorliegenden Verbundes aus schaumbasiertem bzw. schaumförmigem Trägermaterial und Nanofaserschicht entspricht; Material B weist somit ein schaumbasiertes bzw. schaumförmiges Trägermaterial mit im Vergleich zu Material A doppelter Dicke und darüber hinaus keine Nanofaserschicht auf;
   C) ein Flächenfiltermaterial C (Vergleich), welches eine auf einem textilen Träger aufgebrachte Nanofaserschicht, jedoch kein schaumbasiertes bzw. schaumförmiges Trägermaterial aufweist, wobei die Dicke der Nanofaserschicht der Gesamtdicke des in Material A vorliegenden Verbundes aus schaumbasiertem bzw. schaumförmigem Trägermaterial und Nanofaserschicht entspricht; Material C weist somit eine Nanofaserschicht mit im Vergleich zu Material A doppelter Dicke und darüber hinaus kein schaumbasiertes bzw. schaumförmiges Trägermaterial auf.
   A', B', C') Zudem werden weitere Flächenfiltermaterialien A' (erfindungsgemäß) sowie B' (Vergleich) und C' (Vergleich) bereitgestellt, welche den jeweils korrespondierenden Flächenfiltermaterialien A, B und C mit der Maßgabe entsprechen, dass die Flächenfiltermaterialien A', B' und C' mit einem auf einem weiteren textilen Träger fixierten Adsorptionsmaterial in Form von partikulärer Aktivkohle ausgerüstet bzw. ergänzt sind, wobei der Verbund aus Aktivkohle und weiterem textilen Träger lose auf der dem schaumbasierten bzw. schaumförmigen Trägermaterial abgewandten Seite des ersten textilen Trägers derart angeordnet wird, dass die Aktivkohle zwischen dem ersten textilen Träger und dem weiteren textilen Träger positioniert ist.

   Zur Herstellung des vorgenannten Flächenfiltermaterials A wird eine textile Trägerschicht mit einem Flächengewicht von circa 75 g/m² als textiler Träger eingesetzt. Zur Bereitstellung des schaumbasierten bzw. schaumförmigen Trägermaterials wird der textile Träger vollflächig mit einer zuvor unter mechanischem Energieeintrag aufgeschäumten, wässrig basierten Lösung bzw. Dispersion eines Schaumstrukturmaterials (Schaumstrukturpolymers) auf Basis eines Polyurethans (PU) beschichtet, wobei die Klebstoffschicht in Form der aufgeschäumten Lösung bzw. Dispersion auf dem Träger aufgerakelt wird. Das aufgebrachte schaumförmige Material in Form der Lösung bzw. Dispersion weist eine Dichte von etwa 210 g/l bei einem Feststoffgehalt von etwa 55 %, bezogen auf die Lösung bzw. Dispersion, auf. Die Lösung bzw. Dispersion wird in einer definierten Menge zum nachfolgenden Erhalt einer vorgegebenen Dicke aufgetragen. Es erfolgt eine Trocknung bzw. Aushärtung der Lösung bzw. Dispersion des Schaumstrukturmaterials unter Energieeintrag zur Ausbildung eines getrockneten bzw. ausgehärteten gebrochenen bzw. offenporigen Trägermaterials bzw. Schaums Die Trocknung bzw. Aushärtung erfolgt dabei bei Temperaturen zwischen 100 °C und 140 °C. Die resultierende Schicht in Form des Trägermaterials weist eine definierte Dicke im erfindungsgemäß angegebenen Größenbereich auf. Auf das so erhaltene schaumbasierte bzw. schaumförmige Trägermaterial wird nachfolgend eine Nanofaserschicht auf Basis eines Polyurethans mittels Elektrospinnen aufgetragen, wobei die zugrundeliegenden Nanofasern aufgrund ihrer bei der Herstellung vorliegenden Eigenklebrigkeit auf dem Trägermaterial fixiert werden. Die Nanofasern weisen dabei Faserdurchmesser im Bereich von 100 nm bis 500 nm auf. Die Nanofaserschicht wird mit einer vorgegebenen Schichtdicke aufgetragen, welcher derjenigen des schaumbasierten bzw. schaumförmigen Trägermaterials entspricht. Es resultiert somit ein dreischichtiges Material in Form eines auf einem textilen Träger aufgebrachten schaumbasierten bzw. schaumförmigen Trägermaterials mit darauf aufgebrachter Nanofaserschicht.
   Die Herstellung von Flächenfiltermaterial B erfolgt wie für das Flächenfiltermaterial A beschrieben, jedoch mit der Maßgabe, dass das schaumbasierte bzw. schaumförmige Trägermaterial mit doppelter Dicke auf dem textilen Träger fixiert wird und dass darüber hinaus keine Nanofaserschicht aufgebracht wird. Es resultiert somit ein zweischichtiges Material in Form eines mit doppelter Dicke auf einem textilen Träger aufgebrachten schaumbasierten bzw. schaumförmigen Trägermaterials.
   Weiterhin erfolgt die Herstellung von Flächenfiltermaterial C wie für das Flächenfiltermaterial A beschrieben, jedoch mit der Maßgabe, dass die Nanofaserschicht unmittelbar und mit doppelter Dicke auf dem textilen Träger fixiert wird. Es resultiert somit ein zweischichtiges Material in Form einer mit doppelter Dicke auf einem textilen Träger aufgebrachten Nanofaserschicht.
   Für die Herstellung der weiteren Flächenfiltermaterialien A', B' und C' wird zudem derart vorgegangen, dass eine partikuläre Aktivkohle (Teilchendurchmesser etwa 0,3 mm) mit einer Auftragsmenge von etwa 70 g/m² unter Verwendung eines diskontinuierlich-punktförmig aufgetragenen Haftmittels (Klebstoff auf Basis von Polyurethan) auf einem weiteren textilen Träger (Flächengewicht circa 75 g/m²) fixiert wird, wobei eine nahezu vollständige Belegung einer Flachseite des weiteren textilen Trägers mit der Aktivkohle resultiert. Der so erhaltene weitere und mit der Aktivkohle ausgerüstete Träger wird mit den entsprechenden Materialien A, B bzw. C kombiniert, und zwar dahingehend, das der weitere und mi der Aktivkohle ausgerüstete textile Träger jeweils auf der dem schaumbasierten bzw. schaumförmigen Trägermaterial (Material A und B) bzw. auf der der Nanofaserschicht (Material C) abgewandten Seite des ersten textilen Träger angeordnet bzw. lose mit dem ersten Träger in Verbindung gebracht wird, wobei die Aktivkohle zwischen dem ersten Träger und dem zweiten Träger positioniert wird (wobei die Aktivkohle mit dem weiteren textilen Träger fest verbunden ist und lose auf dem ersten textilen Träger aufliegt bzw. hiermit lose in Kontakt steht).
2. Die vorgenannten Flächenfiltermaterialien A, B und C werden wie folgt untersucht:
   a) Zunächst wird an den Flächenfiltermaterialien A, B und C jeweils die Abscheidegradleistung nach DIN EN 1822 (April 1998) und der mittlere Abscheidegrad Aₘ nach DIN EN 779 (Juli 1993) bestimmt. Die Abscheidegradleistung nach DIN EN 1822 (April 1998) beträgt für das Flächenfiltermaterial A etwa 96 %, und der mittlere Abscheidegrad Aₘ nach DIN EN 779 (Juli 1993) beträgt etwa 99 %. Für das Flächenfiltermaterial B ergeben sich diesbezügliche Werte für die Abscheidegradleistung von etwa 80 % und für Aₘ von etwa 85 %, während für das Flächenfiltermaterial C Werte für die Abscheidegradleistung von etwa 92 % und für Aₘ von etwa 96 % ermittelt werden.
      Weiterhin wird der integrale Anfangsdurchlassgrad Dᵢ nach DIN EN 1822 (April 1998; DEHS-Aerosol, MPPS = 0,1 bis 0,3 µm) bestimmt. Der integrale Anfangsdurchlassgrad Dᵢ liegt dabei bei etwa 2 % für das Flächenfiltermaterial A, während sich für das Flächenfiltermaterial B ein Wert von etwa 8 % und für das Flächenfiltermaterial C ein Wert von etwa 4 % ergibt
      Das Flächenfiltermaterial A weist gegenüber Partikeln und Aerosolen mit Durchmessern ≥ 1 µm zudem eine Abscheiderate oberhalb von 98,5 % auf, während sich für das Flächenfiltermaterial B ein Wert von etwa 90 % und für das Flächenfiltermaterial C ein Wert von etwa 95 % ergibt.
      Die obigen Ausführungen zeigen, dass das Flächenfiltermaterial A insgesamt verbesserte Aerosol- bzw. Partikelfiltereigenschaften aufweist.
   b) Darüber hinaus wird an den Flächenfiltermaterialien A, B und C ein Waschtest (DIN EN ISO 5077) durchgeführt. Das Flächenfiltermaterial A weist im Vergleich zu den Flächenfiltermaterialien B und C bessere Werte auf. Flächenfiltermaterial B weist nur eine ausreichende Waschbeständigkeit auf, und für das Flächenfiltermaterial C können allenfalls zufriedenstellende Werte für die Waschbeständigkeit ermittelt werden.
3. Die vorgenannten Flächenfiltermaterialien A', B' und C' werden hinsichtlich ihrer Schutzfunktion gegenüber Schad- und Giftstoffen untersucht.
   Dabei wird die Barrierewirkung gegenüber Senfgas gemäß der Methode 2.2 der CRDEC-SP-84010 im Rahmen des sogenannten konvektiven Strömungstests (*convective flow test*) bestimmt. Zu diesem Zweck wird bei konstantem Strömungswiderstand mit einer Strömungsgeschwindigkeit von ca. 0,45 cm/s ein Senfgas enthaltender Luftstrom auf das jeweilige Flächenfiltermaterial einwirken gelassen und die flächenbezogene Durchbruchmenge nach 16 Stunden bestimmt (80 % relative Luftfeuchtigkeit, 32 °C). Die Durchbruchsmenge liegt für das erfindungsgemäße Flächenfiltermaterial A' bei 2,2 µg/cm², während für das Flächenfiltermaterial B' ein Wert von 3 µg/cm² und für das Flächenfiltermaterial C' ein Wert von 2,7 µg/cm² ermittelt werden kann.
4. Darüber hinaus werden in Anlehnung an die obigen Ausführungen weitere Flächenfiltermaterialien hergestellt, nämlich mit variabler Schichtdicke des schaumbasierten bzw. schaumförmigen Trägermaterials (0,3 µm bis 0,1 µm) und mit konstanter Schichtdicke der Nanofaserschicht (4 µm), und zwar
   A1) ein Flächenfiltermaterial A1 (nicht erfindungsgemäß), welches ein auf einem textilen Träger aufgebrachtes schaumbasiertes bzw. schaumförmiges Trägermaterial mit einer hieran fixierten Nanofaserschicht aufweist, wobei die Nanofaserschicht auf der dem textilen Träger abgewandten Seite des Trägermaterials angeordnet ist und wobei das schaumbasierte bzw. schaumförmige Trägermaterial eine Schichtdicke von 0,3 µm aufweist, während die Schichtdicke der Nanofaserschicht 4µm beträgt;
   A2) ein Flächenfiltermaterial A2 (nicht erfindungsgemäß), welches Flächenfiltermaterial A1 mit der Maßgabe entspricht, dass das schaumbasierte bzw. schaumförmige Trägermaterial eine Schichtdicke von 0,2 µm aufweist; sowie
   A3) ein Flächenfiltermaterial A2 (nicht erfindungsgemäß), welches Flächenfiltermaterial A1 mit der Maßgabe entspricht, dass das schaumbasierte bzw. schaumförmige Trägermaterial eine Schichtdicke von 0,1 µm aufweist.
      An den vorgenannten Flächenfiltermaterialien werden die in Abschnitt 2.) angeführten Untersuchungen durchgeführt (Bestimmung der Abscheidegradleistung nach DIN EN 1822 (April 1998), des mittleren Abscheidegrads Aₘ nach DIN EN 779 (Juli 1993) und des integralen Anfangsdurchlassgrades Dᵢ nach DIN EN 1822 (April 1998; DEHS-Aerosol, MPPS = 0,1 bis 0,3 µm); zudem wird ein Waschtest (DIN EN ISO 5077) durchgeführt). Die erfindungsgemäßen Flächenfiltermaterialen A1 bis A3 weisen dabei zu dem Flächenfiltermaterial A grundsätzlich vergleichbare Eigenschaften auf. Zudem zeigt sich, dass die in Rede stehenden Eigenschaften mit zunehmender Dicke verbessert werden (A1 > A2 > A3).

Die obigen Untersuchungen zeigen insgesamt die hervorragenden Eigenschaften der erfindungsgemäßen Flächenfiltermaterialien.

### Bezugszeichenliste:

- 1: Flächenfiltermaterial
- 2: schaumbasiertes und/oder schaumförmiges Trägermaterial
- 2a: (erste) Seite des schaumbasierten und/oder schaumförmigen Trägermaterials
- 2b: (zweite) Seite des schaumbasierten und/oder schaumförmigen Trägermaterials

- 3: Nanofaserschicht
- 3a: (erste) Seite der Nanofaserschicht
- 3b: (zweite) Seite der Nanofaserschicht
- 3': Nanofasern

- 4: textiler Träger
- 4a: (erste) Seite des textilen Trägers
- 4b: (zweite) Seite des textilen Trägers

- 5: Abdeckschicht
- 5a: (erste) Seite der Abdeckschicht
- 5b: (zweite) Seite der Abdeckschicht

- 6: Adsorptionsschicht
- 6a: (erste) Seite der Adsorptionsschicht
- 6b: (zweite) Seite der Adsorptionsschicht

## Patentansprüche

1. Flächenfiltermaterial (1) mit Aerosol- und/oder Partikelfilterfunktion und mit Schutzfunktion gegenüber chemischen, biologischen und/oder radioaktiven Schad- und Giftstoffen,
wobei das Flächenfiltermaterial (1) umfasst:
(a) ein luft- und/oder wasserdampfdurchlässiges, flächiges schaumbasiertes und/oder schaumförmiges Trägermaterial (2) mit zwei gegenüberliegenden Flachseiten (2a, 2b), wobei das Trägermaterial (2) eine luftdurchlässige und/oder diskontinuierlich ausgebildete Schicht auf Basis einer getrockneten oder ausgehärteten, vorzugsweise vernetzten, gebrochenen Polymerschaumstruktur ist, wobei die gebrochene Polymerschaumstruktur offenporig und/oder nicht geschlossen ausgebildet ist;
(b) eine dem schaumbasierten und/oder schaumförmigen Trägermaterial (2) zugeordnete und an einer (ersten) Flachseite (2a) des schaumbasierten und/oder schaumförmigen Trägermaterials (2) fixierte und/oder zum Haften gebrachte luft- und/oder wasserdampfdurchlässige, vorzugsweise flächige Nanofaserschicht (3), wobei die Nanofaserschicht (3) eine Vielzahl von Nanofasern (3') umfasst oder hieraus besteht;
wobei das Flächenfiltermaterial (1) weiterhin mindestens eine Adsorptionsschicht (6) aufweist, wobei die Adsorptionsschicht (6) eine Vielzahl einzelner und/oder diskreter Adsorberpartikel (6') umfasst oder hieraus gebildet ist, wobei die Adsorptionsschicht (6) zwei gegenüberliegende Flachseiten (6a, 6b) aufweist, wobei die Adsorptionsschicht (6) dem Trägermaterial (2) zugeordnet ist und wobei die Adsorptionsschicht (6) auf der der Nanofaserschicht (3) abgewandten (zweiten) Flachseite (2b) des Trägermaterials (2) angeordnet ist.

2. Flächenfiltermaterial nach Anspruch 1,
wobei das Trägermaterial (2) ein Flächengewicht im Bereich von 5 g/m² bis 500 g/m², insbesondere im Bereich von 10 g/m² bis 300 g/m², vorzugsweise im Bereich von 15 g/m² bis 200 g/m², bevorzugt im Bereich von 20 g/m² bis 150 g/m², besonders bevorzugt im Bereich von 25 g/m² bis 100 g/m², ganz besonders bevorzugt im Bereich von 25 g/m² bis 50 g/m², aufweist; und/oder
wobei das Trägermaterial (2) eine Dicke von höchstens 10 mm, insbesondere höchstens 5 mm, vorzugsweise höchstens 4 mm, bevorzugt höchstens 3 mm, besonders bevorzugt höchstens 1 mm, ganz besonders bevorzugt höchstens 0,5 mm, aufweist und/oder wobei das Trägermaterial (2) eine Dicke im Bereich von 0,01 mm bis 10 mm, insbesondere im Bereich von 0,03 mm bis 5 mm, vorzugsweise im Bereich von 0,05 mm bis 4 mm, bevorzugt im Bereich von 0,08 mm bis 3 mm, besonders bevorzugt im Bereich von 0,09 mm bis 1 mm, ganz besonders bevorzugt im Bereich von 0,1 mm bis 0,5 mm, weiter bevorzugt im Bereich von 0,1 mm bis 0,3 mm, aufweist; und/oder wobei das Trägermaterial (2) eine Dichte im Bereich von 25 g/l bis 250 g/l, insbesondere im Bereich von 50 g/l bis 200 g/l, vorzugsweise im Bereich von 75 g/l bis 175 g/l, bevorzugt im Bereich von 100 g/l bis 170 g/l, aufweist.

3. Flächenfiltermaterial nach Anspruch 1 oder 2,
wobei die gebrochene Polymerschaumstruktur eine Vielzahl getrockneter oder ausgehärteter, insbesondere vernetzter, zerstörter und/oder geplatzter und/oder kollabierter Schaumblasen aufweist; und/oder
wobei die gebrochene Polymerschaumstruktur, bevorzugt die getrockneten oder ausgehärteten, insbesondere vernetzten, zerstörten und/oder geplatzten und/oder kollabierten Schaumblasen der gebrochenen Polymerschaumstruktur eine Vielzahl an zerstörten und/oder gebrochenen und/oder kollabierten Wandungen und/oder Stegen insbesondere aus Schaumstrukturmaterial (Schaumstrukturpolymer) aufweist bzw. aufweisen; und/oder
wobei die gebrochene Polymerschaumstruktur einen Anteil an zerstörten und/oder geplatzten und/oder kollabierten Schaumblasen von mindestens 10 %, insbesondere mindestens 30 %, vorzugsweise mindestens 50 %, bevorzugt mindestens 70 %, besonders bevorzugt mindestens 90 %, ganz besonders bevorzugt mindestens 95 %, bezogen auf die Gesamtanzahl an Schaumblasen in der gebrochenen Polymerschaumstruktur, aufweist.

4. Flächenfiltermaterial nach einem der vorangehenden Ansprüche,
wobei die gebrochene Polymerschaumstruktur eine Vielzahl von insbesondere sich in der gebrochenen Polymerschaumstruktur erstreckenden Durchbrechungen, Poren, Kanälen und/oder Öffnungen und/oder eine Vielzahl von insbesondere die jeweiligen Flachseiten (2a, 2b) des Trägermaterials (2) verbindenden Durchbrechungen, Poren, Kanälen und/oder Öffnungen aufweist; und/oder
wobei die gebrochene Polymerschaumstruktur zusammenhängend und/oder kohärent ausgebildet ist.

5. Flächenfiltermaterial nach einem der vorangehenden Ansprüche,
wobei die Nanofaserschicht (3) zwei gegenüberliegende Flachseiten (3a, 3b) aufweist; und/oder
wobei die Nanofaserschicht (3) zumindest im Wesentlichen vollflächig und/oder ganzseitig eine (erste) Flachseite (3a) der Nanofaserschicht (3) an der (ersten) Flachseite (2a) des Trägermaterials (2) fixiert und/oder zum Haften gebracht ist; und/oder
wobei die Nanofaserschicht (3) unmittelbar und/oder direkt und/oder ohne dazwischenliegende Schicht(en), insbesondere ohne eine dazwischenliegende Adsorptionsschicht, an der (ersten) Flachseite (2a) des Trägermaterials (2) fixiert und/oder zum Haften gebracht ist, insbesondere über eine (erste) Flachseite (3a) der Nanofaserschicht (3); und/oder
wobei die Nanofaserschicht (3) ohne Verwendung und/oder Zugabe eines Haft- und/oder Klebemittels an der (ersten) Flachseite (2a) des Trägermaterials (2) fixiert und/oder zum Haften gebracht ist.

6. Flächenfiltermaterial nach einem der vorangehenden Ansprüche,
wobei die Nanofaserschicht (3) als ein die Nanofasern (3') aufweisendes und/oder hieraus bestehendes textiles Flächengebilde, insbesondere dreidimensionales textiles Flächengebilde, ausgebildet ist; und/oder
wobei die Nanofasern (3') in der Nanofaserschicht (3) zufällig und/oder wirr angeordnet sind; und/oder
wobei die Nanofasern (3') in der Nanofaserschicht (3) miteinander verbunden sind, insbesondere dauerhaft miteinander verbunden und/oder verklebt sind, insbesondere mittels und/oder infolge einer insbesondere bei der Herstellung des Flächenfiltermaterials (1) und/oder der Nanofaserschicht (3) vorliegenden Eigenklebrigkeit der Nanofasern (3'); und/oder
wobei die Nanofasern (3') in der Nanofaserschicht (3) ein zusammenhängendes und/oder kohärentes Flächengebilde, insbesondere Flächenverbundmaterial, ausbilden; und/oder
wobei die Nanofaserschicht (3) als zusammenhängendes und/oder kohärentes Flächengebilde, insbesondere Flächenverbundmaterial, ausgebildet ist; und/oder
wobei die Nanofaserschicht (3) als ein Gelege oder Textilverbundstoff, insbesondere Vlies (Non-Woven), vorzugsweise als ein Vlies (Non-Woven), bevorzugt als ein Wirrvlies, ausgebildet ist; und/oder
wobei die Nanofaserschicht (3) eine Dicke von höchstens 100 µm, insbesondere höchstens 50 µm, vorzugsweise höchstens 20 µm, bevorzugt höchstens 10 µm, besonders bevorzugt höchstens 5 µm, aufweist und/oder wobei die Nanofaserschicht (3) eine Dicke im Bereich von 0,001 µm bis 100 µm, insbesondere im Bereich von 0,005 µm bis 50 µm, vorzugsweise im Bereich von 0,008 µm bis 20 µm, bevorzugt im Bereich von 0,01 µm bis 10 µm, besonders bevorzugt im Bereich von 0,15 µm bis 8 µm, ganz besonders bevorzugt im Bereich von 1 µm bis 7 µm, weiter bevorzugt im Bereich von 2 µm bis 6 µm, noch weiter bevorzugt im Bereich von 3 µm bis 5 µm, wiederum weiter bevorzugt im Bereich von 4 µm bis 5 µm, aufweist.

7. Flächenfiltermaterial nach einem der vorangehenden Ansprüche,
wobei die Nanofasern (3') einen mittleren Faserdurchmesser D₅₀ im Bereich von 2 nm bis 4.500 nm, insbesondere im Bereich von 10 nm bis 2.000 nm, vorzugsweise im Bereich von 15 nm bis 1.750 nm, bevorzugt im Bereich von 20 nm bis 1.250 nm, besonders bevorzugt im Bereich von 25 nm bis 750 nm, ganz besonders bevorzugt im Bereich von 30 nm bis 500 nm, weiter bevorzugt im Bereich von 60 nm bis 400 nm, aufweisen; und/oder
wobei die Nanofaserschicht (3) durch die Nanofasern (3') begrenzte Öffnungen oder Poren aufweist, insbesondere wobei die Nanofaserschicht (3) eine mittlere Öffnungsgröße oder mittlere Porengröße von höchstens 150 µm, insbesondere höchstens 100 µm, vorzugsweise höchstens 50 µm, besonders bevorzugt höchstens 30 µm, ganz besonders bevorzugt höchstens 20 µm, noch mehr bevorzugt höchstens 10 µm, aufweist und/oder insbesondere wobei das Verhältnis der mittleren Öffnungsgröße oder der mittleren Porengröße einerseits zum mittleren Faserdurchmesser D₅₀ der Nanofasern 3' andererseits im Bereich von 0,2 bis 1.800, insbesondere 1 bis 400, vorzugsweise 5 bis 300, besonders bevorzugt 10 bis 250, ganz besonders bevorzugt 25 bis 225, liegt.

8. Flächenfiltermaterial nach einem der vorangehenden Ansprüche,
wobei das Flächenfiltermaterial (1) weiterhin (c) mindestens einen textilen Träger (4) aufweist,
insbesondere wobei der textile Träger (4) zwei gegenüberliegende Flachseiten (4a, 4b) aufweist; und/oder
insbesondere wobei der textile Träger (4) dem Trägermaterial (2) zugeordnet ist und/oder insbesondere wobei der textile Träger (4) auf der der Nanofaserschicht (3) abgewandten (zweiten) Flachseite (2b) des Trägermaterials (2) angeordnet ist; und/oder
insbesondere wobei der textile Träger (4) an dem Trägermaterial (2) fixiert und/oder zum Haften gebracht ist; und/oder
insbesondere wobei der textile Träger (4) an einer (zweiten) Flachseite (2b) des Trägermaterials (2) fixiert und/oder zum Haften gebracht ist, insbesondere über eine (erste) Flachseite (4a) des textilen Trägers (4); und/oder
wobei der textile Träger (4) als ein textiles Flächengebilde, vorzugsweise ein luftdurchlässiges und/oder wasserdampfdurchlässiges Textilmaterial, bevorzugt ein Gewebe, Gewirke, Gestricke, Gelege, Vlies oder Textilverbundstoff, ausgebildet ist.

9. Flächenfiltermaterial nach einem der vorangehenden Ansprüche,
wobei das Flächenfiltermaterial (1) weiterhin (d) mindestens eine Abdeckschicht (5) aufweist,
insbesondere wobei die Abdeckschicht (5) zwei gegenüberliegende Flachseiten (5a, 5b) aufweist; und/oder
insbesondere wobei die Abdeckschicht (5) der Nanofaserschicht (3) zugeordnet ist und/oder insbesondere wobei die Abdeckschicht (5) auf der dem Trägermaterial (2) abgewandten (zweiten) Flachseite (3b) der Nanofaserschicht (3) angeordnet ist; und/oder
insbesondere wobei die Abdeckschicht (5) an der Nanofaserschicht (3) fixiert und/oder zum Haften gebracht ist; und/oder
insbesondere wobei die Abdeckschicht (5) an einer (zweiten) Flachseite (3b) der Nanofaserschicht (3) fixiert und/oder zum Haften gebracht ist, insbesondere über eine (erste) Flachseite (5a) der Abdeckschicht (5); und/oder
wobei die Abdeckschicht (5) als ein textiles Flächengebilde, vorzugsweise ein luftdurchlässiges und/oder wasserdampfdurchlässiges Textilmaterial, bevorzugt ein Gewebe, Gewirke, Gestricke, Gelege, Vlies oder Textilverbundstoff, ausgebildet ist.

10. Flächenfiltermaterial nach einem der vorangehenden Ansprüche,
wobei die Adsorptionsschicht (6), insbesondere die Adsorberpartikel (6') der Adsorptionsschicht (6), an dem Trägermaterial (2) fixiert und/oder zum Haften gebracht ist; und/oder
wobei die Adsorptionsschicht (6), insbesondere die Adsorberpartikel (6') der Adsorptionsschicht (6), an einer (zweiten) Flachseite (2b) des Trägermaterials (2) fixiert und/oder zum Haften gebracht ist, insbesondere über eine (erste) Flachseite (6a) der Adsorptionsschicht (6); und/oder
wobei die Adsorptionsschicht (6), insbesondere die Adsorberpartikel (6') der Adsorptionsschicht (6), mittels Verwendung eines insbesondere diskontinuierlich-punktförmig aufgetragenen Haftmittels an dem Trägermaterial (2), insbesondere an der (zweiten) Flachseite (2b) des Trägermaterials (2) fixiert und/oder zum Haften gebracht ist; und/oder
wobei die Adsorptionsschicht (6) nicht der Nanofaserschicht (3) zugeordnet ist und/oder wobei die Adsorptionsschicht (6) nicht zwischen dem Trägermaterial (2) und der Nanofaserschicht (3) angeordnet ist; und/oder
wobei die Adsorptionsschicht (6), insbesondere die Adsorberpartikel (6') der Adsorptionsschicht (6), nicht an dem Trägermaterial (2) und/oder nicht an dem textilen Träger (4) fixiert ist bzw. sind und/oder wobei die Adsorptionsschicht (6), insbesondere die Adsorberpartikel (6') der Adsorptionsschicht (6), nicht an dem Trägermaterial (2) und/oder nicht an dem textilen Träger (4) zum Haften gebracht ist bzw. sind; und/oder wobei die Adsorptionsschicht (6) als separate und/oder nicht mit dem Trägermaterial (2) und/oder nicht mit Nanofaserschicht (3) und/oder nicht mit dem textilen Träger (4) verbundene, insbesondere nicht an den vorgenannten Schichten (2, 3, 4) fixierte und/oder nicht hieran zum Haften gebrachte Schicht ausgebildet ist.

11. Flächenfiltermaterial nach einem der vorangehenden Ansprüche,
wobei das Flächenfiltermaterial (1) selbsttragend ist und/oder wobei das Flächenfiltermaterial (1) als Verbundmaterial ausgebildet ist; und/oder
wobei das Flächenfiltermaterial (1) ein Gesamtflächengewicht im Bereich von 10 g/m² bis 800 g/m², insbesondere im Bereich von 20 g/m² bis 600 g/m², vorzugsweise im Bereich von 50 g/m² bis 400 g/m², bevorzugt im Bereich von 75 g/m² bis 300 g/m², besonders bevorzugt im Bereich von 100 g/m² bis 200 g/m², aufweist; und/oder
wobei das Flächenfiltermaterial (1) eine Luftdurchlässigkeit von mindestens 10 l·m^{- 2}·s⁻¹, insbesondere mindestens 25 l·m⁻²·s⁻¹, vorzugsweise mindestens 30 l·m⁻²·s⁻¹, bevorzugt mindestens 40l·m⁻²·s⁻¹, besonders bevorzugt mindestens 50 l·m⁻²·s⁻¹, ganz besonders bevorzugt mindestens 100 l·m⁻²·s⁻¹, bei einem Strömungswiderstand von 127 Pa, aufweist; und/oder
wobei das Flächenfiltermaterial (1) bei 25 °C eine Wasserdampfdurchlässigkeit von mindestens 10 l/m² pro 24 h, insbesondere mindestens 20 l/m² pro 24 h, vorzugsweise mindestens 30 l/m² pro 24 h, aufweist; und/oder
wobei das Flächenfiltermaterial (1) einen Wasserdampfdurchgangswiderstand Rₑₜ unter stationären Bedingungen, gemessen nach DIN EN 31 092:1993 (Februar 1994) und internationaler Norm ISO 11 092, bei 35 °C, von höchstens 25 (m²·Pascal)/Watt, insbesondere höchstens 20(m²·Pascal)/Watt, vorzugsweise höchstens 10 (m²·Pascal)/Watt, aufweist; und/oder
wobei das Flächenfiltermaterial (1) eine Gesamtdicke im Bereich von 0,1 mm bis 30 mm, insbesondere im Bereich von 0,2 mm bis 20 mm, vorzugsweise im Bereich von 0,5 mm bis 10 mm, bevorzugt im Bereich von 0,6 mm bis 6 mm, besonders bevorzugt im Bereich von 0,8 mm bis 2 mm, aufweist.

12. Verfahren zur Herstellung eines Flächenfiltermaterials (1) mit Aerosol- und/oder Partikelfilterfunktion und mit Schutzfunktion gegenüber chemischen, biologischen und/oder radioaktiven Schad- und Giftstoffen, insbesondere wie in einem der vorangehenden Ansprüche definiert, wobei das Verfahren die nachfolgenden Schritte umfasst:
(a) Bereitstellung und/oder Herstellung eines luft- und/oder wasserdampfdurchlässigen, flächigen schaumbasierten und/oder schaumförmigen Trägermaterials (2) mit zwei gegenüberliegenden Flachseiten (2a, 2b), wobei das Trägermaterial (2) eine luftdurchlässige und/oder diskontinuierlich ausgebildete Schicht auf Basis einer getrockneten oder ausgehärteten, vorzugsweise vernetzten, gebrochenen Polymerschaumstruktur ist, wobei die gebrochene Polymerschaumstruktur offenporig und/oder nicht geschlossen ausgebildet wird;
(b) Aufbringen einer luft- und/oder wasserdampfdurchlässigen, vorzugsweise flächigen Nanofaserschicht (3), wobei die Nanofaserschicht (3) eine Vielzahl von Nanofasern (3') umfasst oder hieraus besteht, an einer (ersten) Flachseite (2a) des schaumbasierten und/oder schaumförmigen Trägermaterials (2), derart, dass die Nanofaserschicht (3) an der (ersten) Flachseite (2a) des Trägermaterials (2) fixiert und/oder zum Haften gebracht wird;
wobei das Flächenfiltermaterial (1) weiterhin mit mindestens einer Adsorptionsschicht (6) ausgerüstet und/oder versehen wird, wobei die Adsorptionsschicht (6) eine Vielzahl einzelner und/oder diskreter Adsorberpartikel (6') umfasst oder hieraus gebildet wird, wobei die Adsorptionsschicht (6) zwei gegenüberliegende Flachseiten (6a, 6b) aufweist, wobei die Adsorptionsschicht (6) dem Trägermaterial (2) zugeordnet wird und wobei die Adsorptionsschicht (6) auf der der Nanofaserschicht (3) abgewandten (zweiten) Seite (2b) des Trägermaterials (2) angeordnet wird.

13. Verwendung eines Flächenfiltermaterials, wie in einem der Ansprüche 1 bis 11 definiert,
zur Herstellung von Schutzausrüstung und/oder Schutzgegenständen aller Art, insbesondere von Schutzbekleidung, insbesondere für den zivilen oder militärischen Bereich, wie Schutzanzügen, Schutzhandschuhen, Schutzschuhwerk, Schutzsocken, Kopfschutzbekleidung, oder dergleichen, und/oder zur Herstellung von Schutzabdeckungen aller Art, vorzugsweise alle vorgenannten Schutzmaterialien und/oder Schutzbekleidungsstücke für den ABC-Einsatz und/oder mit Schutzfunktion gegenüber chemischen, biologischen und/oder radioaktiven Schad- und Giftstoffen; und/oder
zur Herstellung von Sport- und/oder Freizeitbekleidung und/oder Sport- und/oder Freizeitausrüstung, wie Sport- und/oder Freizeithosen und/oder -jacken, Sport- und/oder Freizeitunterwäsche, Sport- und/oder Freizeitsocken, Sport- und/oder Freizeithandschuhe, Sport- und/oder Freizeitkopfbedeckungen und Sport- und/oder Freizeitschuhmaterialien, insbesondere Sport- und/oder Freizeitschuhwerk, vorzugsweise alle vorgenannten Materialien und/oder Bekleidungsstücke für den Außeneinsatz (Outdoor-Einsatz) und/oder mit wasserabweisenden (d.h. Wasser in flüssiger Form abweisenden) und/oder windabweisenden und/oder atmungsaktiven (d.h. wasserdampfdurchlässigen und bevorzugt auch luftdurchlässigen) Eigenschaften; und/oder
zur Herstellung von Filtern und Filtermaterialien aller Art, insbesondere zur Entfernung von Schad-, Geruchs- und Giftstoffen aller Art, vorzugsweise zur Entfernung von chemischen biologischen und/oder radioaktiven Schad- und Giftstoffen, insbesondere aus Luft- und/oder Gasströmen, wie ABC-Schutzmaskenfiltern, Geruchsfiltern, Flächenfiltern, Luftfiltern, insbesondere Filtern für die Raumluftreinigung, adsorptionsfähigen Trägerstrukturen und Filtern für den medizinischen Bereich.

14. Schutzausrüstungen und/oder Schutzgegenstände aller Art, insbesondere für den zivilen oder militärischen Bereich, insbesondere Schutzbekleidung, wie Schutzanzüge, Schutzhandschuhe, Schutzschuhwerk, Schutzsocken, Kopfschutzbekleidung und dergleichen, sowie Schutzabdeckungen, vorzugsweise alle vorgenannten Schutzausrüstungen und/oder Schutzgegenstände für den ABC-Einsatz und/oder mit Schutzfunktion gegenüber chemischen, biologischen und/oder radioaktiven Schad- und Giftstoffen, und/oder
Sport- und/oder Freizeitbekleidung und/oder Sport- und/oder Freizeitausrüstung aller Art, insbesondere für den Außeneinsatz (Outdoor-Einsatz) und/oder mit wasserabweisenden (d.h. Wasser in flüssiger Form abweisenden) und/oder windabweisenden und/oder atmungsaktiven (d.h. wasserdampfdurchlässigen und bevorzugt auch luftdurchlässigen) Eigenschaften, wie Sport- und/oder Freizeithosen und/oder -jacken, Sport- und/oder Freizeitunterwäsche, Sport- und/oder Freizeitsocken, Sport- und/oder Freizeithandschuhe, Sport- und/oder Freizeitkopfbedeckungen und Sport- und/oder Freizeitschuhmaterialien, insbesondere Sport- und/oder Freizeitschuhwerk,
jeweils hergestellt unter Verwendung eines Flächenfiltermaterials (1), wie in einem der Ansprüche 1 bis 11 definiert, und/oder aufweisend ein Flächenfiltermaterial (1), wie in einem der Ansprüche 1 bis 11 definiert.

15. Filter und Filtermaterialien aller Art, insbesondere zur Entfernung von Schad-, Geruchs- und Giftstoffen aller Art, vorzugsweise zur Entfernung von chemischen, biologischen und/oder radioaktiven Schad- und Giftstoffen, insbesondere aus Luft- und/oder Gasströmen, wie Schutzmaskenfilter, Geruchsfilter, Flächenfilter, Luftfilter, insbesondere Filter für die Raumluftreinigung, adsorptionsfähige Trägerstrukturen und Filter für den medizinischen Bereich, hergestellt unter Verwendung eines Flächenfiltermaterials (1), wie in einem der Ansprüche 1 bis 11 definiert, und/oder aufweisend ein Flächenfiltermaterial (1), wie in einem der Ansprüche 1 bis 11 definiert.

## Claims

1. A sheet filter material (1) having aerosol and/or particle filter functions and having a protective function against chemical, biological and/or radioactive pollutants and toxins, wherein the sheet filter material (1) comprises:
(a) an air-permeable and/or water-vapour-permeable, flat, foam-based and/or foam-formed carrier material (2) with two opposite flat sides (2a, 2b), wherein the carrier material (2) is an air-permeable and/or discontinuously formed layer based on a dried or cured, preferably cross-linked, broken polymer foam structure, wherein the broken polymer foam structure is formed to be open-pored and/or not closed;
(b) an air-permeable and/or water-vapour-permeable, preferably flat nanofibre layer (3) associated with the foam-based and/or foam-formed carrier material (2) and fixed and/or adhered to a (first) flat side (2a) of the foam-based and/or foam-formed carrier material (2), wherein the nanofibre layer (3) comprises or consists of a plurality of nanofibres (3');
wherein the sheet filter material (1) further has at least one adsorption layer (6), wherein the adsorption layer (6) comprises or is formed from a plurality of individual and/or discrete adsorber particles (6'), wherein the adsorption layer (6) has two opposite flat sides (6a, 6b), wherein the adsorption layer (6) is associated with the carrier material (2) and wherein the adsorption layer (6) is arranged on the (second) flat side (2b) of the carrier material (2) facing away from the nanofibre layer (3).

2. The sheet filter material according to Claim 1,
wherein the carrier material (2) has a weight per unit area in the range from 5 g/m² to 500 g/m², in particular in the range from 10 g/m² to 300 g/m², preferably in the range from 15 g/m² to 200 g/m², preferably in the range from 20 g/m² to 150 g/m², particularly preferably in the range from 25 g/m² to 100 g/m², more particularly preferably in the range from 25 g/m² to 50 g/m²; and/or
wherein the carrier material (2) has a thickness of at most 10 mm, in particular at most 5 mm, preferably at most 4 mm, preferably at most 3 mm, particularly preferably at most 1 mm, more particularly preferably at most 0.5 mm, and/or wherein the carrier material (2) has a thickness in the range from 0.01 mm to 10 mm, in particular in the range from 0.03 mm to 5 mm, preferably in the range from 0.05 mm to 4 mm, preferably in the range from 0.08 mm to 3 mm, particularly preferably in the range from 0.09 mm to 1 mm, more particularly preferably in the range from 0.1 mm to 0.5 mm, further preferably in the range from 0.1 mm to 0.3 mm; and/or
wherein the carrier material (2) has a density in the range from 25 to 250 g/l, in particular in the range from 50 to 200 g/l, preferably in the range from 75 to 175 g/l, preferably in the range from 100 to 170 g/l.

3. The sheet filter material according to Claim 1 or 2,
wherein the broken polymer foam structure has a plurality of dried or cured, preferably cross-linked foam bubbles that are crushed and/or burst and/or collapsed; and/or
wherein the broken polymer foam structure, preferably the dried or cured, preferably cross-linked foam bubbles of the broken polymer foam structure that are crushed and/or burst and/or collapsed, has/have a plurality of crushed and/or broken and/or collapsed walls and/or webs, in particular made of foam structure material (foam structure polymer); and/or
wherein the broken polymer foam structure has a proportion of crushed and/or burst and/or collapsed foam bubbles of at least 10%, in particular at least 30%, preferably at least 50%, preferably at least 70%, particularly preferably at least 90%, more particularly preferably at least 95%, based on the total number of foam bubbles in the broken polymer foam structure.

4. The sheet filter material according to any one of the preceding claims,
wherein the broken polymer foam structure has a plurality of perforations, pores, ducts and/or openings in particular extending within the broken polymer foam structure and/or a plurality of openings, pores, ducts and/or openings in particular connecting the respective flat sides (2a, 2b) of the carrier material (2); and/or
wherein the broken polymer foam structure is formed to be contiguous and/or coherent.

5. The sheet filter material according to any one of the preceding claims,
wherein the nanofibre layer (3) has two opposite flat sides (3a, 3b); and/or
wherein the nanofibre layer (3) is at least essentially a (first) flat side (3a) of the nanofibre layer (3) fixed and/or adhered to the (first) flat side (2a) of the carrier material (2) over its entire surface and/or the entire side; and/or
wherein the nanofibre layer (3) is immediately and/or directly and/or without interposing layer(s), in particular without an interposing adsorption layer, fixed and/or adhered to the (first) flat side (2a) of the carrier material (2), in particular via a (first) flat side (3a) of the nanofibre layer (3); and/or
wherein the nanofibre layer (3) is fixed and/or adhered to the (first) flat side (2a) of the carrier material (2) without the use and/or addition of an adhering and/or adhesive means.

6. The sheet filter material according to any one of the preceding claims,
wherein the nanofibre layer (3) is formed as a textile sheet fabric, in particular a three-dimensional textile sheet fabric, having and/or consisting of the nanofibres (3');
and/or
wherein the nanofibres (3') are arranged in the nanofibre layer (3) randomly and/or chaotically; and/or
wherein the nanofibres (3') are connected to one another, in particular permanently connected and/or glued to one another, in the nanofibre layer (3), in particular by means of and/or as a result of self-adhesiveness on the part of the nanofibres (3') present, in particular, during the production of the sheet filter material (1) and/or the nanofibre layer (3); and/or
wherein the nanofibres (3') in the nanofibre layer (3) form a contiguous and/or coherent sheet fabric, in particular a sheet composite material; and/or
wherein the nanofibre layer (3) is formed as a contiguous and/or coherent flat structure, in particular a flat composite material; and/or
wherein the nanofibre layer (3) is formed as a scrim or textile composite material, in particular a non-woven fabric, preferably a non-woven fabric, preferably a random non-woven fabric; and/or
wherein the nanofibre layer (3) has a thickness of at most 100 µm, in particular at most 50 µm, preferably at most 20 µm, preferably at most 10 µm, particularly preferably at most 5 µm, and/or wherein the nanofibre layer (3) has a thickness in the range from 0.001 µm to 100 µm, in particular in the range from 0.005 µm to 50 µm, preferably in the range from 0.008 µm to 20 µm, preferably in the range from 0.01 µm to 10 µm, particularly preferably in the range from 0.15 µm to 8 µm, more particularly preferably in the range from 1 µm to 7 µm, further preferably in the range from 2 µm to 6 µm, even further preferably in the range from 3 µm to 5 µm, yet further preferably in the range from 4 µm to 5 µm.

7. The sheet filter material according to any one of the preceding claims,
wherein the nanofibres (3') have an average fibre diameter D₅₀ in the range from 2 nm to 4,500 nm, in particular in the range from 10 nm to 2,000 nm, preferably in the range from 15 nm to 1,750 nm, preferably in the range from 20 nm to 1,250 nm, particularly preferably in the range from 25 nm to 750 nm, more particularly preferably in the range from 30 nm to 500 nm, further preferably in the range from 60 nm to 400 nm; and/or
wherein the nanofibre layer (3) has openings or pores delimited by the nanofibres (3'), in particular wherein the nanofibre layer (3) has an average opening size or average pore size of at most 150 µm, in particular at most 100 µm, preferably at most 50 µm, particularly preferably at most 30 µm, especially preferably at most 20 µm, even more preferably at most 10 µm, and/or in particular wherein the ratio of the mean opening size or the mean pore size on the one hand to the mean fibre diameter D₅₀ of the nanofibres 3' on the other hand is in the range from 0.2 to 1,800, in particular 1 to 400, preferably 5 to 300, particularly preferably 10 to 250, more particularly preferably 25 to 225.

8. The sheet filter material according to any one of the preceding claims,
wherein the sheet filter material (1) further (c) has at least one textile carrier (4),
in particular wherein the textile carrier (4) has two opposite flat sides (4a, 4b); and/or in particular wherein the textile carrier (4) is associated with the carrier material (2) and/or in particular wherein the textile carrier (4) is arranged on the (second) flat side (2b) of the carrier material (2) facing away from the nanofibre layer (3); and/or
in particular wherein the textile carrier (4) is fixed and/or adhered to the carrier material (2); and/or
in particular wherein the textile carrier (4) is fixed and/or adhered to a (second) flat side (2b) of the carrier material (2), in particular via a (first) flat side (4a) of the textile carrier (4); and/or
wherein the textile carrier (4) is formed as a textile sheet fabric, preferably an air-permeable and/or water-vapour-permeable textile material, preferably a woven fabric, a knitted fabric, a crocheted fabric, a scrim, a non-woven fabric or a textile composite material.

9. The sheet filter material according to any one of the preceding claims,
wherein the sheet filter material (1) further (d) has at least one cover layer (5),
in particular wherein the cover layer (5) has two opposite flat sides (5a, 5b); and/or in particular wherein the cover layer (5) is associated with the nanofibre layer (3) and/or in particular wherein the cover layer (5) is arranged on the (second) flat side (3b) of the nanofibre layer (3) facing away from the carrier material (2); and/or
in particular wherein the cover layer (5) is fixed and/or adhered to the nanofibre layer (3); and/or
in particular wherein the cover layer (5) is fixed and/or adhered to a (second) flat side (3b) of the nanofibre layer (3), in particular via a (first) flat side (5a) of the cover layer (5); and/or wherein the cover layer (5) is formed as a textile sheet fabric, preferably an air-permeable and/or water-vapour-permeable textile material, preferably a woven fabric, a knitted fabric, a crocheted fabric, a scrim, a non-woven fabric or a textile composite material.

10. The sheet filter material according to any one of the preceding claims,
wherein the adsorption layer (6), in particular the adsorber particles (6') of the adsorption layer (6), is fixed and/or adhered to the carrier material (2); and/or
wherein the adsorption layer (6), in particular the adsorber particles (6') of the adsorption layer (6), is fixed and/or adhered to a (second) flat side (2b) of the carrier material (2), in particular via a (first) flat side (6a) of the adsorption layer (6); and/or
wherein the adsorption layer (6), in particular the adsorber particles (6') of the adsorption layer (6) is fixed and/or adhered to the carrier material (2), in particular to the (second) flat side (2b) of the carrier material (2), by using an adhesive applied in particular in a discontinuously punctiform manner; and/or
wherein the adsorption layer (6) is not associated with the nanofibre layer (3) and/or wherein the adsorption layer (6) is not arranged between the carrier material (2) and the nanofibre layer (3); and/or
wherein the adsorption layer (6), in particular the adsorber particles (6') of the adsorption layer (6), is/are not fixed to the carrier material (2) and/or to the textile carrier (4) and/or wherein the adsorption layer (6), in particular the adsorber particles (6') of the adsorption layer (6), is/are not adhered to the carrier material (2) and/or to the textile carrier (4); and/or wherein the adsorption layer (6) is formed as a layer separate from and/or not connected to the carrier material (2) and/or to the nanofibre layer (3) and/or to the textile carrier (4), in particular as one not fixed and/or adhered to the aforementioned layers (2, 3, 4).

11. The sheet filter material according to any one of the preceding claims,
wherein the sheet filter material (1) is self-carrying and/or wherein the sheet filter material (1) is formed as a composite material; and/or
wherein the sheet filter material (1) has a total weight per unit area in the range from 10 g/m² to 800 g/m², in particular in the range from 20 g/m² to 600 g/m², preferably in the range from 50 g/m² to 400 g/m², preferably in the range from 75 g/m² to 300 g/m², particularly preferably in the range from 100 g/m² to 200 g/m²; and/or
wherein the sheet filter material (1) has an air permeability of at least 10 l·m⁻²·s⁻¹, in particular at least 25 l·m⁻²·s⁻¹, preferably at least 30 l·m⁻²·s⁻¹, preferably at least 40 l·m⁻²·s⁻¹, particularly preferably at least 50 l·m⁻²·s⁻¹, more particularly preferably at least 100 l·m⁻²·s⁻¹, at a flow resistance of 127 Pa; and/or
wherein the sheet filter material (1) has a water-vapour permeability of at least 10 l/m² per 24h, in particular at least 20 l/m² per 24h, preferably at least 30 l/m² per 24h at 25°C; and/or
wherein the sheet filter material (1) has a water vapour transmission resistance Rₑₜ under stationary conditions, measured according to DIN EN 31 092:1993 (February 1994) and International Standard ISO 11 092, of at most 25 (m²·Pascal)/watt, in particular at most 20 (m²·Pascal)/watt, preferably at most 10 (m²·Pascal)/watt at 35°C; and/or wherein the sheet filter material (1) has a total thickness in the range from 0.1 mm to 30 mm, in particular in the range from 0.2 mm to 20 mm, preferably in the range from 0.5 mm to 10 mm, preferably in the range from 0.6 mm to 6 mm, particularly preferably in the range from 0.8 mm to 2 mm.

12. A method for producing a sheet filter material (1) with an aerosol and/or particle filter function and with a protective function against chemical, biological and/or radioactive pollutants and toxins, in particular as defined in any one of the preceding claims, wherein the method comprises the following steps:
(a) providing and/or producing an air-permeable and/or water-vapour-permeable, flat foam-based and/or foam-shaped carrier material (2) with two opposite flat sides (2a, 2b), wherein the carrier material (2) is an air-permeable and/or discontinuous layer based on a dried or cured, preferably cross-linked, broken polymer foam structure, wherein the broken polymer foam structure is open-pored and/or not closed;
(b) applying an air-permeable and/or water-vapour-permeable, preferably flat nanofibre layer (3), the nanofibre layer (3) comprising or consisting of a plurality of nanofibres (3'), on a (first) flat side (2a) of the foam-based and/or foam-shaped carrier material (2) such that the nanofibre layer (3) is fixed and/or adhered to the (first) flat side (2a) of the carrier material (2);
wherein the sheet filter material (1) is further equipped and/or provided with at least one adsorption layer (6), wherein the adsorption layer (6) comprises or is formed from a plurality of individual and/or discrete adsorber particles (6'), wherein the adsorption layer (6) has two opposite flat sides (6a, 6b), wherein the adsorption layer (6) is associated with the carrier material (2) and wherein the adsorption layer (6) is arranged on the (second) side (2b) of the carrier material (2) facing away from the nanofibre layer (3).

13. A use of a sheet filter material as defined in any one of claims 1 to 11,
for producing protective equipment and/or protective objects of all kinds, in particular protective clothing, in particular for the civil or military sector, such as protective suits, protective gloves, protective footwear, protective socks, protective headgear, or the like, and/or for the production of protective covers of all kinds, preferably all of the aforementioned protective materials and/or protective clothing for NBC deployment and/or with a protective function against chemical, biological and/or radioactive pollutants and toxins; and/or
for producing sports and/or leisure clothing and/or sports and/or leisure equipment, such as sports and/or leisure trousers and/or jackets, sports and/or leisure underwear, sports and/or leisure socks, sports and/or or leisure gloves, sports and/or leisure headgear and sports and/or leisure shoe materials, in particular sports and/or leisure footwear, preferably all of the aforementioned materials and/or items of clothing for outside use (outdoor deployment) and/or with water-repellent (i.e. repelling water in its liquid form) and/or wind-repellent and/or breathable (i.e. water-vapour-permeable and preferably also air-permeable) properties; and/or
for producing filters and filter materials of all kinds, in particular for removing harmful,
odorous and toxic substances of all kinds, preferably for removing chemical, biological and/or radioactive pollutants and toxins, in particular from air and/or gas flows, such as NBC protective mask filters, odour filters, surface filters, air filters, in particular filters for room air purification, adsorptive support structures and filters for the medical sector.

14. Protective equipment and/or protective objects of all kinds, in particular protective clothing, in particular for the civil or military sector, such as protective suits, protective gloves, protective footwear, protective socks, protective headgear, or the like, as well as protective covers, preferably all of the aforementioned protective materials and/or protective clothing for NBC deployment and/or with a protective function against chemical, biological and/or radioactive pollutants and toxins; and/or
sports and/or leisure clothing and/or sports and/or leisure equipment of all kinds, in particular for outside use (outdoor deployment) and/or with water-repellent (i.e. repelling water in its liquid form) and/or wind-repellent and/or breathable (i.e. water-vapour-permeable and preferably also air-permeable) properties, such as sports and/or leisure trousers and/or jackets, sports and/or leisure underwear, sports and/or leisure socks, sports and/or or leisure gloves, sports and/or leisure headgear and sports and/or leisure shoe materials, in particular sports and/or leisure footwear,
each produced using a sheet filter material (1) as defined in any one of claims 1 to 11 and/or having a sheet filter material (1) as defined in any one of claims 1 to 11.

15. Filters and filter materials of all kinds, in particular for removing harmful, odorous and toxic substances of all kinds, preferably for removing chemical, biological and/or radioactive harmful and toxic substances, in particular from air and/or gas flows, such as protective mask filters, odour filters, sheet filters, air filters, in particular filters for cleaning indoor air, adsorptive carrier structures and filters for the medical sector, produced using a sheet filter material (1) as defined in any one of claims 1 to 11, and/or having a sheet filter material (1) as defined in any one of the Claims 1 to 11.

## Revendications

1. Matériau filtrant plan (1) ayant une fonction d'aérosol et/ou de filtre de particules et ayant une fonction de protection contre les polluants et substances toxiques chimiques, biologiques et/ou radioactifs,
le matériau filtrant plan (1) comprenant :
(a) un matériau support (2) à base de mousse et/ou en forme de mousse plat perméable à l'air et/ou à la vapeur d'eau ayant deux côtés plats opposés (2a, 2b), le matériau support (2) étant constitué d'une couche perméable à l'air et/ou de forme discontinue à base d'une couche séchée ou durcie, de préférence une structure de mousse polymère réticulée, cassée, la structure de mousse polymère cassée étant à pores ouverts et/ou non fermée ;
(b) une couche de nanofibres (3) perméable à l'air et/ou à la vapeur d'eau, de préférence plate, associée au matériau support (2) à base de mousse et/ou en forme de mousse, fixée et/ou amenée pour être collée sur un (premier) côté plat (2a) du matériau support à base de mousse et/ou en forme de mousse (2), la couche de nanofibres (3) comprenant ou étant constituée d'une pluralité de nanofibres (3') ;
dans lequel le matériau filtrant plan (1) a en outre au moins une couche d'adsorption (6), la couche d'adsorption (6) comprenant ou étant constituée d'une pluralité de particules d'adsorption (6') individuelles et/ou discrètes, la couche d'adsorption (6) ayant deux côtés plats opposés (6a , 6b), la couche d'adsorption (6) étant associée au matériau support (2) et la couche d'adsorption (6) étant disposée sur le (second) côté plat (2b) du matériau support (2) opposé à la couche de nanofibres (3).

2. Matériau filtrant plan selon la revendication 1,
dans lequel le matériau support (2) présente une masse surfacique comprise dans la plage de 5 g/m² à 500 g/m² , en particulier dans la plage de 10 g/m² à 300 g/m², de préférence dans la plage de 15 g/m² à 200 g/m², de préférence dans la plage de 20 g/m² à 150 g/m², de préférence encore dans la plage de 25 g/m² à 100 g/m², mieux encore dans la plage de 25 g/m² à 50 g/m² ; et/ou
dans lequel le matériau support (2) a une épaisseur inférieure ou égale à 10 mm, en particulier inférieure ou égale à 5 mm, de préférence inférieure ou égale à 4 mm, de préférence inférieure ou égale à 3 mm, de préférence encore inférieure ou égale à 1 mm, mieux encore inférieure ou égale à 0,5 mm, et/ou dans lequel le matériau support (2) a une épaisseur comprise dans la plage de 0,01 mm à 10 mm, en particulier dans la plage de 0,03 mm à 5 mm, de préférence dans la plage de 0,05 mm à 4 mm, de préférence dans la plage de 0,08 mm à 3 mm, de préférence encore dans la plage de 0,09 mm à 1 mm, mieux encore dans la plage de 0,1 mm à 0,5 mm, idéalement dans la plage de 0,1 mm à 0,3 mm ; et/ou
dans lequel matériau support (2) a une densité comprise dans la plage de 25 g/l à 250 g/l, en particulier de 50 g/l à 200 g/l, de préférence de 75 g/l à 175 g/l, de préférence de 100 g/l à 170 g/l.

3. Matériau filtrant plan selon la revendication 1 ou 2,
dans lequel la structure de mousse polymère cassée a une pluralité de bulles de mousse séchées ou durcies, en particulier réticulées, détruites et/ou éclatées et/ou aplaties ; et ou dans lequel la structure de mousse polymère cassée, de préférence les bulles de mousse séchées ou durcies, en particulier réticulées, détruites et/ou éclatées et/ou aplaties de la structure de mousse polymère cassée ont une pluralité de parois et/ou d'éléments jointifs détruits et/ou cassés et/ou aplatis, en particulier en matériau de structure de mousse (polymère de structure de mousse) ; et/ou
dans laquelle la structure de mousse polymère cassée présente une proportion de bulles de mousse détruites et/ou éclatées et/ou aplaties d'au moins 10 % , en particulier d'au moins 30 % , de préférence d'au moins 50 % , de préférence d'au moins 70 %, de préférence encore d'au moins 90 %, mieux encore d'au moins 95 %, par rapport au nombre total de bulles de mousse dans la structure de mousse polymère cassée.

4. Matériau filtrant plan selon l'une quelconque des revendications précédentes,
dans lequel la structure de mousse polymère cassée a une pluralité de perforations, pores, canaux et/ou ouvertures s'étendant dans la structure de mousse polymère cassée et/ou une pluralité d'ouvertures, pores, canaux et/ou ouvertures reliant en particulier les côtés plats respectifs (2a, 2b) du matériau support (2) ; et ou
dans lequel la structure de mousse polymère cassée est contiguë et/ou cohérente.

5. Matériau filtrant plan selon l'une quelconque des revendications précédentes,
dans lequel la couche de nanofibres (3) a deux côtés plats opposés (3a, 3b) ; et/ou dans lequel la couche de nanofibres (3) est fixée et/ou amenée à adhérer au (premier) côté plat (2a) du matériau support (2) au moins essentiellement sur toute la surface et/ou sur tout un (premier) côté plat (3a) de la couche de nanofibres (3) ; et/ou dans lequel la couche de nanofibres (3) est fixée et/ou est amenée à adhérer directement et/ou directement et/ou sans couche(s) intermédiaire(s), en particulier sans couche d'adsorption intermédiaire, sur le (premier) côté plat (2a) du matériau support (2), en particulier sur un (premier) côté plat (3a) de la couche de nanofibres (3) ; et/ou dans lequel la couche de nanofibres (3) est fixée et/ou amenée à adhérer au (premier) côté plat (2a) du matériau support (2) sans l'utilisation et/ou l'addition d'un adhésif et/ou d'une colle.

6. Matériau filtrant plan selon l'une quelconque des revendications précédentes,
dans lequel la couche de nanofibres (3) est conçue comme un tissu textile, en particulier un tissu textile tridimensionnel, comprenant et/ou constitué des nanofibres (3') ; et/ou dans lequel les nanofibres (3') dans la couche de nanofibres (3) sont disposées de manière aléatoire et/ou éparse ; et/ou
dans lequel les nanofibres (3') dans la couche de nanofibres (3) sont reliées entre elles, en particulier sont reliées en permanence et/ou collées les unes aux autres, notamment au moyen et/ou à la suite de, en particulier, lors de la fabrication du matériau filtrant plan (1) et/ou de la couche de nanofibres (3) présentant une adhérence inhérente aux nanofibres (3') ; et/ou
dans laquelle les nanofibres (3') de la couche de nanofibres (3) forment une structure plane cohérente et/ou cohésive, en particulier un matériau composite plan ; et/ou dans lequel la couche de nanofibres (3) est conçue comme un tissu textile cohérente et/ou cohésive, en particulier un matériau composite plan ; et/ou
dans lequel la couche de nanofibres (3) est conçue sous forme d'un canevas ou d'un composite textile, en particulier un non-tissé, de préférence sous forme d'un non-tissé, de préférence sous forme d'un non-tissé aléatoire ; et/ou
dans lequel la couche de nanofibres (3) a une épaisseur inférieure ou égale à 100 µm, en particulier inférieure ou égale à 50 µm, de préférence inférieure ou égale à 20 µm, de préférence inférieure ou égale à 10 µm, de préférence encore inférieure ou égale à 5 µm, et/ou dans lequel la couche de nanofibres (3) a une épaisseur comprise dans la plage de 0,001 µm à 100 µm, en particulier dans la plage de 0,005 µm à 50 µm, de préférence dans la plage de 0,008 µm à 20 µm, de préférence dans la plage de 0,01 µm à 10 µm, de préférence encore dans la plage de 0,15 µm à 8 µm, mieux encore dans la gamme de 1 µm à 7 µm, plus préférentiellement dans la plage de 2 µm à 6 µm, plus préférentiellement dans la plage de 3 µm à 5 µm, idéalement dans la plage de 4 µm à 5 µm.

7. Matériau filtrant plan selon l'une quelconque des revendications précédentes,
dans lequel les nanofibres (3') ont un diamètre moyen de fibre D₅₀ compris entre 2 nm et 4500 nm, en particulier entre 10 nm et 2000 nm, de préférence entre 15 nm et 1750 nm, de préférence entre 20 nm et 1250 nm , de préférence encore entre 25 nm et 750 nm, tout particulièrement de préférence entre 30 nm et 500 nm, mieux encore entre 60 nm et 400 nm ; et/ou
dans lequel la couche de nanofibres (3) a des ouvertures ou des pores délimités par les nanofibres (3'), en particulier dans lequel la couche de nanofibres (3) a une taille d'ouverture moyenne ou une taille moyenne de pores inférieure ou égale à 150 µm, en particulier inférieure ou égale à 100 µm, de préférence inférieure ou égale à 50 µm, de manière particulièrement préférée inférieure ou égale à 30 µm, tout particulièrement préférentiellement inférieure ou égale à 20 µm, encore plus préférentiellement inférieure ou égale à 10 µm, et/ou en particulier le rapport de la taille moyenne d'ouverture ou de la taille moyenne des pores d'une part au diamètre moyen des fibres D₅₀ des nanofibres (3') d'autre part étant compris entre 0,2 et 1800, en particulier entre 1 et 400, de préférence entre 5 et 300, de manière particulièrement préférée entre 10 et 250, tout particulièrement entre 25 et 225.

8. Matériau filtrant plan selon l'une quelconque des revendications précédentes,
dans lequel le matériau filtrant plan (1) présente en outre (c) au moins un support textile (4),
en particulier dans lequel le support textile (4) présente deux côtés plats opposés (4a, 4b) ; et/ou
en particulier le support textile (4) étant associé au matériau support (2) et/ou en particulier le support textile (4) étant disposé sur le (second) côté plat (2b) du matériau support (2) opposé à la couche de nanofibres (3) ; et/ou
en particulier dans lequel le support textile (4) est fixé et/ou amené pour adhérer au matériau support (2) ; et/ou
en particulier dans lequel le support textile (4) est fixé et/ou amené pour adhérer à un (second) côté plat (2b) du matériau support (2), en particulier par l'intermédiaire d'un (premier) côté plat (4a) du support textile (4) ; et/ou
dans lequel le support textile (4) est conçu sous forme d'un tissu textile, de préférence un matériau textile perméable à l'air et/ou perméable à la vapeur d'eau, de préférence un tissu tissé, un article à mailles, un tricot à mailles, un canevas, un non-tissé ou un textile composite.

9. Matériau filtrant plan selon l'une quelconque des revendications précédentes,
dans lequel le matériau filtrant plan (1) présente en outre (d) au moins une couche de recouvrement (5),
en particulier dans lequel la couche de recouvrement (5) a deux côtés plats opposés (5a, 5b) ; et/ou
en particulier dans lequel la couche de recouvrement (5) est associée à la couche de nanofibres (3) et/ou en particulier dans lequel la couche de recouvrement (5) est disposée sur le (second) côté plat (3b) de la couche de nanofibres (3) opposé au matériau support (2) ; et/ou
en particulier dans lequel la couche de recouvrement (5) est fixée et/ou amenée à adhérer à la couche de nanofibres (3) ; et/ou
en particulier dans lequel la couche de recouvrement (5) est fixée et/ou amenée à adhérer à un (second) côté plat (3b) de la couche de nanofibres (3), en particulier sur un (premier) côté plat (5a) de la couche de recouvrement (5) ; et/ou
dans lequel la couche de recouvrement (5) est conçue comme un tissu textile, de préférence un matériau textile perméable à l'air et/ou perméable à la vapeur d'eau, de préférence un tissu tissé, un article à mailles, un tricot à mailles, un canevas, un non-tissé ou un textile composite.

10. Matériau filtrant plan selon l'une quelconque des revendications précédentes,
dans lequel la couche d'adsorption (6), en particulier les particules adsorbantes (6') de la couche d'adsorption (6), est fixée et/ou est amenée à adhérer au matériau support (2) ; et/ou
dans lequel la couche d'adsorption (6), en particulier les particules adsorbantes (6') de la couche d'adsorption (6), est fixée et/ou amenée à adhérer à un (second) côté plat (2b) du matériau support (2), en particulier par l'intermédiaire d'un (premier) côté plat (6a) de la couche d'adsorption (6) ; et/ou
dans lequel la couche d'adsorption (6), en particulier les particules adsorbantes (6') de la couche d'adsorption (6), est fixée et/ou amenée pour adhérer en particulier sur le (second) côté plat (2b) du matériau support (2) au moyen d'un adhésif appliqué notamment en forme de points discontinus sur le matériau support (2) ; et/ou dans lequel la couche d'adsorption (6) n'est pas associée à la couche de nanofibres (3) et/ou dans lequel la couche d'adsorption (6) n'est pas disposée entre le matériau support (2) et la couche de nanofibres (3) ; et/ou
dans lequel la couche d'adsorption (6), en particulier les particules adsorbantes (6') de la couche d'adsorption (6), n'est pas/ne sont pas fixée(s) au matériau support (2) et/ou au support textile (4) et/ou dans lequel la couche d'adsorption (6), en particulier les particules adsorbantes (6') de la couche d'adsorption (6), n'est pas/ne sont pas fixée(s) et/ou amenée(s) à adhérer au matériau support (2) et/ou au support textile (4) ; et/ou dans lequel la couche d'adsorption (6) est conçue comme une couche séparée et/ou non liée au matériau support (2) et/ou non liée à la couche de nanofibres (3) et/ou non liée au support textile (4), en particulier qui n'est pas fixée et/ou n'est pas amenée à adhérer aux couches précitées (2, 3, 4).

11. Matériau filtrant plan selon l'une quelconque des revendications précédentes,
dans lequel le matériau filtrant plan (1) est autoportant et/ou dans lequel le matériau filtrant plan (1) est conçu comme un matériau composite ; et/ou
dans lequel le matériau filtrant plan (1) présente une masse surfacique comprise dans la plage de 10 g/m² à 800 g/m² , en particulier de 20 g/m² à 600 g/m², de préférence de 50 g/m² à 400 g/m², de préférence de 75 g/m² à 300 g/m², de préférence encore de 100 g/m² à 200 g/m² ; et/ou
dans lequel le matériau filtrant plan (1) a une perméabilité à l'air supérieure ou égale à 10 l·m⁻²·s⁻¹, en particulier supérieure ou égale à 25 l·m⁻²·s⁻¹, de préférence supérieure ou égale à 30 l·m⁻²·s⁻¹, de préférence encore supérieure ou égale à 40 l·m⁻²·s⁻¹, de manière particulièrement préférée supérieure ou égale à 50 l·m⁻²·s⁻¹, de manière tout particulièrement préférée supérieure ou égale à 100 l·m⁻²·s⁻¹, avec une résistance à l'écoulement de 127 Pa ; et/ou
dans lequel le matériau filtrant plan (1) présente à 25 °C une perméabilité à la vapeur d'eau supérieure ou égale à 10 l/m² par 24 h, en particulier supérieure ou égale à 20 l/m² par 24 h, de préférence supérieure ou égale à 30 l/m² par 24 h ; et/ou dans lequel le matériau filtrant plan (1) présente une résistance à la transmission de la vapeur d'eau Rₑₜ dans des conditions stationnaires, mesurée selon la norme DIN EN 31 092 : 1993 (février 1994) et la norme internationale ISO 11 092, à 35 °C, inférieure ou égale à 25 (m²·Pascal)/watt en particulier inférieure ou égale à 20 (m²· Pascal)/watt, de préférence inférieure ou égale à 10 (m²·Pascal)/watt ; et/ou dans lequel le matériau filtrant plan (1) a une épaisseur totale comprise entre 0,1 mm et 30 mm, en particulier comprise entre 0,2 mm et 20 mm, de préférence comprise entre 0,5 mm et 10 mm, de préférence encore comprise entre 0,6 mm et 6 mm, de manière particulièrement préférée comprise entre 0,8 mm et 2 mm.

12. Procédé de fabrication d'un matériau filtrant plan (1) ayant une fonction d'aérosol et/ou de filtre de particules et ayant une fonction de protection contre les polluants et substances toxiques chimiques, biologiques et/ou radioactifs, en particulier tels que définis dans l'une des revendications précédentes, le procédé comprenant les étapes suivantes :
(a) préparation et/ou fabrication d'un matériau support (2) à base de mousse plate et/ou en forme de mousse perméable à l'air et/ou à la vapeur d'eau avec deux côtés plats opposés (2a, 2b), le matériau support (2) étant une couche perméable à l'air et/ou discontinue à base d'une structure de mousse polymère cassée, séchée ou durcie, de préférence réticulée, dans laquelle la structure de mousse polymère cassée est à pores ouverts et/ou non fermée ;
(b) application d'une couche de nanofibres (3) perméable à l'air et/ou à la vapeur d'eau, de préférence plate, la couche de nanofibres (3) comprenant ou consistant en une pluralité de nanofibres (3') sur un (premier) côté plat (2a) du matériau support (2) à base de mousse et/ou en forme de mousse de telle sorte que la couche de nanofibres (3) est fixée et/ou amenée à adhérer au (premier) côté plat (2a) du matériau de support (2) ;
dans lequel le matériau filtrant plan (1) est en outre équipé et/ou pourvu d'au moins une couche d'adsorption (6), dans lequel la couche d'adsorption (6) comprend ou est formée d'une pluralité de particules d'adsorption individuelles et/ou discrètes (6'), la couche d'adsorption (6) ayant deux côtés plats opposés (6a, 6b), la couche d'adsorption (6) étant associée au matériau support (2) et la couche d'adsorption (6) étant disposée sur le (second) côté (2b) du matériau support (2) opposé à la couche de nanofibres (3).

13. Utilisation d'un matériau filtrant plan selon l'une quelconque des revendications 1 à 11, pour la fabrication d'équipements de protection et/ou d'objets de protection de tous types, en particulier de vêtements de protection, en particulier pour le secteur civil ou militaire, tels que combinaisons de protection, gants de protection, chaussures de protection, chaussettes de protection, couvre-chefs de protection, ou similaires, et/ou pour la fabrication de housses de protection de toutes sortes, de préférence tous les matériaux de protection précités et/ou vêtements de protection à usage NRBC et/ou ayant une fonction de protection contre les polluants et substances toxiques chimiques, biologiques et/ou radioactifs ; et/ou
pour la fabrication de vêtements de sport et/ou de loisirs et/ou d'équipements de sport et/ou de loisirs, tels que pantalons et/ou vestes de sport et/ou de loisirs, sous-vêtements de sport et/ou de loisirs, chaussettes de sport et/ou de loisirs et/ou de sports et/ou ou gants de loisirs, couvre-chefs de sport et/ou de loisir et matériaux pour chaussures de sport et/ou de loisir, en particulier chaussures de sport et/ou de loisir, de préférence tous les matériaux et/ou vêtements précités à usage extérieur et/ou ayant des propriétés hydrofuges (c'est-à-dire repoussant l'eau sous forme liquide) et/ou de coupe-vent et/ou respirantes (c'est-à-dire perméables à la vapeur d'eau et de préférence également perméables à l'air) ; et/ou
pour la fabrication de filtres et de matériaux filtrants de tous types, en particulier pour l'élimination de substances nocives, odorantes et toxiques de tous types, de préférence pour l'élimination de polluants chimiques, biologiques et/ou radioactifs et de substances toxiques, en particulier des flux d'air et/ou de gaz, tels que filtres pour masque de protection NRBC, filtres anti-odeurs, filtres plans, filtres à air, en particulier filtres pour la purification de l'air ambiant, structures de support adsorbantes et filtres pour le secteur médical.

14. Équipements de protection et/ou objets de protection de tous types, en particulier pour le secteur civil ou militaire, en particulier vêtements de protection tels que combinaisons de protection, gants de protection, chaussures de protection, chaussettes de protection, couvre-chefs de protection et similaires, ainsi que des housses de protection, de préférence tous les équipements de protection et/ou objets de protection précités à usage NRBC et/ou ou ayant une fonction de protection contre les polluants et substances toxiques chimiques, biologiques et/ou radioactifs ; et/ou
vêtements de sport et/ou de loisirs et/ou équipements de sport et/ou de loisir de tous types, en particulier pour l'extérieur et/ou ayant des propriétés hydrofuges (c'est-à-dire repoussant l'eau sous forme liquide) et/ou de coupe-vent et/ou respirantes (c'est-à-dire perméables à la vapeur d'eau et de préférence également perméables à l'air), tels que pantalons et/ou vestes de sport et/ou de loisirs, sous-vêtements de sport et/ou de loisirs, chaussettes de sport et/ou de loisirs, gants de sport et/ou de loisirs, couvre-chefs de sport et/ou de loisirs et/ou matériaux de chaussures de loisirs, en particulier chaussures de sport et/ou de loisirs,
chaque produit étant fabriqué à partir d'un matériau filtrant plan (1) tel que défini dans l'une quelconque des revendications 1 à 11 et/ou ayant un matériau filtrant plan (1) tel que défini dans l'une quelconque des revendications 1 à 11.

15. Filtres et matériaux filtrants de tous types, en particulier pour éliminer les substances nocives, odorantes et toxiques de tous types, de préférence pour éliminer les substances chimiques, biologiques et/ou radioactives nocives et toxiques, en particulier dans les flux d'air et/ou de gaz, tels que les masques de protection, les filtres anti-odeurs, les filtres plans, les filtres à air, en particulier filtres pour le nettoyage de l'air intérieur, structures de support adsorbantes et filtres pour le secteur médical, réalisés à l'aide d'un matériau filtrant plan (1) tel que définis dans l'une quelconque des revendications 1 à 11, et/ou ayant un matériau filtrant plan (1), tel que défini dans l'une quelconque des revendications 1 à 11.
